(19) Europäisches Patentamt
European Patent Office
Office européen des brevets

(11) **EP 1 636 599 B1**

(12) **EUROPÄISCHE PATENTSCHRIFT**

(45) Veröffentlichungstag und Bekanntmachung des Hinweises auf die Patenterteilung:
**02.05.2007 Patentblatt 2007/18**

(51) Int Cl.:
*G01R 19/12* (2006.01)    *G01N 27/49* (2006.01)
*C12Q 1/68* (2006.01)

(21) Anmeldenummer: **04732086.6**

(22) Anmeldetag: **11.05.2004**

(86) Internationale Anmeldenummer:
**PCT/DE2004/000977**

(87) Internationale Veröffentlichungsnummer:
**WO 2004/102211 (25.11.2004 Gazette 2004/48)**

(54) **SCHALTKREISANORDNUNG ZUR POTENTIALKONSTANTHALTUNG AN EINEM BIOSENSOR UND ZUR DIGITALISIERUNG DES MESSSTROMS**

POTENTIOSTATIC CIRCUIT ARRANGEMENT ON A BIOSENSOR FOR DIGITISATION OF THE MEASURED CURRENT

ENSEMBLE CIRCUIT DE COMMUTATION POUR LA STABILISATION DE POTENTIEL SUR UN BIOCAPTEUR ET LA NUMERISATION DU COURANT DE MESURE

(84) Benannte Vertragsstaaten:
**DE FR GB**

(30) Priorität: **13.05.2003 DE 10321490**

(43) Veröffentlichungstag der Anmeldung:
**22.03.2006 Patentblatt 2006/12**

(73) Patentinhaber: **SIEMENS AKTIENGESELLSCHAFT**
**80333 München (DE)**

(72) Erfinder:
- **FREY, Alexander**
  **81737 München (DE)**
- **PAULUS, Christian**
  **82362 Weilheim (DE)**
- **SCHIENLE, Meinrad**
  **85521 Ottobrunn (DE)**
- **THEWES, Roland**
  **82194 Gröbenzell (DE)**

(56) Entgegenhaltungen:
**WO-A-02/33397**

- **THEWES R ET AL: "Sensor arrays for fully-electronic DNA detection on CMOS" ISSCC2002, Bd. 1, 2002, Seiten 350-351, 472, XP010585601**
- **HOFMANN F ET AL: "PASSIVE DNA SENSOR WITH GOLD ELECTRODES FABRICATED IN A CMOS BACKEND PROCESS" PROCEEDINGS OF THE EUROPEAN SOLID STATE DEVICE RESEARCH CONFERENCE (ESSDERC), EDITION FRONTIERES, GIL-SUR-YVETTE, FR, 24. September 2002 (2002-09-24), Seiten 487-490, XP008033777**
- **USTER M ET AL: "INTEGRATING ADC USING A SINGLE TRANSISTOR AS INTEGRATOR AND AMPLIFIER FOR VERY LOW (1 FA MINIMUM) INPUT CURRENTS" INTERNATIONAL CONFERENCE ON ADVANCED A/D AND D/A CONVERSION TECHNIQUES AND THEIR APPLICATIONS, XX, XX, 27. Juli 1999 (1999-07-27), Seiten 86-89, XP001147236 in der Anmeldung erwähnt**
- **BRETEN M ET AL: "Integrating data converters for picoampere currents from electrochemical transducers" ISCAS 2000, Bd. 5, 28. Mai 2000 (2000-05-28), Seiten 709-712, XP010504295**

EP 1 636 599 B1

**Beschreibung**

Die Erfindung betrifft eine Schaltkreis-Anordnung, für einen elektrochemischen Sensor.

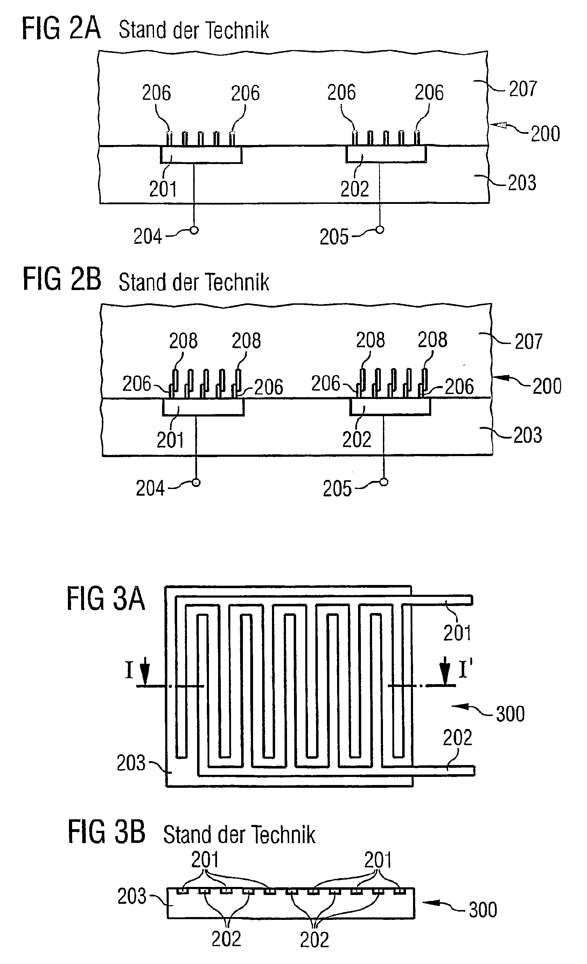

In **Fig.2A, Fig.2B** ist ein Biosensorchip gezeigt, wie er in [1] beschrieben ist. Der Sensor 200 weist zwei Elektroden 201, 202 aus Gold auf, die in einer Isolatorschicht 203 aus elektrisch isolierendem Material eingebettet sind. An die Elektroden 201, 202 sind Elektrodenanschlüsse 204, 205 angeschlossen, mittels derer das elektrische Potential an der Elektrode 201, 202 angelegt werden kann. Die Elektroden 201, 202 sind als Planarelektroden ausgestaltet. Auf jeder Elektrode 201, 203 sind DNA-Sondenmoleküle 206 (auch als Fängermoleküle bezeichnet) immobilisiert (vgl. **Fig. 2A**). Die Immobilisierung erfolgt gemäß der Gold-Schwefel-Kopplung. Auf den Elektroden 201, 202 ist der zu untersuchende Analyt, beispielsweise ein Elektrolyt 207, aufgebracht.

Sind in dem Elektrolyt 207 DNA-Stränge 208 mit einer Basensequenz enthalten, die zu der Sequenz der DNA-Sondenmoleküle 206 komplementär ist, d.h., die zu den Fängermolekülen gemäß dem Schlüssel-Schloss-Prinzip sterisch passen, so hybridisieren diese DNA-Stränge 208 mit den DNA-Sondenmolekülen 206 (vgl. **Fig.2B**).

Eine Hybridisierung eines DNA-Sondenmoleküls 206 und eines DNA-Strangs 208 findet nur dann statt, wenn die Sequenzen des jeweiligen DNA-Sondenmoleküls und des entsprechenden DNA-Strangs 208 zueinander komplementär sind. Ist dies nicht der Fall, so findet keine Hybridisierung statt. Somit ist ein DNA-Sondenmolekül einer vorgegebenen Sequenz jeweils nur in der Lage, einen bestimmten, nämlich den DNA-Strang mit jeweils komplementärer Sequenz, zu binden, d.h. mit ihm zu hybridisieren, woraus der hohe Grad an Selektivität des Sensors 200 resultiert.

Findet eine Hybridisierung statt, so verändert sich, wie aus **Fig.2B** ersichtlich, der Wert der Impedanz zwischen den Elektroden 201 und 202. Diese veränderte Impedanz wird mittels Anlegens einer geeigneten elektrischen Spannung an die Elektrodenanschlüsse 204, 205 und mittels Erfassens des daraus resultierenden Stroms detektiert.

Im Falle einer Hybridisierung verändert sich die Impedanz zwischen den Elektroden 201, 202. Dies ist darauf zurückzuführen, dass sowohl die DNA-Sondenmoleküle 206 als auch die DNA-Stränge 208, die möglicherweise mit den DNA-Sondenmolekülen 206 hybridisieren, elektrisch schlechter leitend sind als der Elektrolyt 207 und somit anschaulich die jeweilige Elektrode 201, 202 teilweise elektrisch abschirmen.

Zur Verbesserung der Messgenauigkeit ist es aus [2] bekannt, eine Mehrzahl von Elektrodenpaaren 201, 202 zu verwenden und diese parallel zueinander anzuordnen, wobei diese anschaulich miteinander verzahnt angeordnet sind, so dass sich eine sogenannte Interdigitalelektrode 300 ergibt, deren Draufsicht in **Fig.3A** und deren Querschnittsansicht entlang der Schnittlinie I-I' aus **Fig.3A** in **Fig.3B** gezeigt ist.

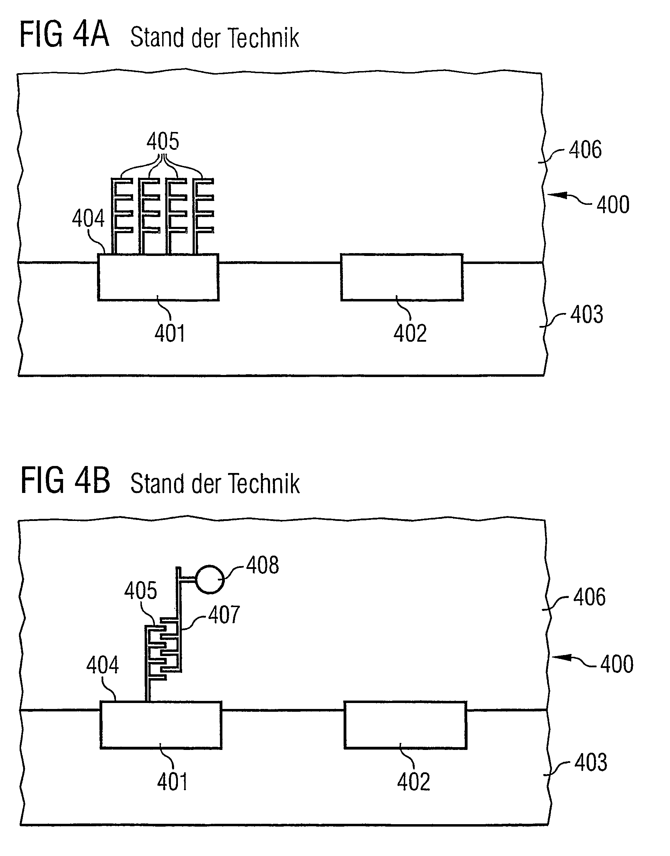

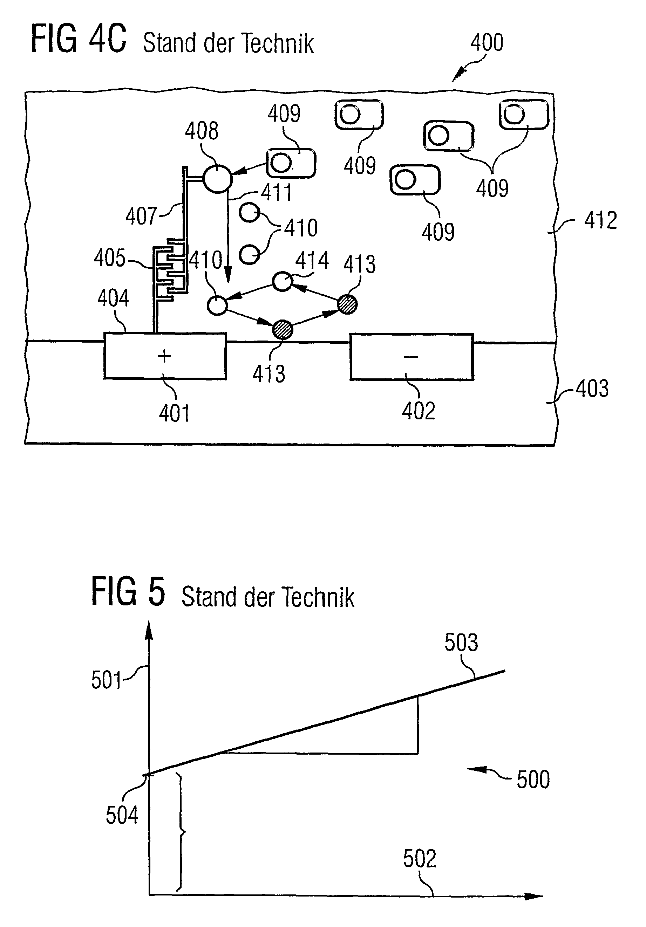

Weiterhin sind Grundlagen über einen Reduktions-/Oxidations-Recycling-Vorgang zum Erfassen makromolekularer Biomoleküle, beispielsweise aus [1], [3] bekannt. Der Reduktions-/Oxidations-Recycling-Vorgang, im Weiteren auch als Redox-Cycling-Vorgang bezeichnet, wird im Weiteren anhand **Fig.4A, Fig.4B, Fig.4C** näher erläutert.

In **Fig.4A** ist ein Biosensor 400 mit einer ersten Elektrode 401 und einer zweiten Elektrode 402 gezeigt, die auf einer Isolatorschicht 403 aufgebracht sind. Auf der ersten Elektrode 401 als Gold ist ein Haltebereich 404 aufgebracht. Der Haltebereich 404 dient zum Immobilisieren von DNA-Sondenmolekülen 405 auf der ersten Elektrode 401. Auf der zweiten Elektrode 402 ist ein solcher Haltebereich nicht vorgesehen.

Sollen mittels des Biosensors 400 DNA-Stränge 407 mit einer Sequenz, die komplementär ist zu der Sequenz der immobilisierten DNA-Sondenmoleküle 405 erfasst werden, so wird der Sensor 400 mit einer zu untersuchenden Lösung, beispielsweise einem Elektrolyt 406, in Kontakt gebracht derart, dass in der zu untersuchenden Lösung 406 eventuell enthaltene DNA-Stränge 407 mit der komplementären Sequenz zu der Sequenz der DNA-Sondenmoleküle 405 hybridisieren können.

**Fig.4B** zeigt den Fall, dass in der zu untersuchenden Lösung 406 die zu erfassenden DNA-Stränge 407 enthalten sind und mit den DNA-Sondenmolekülen 405 hybridisiert sind.

Die DNA-Stränge 407 in der zu untersuchenden Lösung sind mit einem Enzym 408 markiert, mit dem es möglich ist, im Weiteren beschriebene Moleküle in Teilmoleküle zu spalten, von denen mindestens eines redox-aktiv ist. Üblicherweise ist eine erheblich größere Anzahl von DNA-Sondenmolekülen 405 bereitgestellt, als zu ermittelnde DNA-Stränge 407 in der zu untersuchenden Lösung 406 enthalten sind.

Nachdem die in der zu untersuchenden Lösung 406 möglicherweise enthaltenen DNA-Stränge 407 samt dem Enzym 408 mit den immobilisierten DNA-Sondenmolekülen 405 hybridisiert sind, erfolgt eine Spülung des Biosensors 400, wodurch die nicht hybridisierten DNA-Stränge entfernt werden und der Biosensorchip 400 von der zu untersuchenden Lösung 406 gereinigt wird. Der zur Spülung verwendeten Spüllösung oder einer in einer weiteren Phase eigens zugeführten weiteren Lösung wird eine elektrisch ungeladene Substanz beigegeben, die Moleküle enthält, die mittels des Enzyms 408 an den hybridisierten DNA-Strängen 407 gespalten werden können, in ein erstes Teilmolekül 410 und in ein zweites. Eines der beiden Moleküle ist redox-aktiv.

Die beispielsweise negativ-geladenen ersten Teilmoleküle 410 werden, wie in **Fig.4C** gezeigt, zu der positiv geladenen ersten Elektrode 401 gezogen, was mittels des Pfeils 411 in **Fig.4C** angedeutet ist. Die negativ geladenen ersten Teilmoleküle 410 werden an der ersten Elektrode 401, die ein positives elektrisches Potential aufweist, oxidiert und werden als oxidierte Teilmoleküle 413 an die negativ geladene zweite Elektrode 402 gezogen, wo sie wieder reduziert werden. Die reduzierten Teilmoleküle 414 wiederum wandern zu der positiv geladenen ersten Elektrode 401. Auf diese Weise wird ein elektrischer Kreisstrom generiert, der proportional ist zu der Anzahl der jeweils mittels der Enzyme 406 erzeugten Ladungsträger.

Der elektrische Parameter, der bei dieser Methode ausgewertet wird, ist die Änderung des elektrischen Stroms m=dI/dt als Funktion der Zeit t, wie dies in dem Diagramm 500 in **Fig.5** schematisch dargestellt ist.

**Fig.5** zeigt die Funktion des elektrischen Stroms 501 in Abhängigkeit von der Zeit 502. Der sich ergebende Kurvenverlauf 503 weist einen Offsetstrom $I_{offset}$ 504 auf, der unabhängig ist von dem zeitlichen Verlauf. Der Offsetstrom $I_{offset}$ 504 wird erzeugt aufgrund von Nichtidealitäten des Biosensors 400. Eine wesentliche Ursache für den Offsetstrom $I_{offset}$ liegt darin, dass die Bedeckung der ersten Elektrode 401 mit den DNA-Sondenmolekülen 405 nicht ideal, d.h. nicht vollständig dicht erfolgt. Im Falle einer vollständig dichten Bedeckung der ersten Elektrode 401 mit den DNA-Sondenmolekülen 405 ergebe sich aufgrund der sogenannten Doppelschichtkapazität, die durch die immobilisierten DNA-Sondenmoleküle 405 entsteht, zwischen der ersten Elektrode 401 und der elektrisch leitenden, zu untersuchenden Lösung 406 eine im Wesentlichen kapazitive elektrische Kopplung. Die nicht vollständige Bedeckung führt jedoch zu parasitären Strompfaden zwischen der ersten Elektrode 401 und der zu untersuchenden Lösung 406, die unter anderem auch ohmsche Anteile aufweisen.

Um jedoch den Oxidations-/Reduktions-Prozess zu ermöglichen, soll die Bedeckung der ersten Elektrode 401 mit den DNA-Sondenmolekülen 405 gar nicht vollständig sein, damit die elektrisch geladenen Teilmoleküle, d.h. die negativ geladenen ersten Teilmoleküle 410 zu der ersten Elektrode 401 infolge einer elektrischen Kraft sowie durch Diffusionsprozesse gelangen können. Um andererseits eine möglichst große Sensitivität eines solchen Biosensors zu erreichen, und um simultan möglichst geringe parasitäre Effekte zu erreichen, sollte die Bedeckung der ersten Elektrode 401 mit DNA-Sondenmolekülen 405 ausreichend dicht sein. Um eine hohe Reproduzierbarkeit der mit einem solchen Biosensor 400 bestimmten Messwerte zu erreichen, sollen beide Elektroden 401,402 stets ein hinreichend großes Flächenangebot für den Oxidations-/Reduktions-Prozess im Rahmen des Redox-Cycling-Vorgangs bereitstellen.

Unter makromolekularen Biomolekülen sind beispielsweise Proteine oder Peptide oder auch DNA-Stränge einer jeweils vorgegebenen Sequenz zu verstehen. Sollen als makromolekulare Biomoleküle, Proteine oder Peptide erfasst werden, so sind die ersten Moleküle und die zweiten Moleküle Liganden, beispielsweise Wirkstoffe mit einer möglichen Bindungsaktivität, welche die zu erfassenden Proteine oder Peptide an die jeweilige Elektrode binden, auf der die entsprechenden Liganden angeordnet sind.

Als Liganden können Enzymagonisten, Pharmazeutika, Zucker oder Antikörper verwendet werden oder irgendein anderes Molekül, das die Fähigkeit aufweist, Proteine oder Peptide spezifisch zu binden.

Werden als makromolekulare Biomoleküle DNA-Stränge einer vorgegebenen Sequenz verwendet, die mittels des Biosensors erfasst werden sollen, so können mittels des Biosensors DNA-Stränge einer vorgegebenen Sequenz mit DNA-Sondenmolekülen mit der zu der Sequenz der DNA-Stränge komplementären Sequenz als Moleküle auf der ersten Elektrode hybridisiert werden.

Unter einem Sondenmolekül(auch Fängermolekül genannt) ist ein Ligand oder ein DNA-Sondenmolekül zu verstehen.

Der oben eingeführte Wert m=dI/dt, welcher der Steigung der Geraden 503 aus **Fig.5** entspricht, hängt von der Länge sowie der Breite der zur Erfassung des Mess-Stroms verwendeten Elektroden ab. Daher ist der Wert m näherungsweise proportional zur Längsausdehnung der verwendeten Elektroden, beispielsweise bei der ersten Elektrode 201 und der zweiten Elektrode 202 proportional zu deren Länge senkrecht zur Zeichenebene in **Fig.2A** und **Fig.2B.** Sind mehrere Elektroden parallel geschaltet, beispielsweise in der bekannten Interdigitalelektroden-Anordnung (vgl. **Fig.3A, Fig.3B**), so ist die Änderung des Mess-Stroms proportional zur Anzahl der jeweils parallel geschalteten Elektroden.

Der Wert der Änderung des Mess-Stroms kann jedoch aufgrund unterschiedlicher Einflüsse einen sehr stark schwankenden Wertebereich aufweisen, wobei der von einem Sensor detektierbare Strom-Bereich als Dynamikbereich bezeichnet wird. Häufig wird als wünschenswerter Dynamikbereich ein Stromstärke-Bereich von fünf Dekaden genannt. Ursachen für die starken Schwankungen können neben der Sensor-Geometrie auch biochemische Randbedingungen sein. So ist es möglich, dass zu erfassende makromolekulare Biomoleküle unterschiedlicher Typen stark unterschiedliche Wertebereiche für das sich ergebende Mess-Signal, d.h. insbesondere den Mess-Strom und dessen zeitliche Änderung bewirken, was wiederum zu einer Ausweitung des erforderlichen gesamten Dynamikbereichs mit entsprechenden Anforderungen für eine vorgegebene Elektrodenkonfiguration mit nachfolgender einheitlicher Mess-Elektronik führt.

Die Anforderungen an den großen Dynamikbereich einer solchen Schaltung führen dazu, dass die Messelektronik teuer und kompliziert ausgestaltet ist, um in dem erforderlichen Dynamikbereich ausreichend genau und zuverlässig zu arbeiten.

Ferner ist häufig der Offsetstrom $I_{offset}$ viel größer als die zeitliche Änderung des Mess-Stroms m über die gesamte Messdauer hinweg. In einem solchen Szenario muss innerhalb eines großen Signals eine sehr kleine zeitabhängige Änderung mit hoher Genauigkeit gemessen werden. Dies stellt sehr hohe Anforderungen an die eingesetzten Messinstrumente, was das Erfassen des Mess-Stroms aufwändig, kompliziert und teuer gestaltet. Auch wirkt diese Tatsache einer angestrebten Miniaturisierung von Sensor-Anordnungen entgegen.

Zusammenfassend sind die Anforderungen an den Dynamikbereich und daher an die Güte einer Schaltung zum Detektieren von Sensor-Ereignissen ausgesprochen hoch.

Es ist bekannt, beim Schaltungsdesign die Nicht-Idealitäten der verwendeten Bauelemente (Rauschen, Parametervariationen) in der Form zu berücksichtigen, dass für diese Bauelemente in der Schaltung ein Arbeitspunkt gewählt wird, in dem diese Nichtidealitäten eine möglichst vernachlässigbare Rolle spielen.

Sofern eine Schaltung über einen großen Dynamikbereich betrieben werden soll, wird die Einhaltung eines optimalen Arbeitspunkts über alle Bereiche hinweg jedoch zunehmend schwieriger, aufwändiger und damit teurer.

Kleine Signalströme, wie sie beispielsweise an einem Sensor anfallen, können mit Hilfe von Verstärkerschaltungen auf ein Niveau angehoben werden, das eine Weiterleitung des Signalstroms beispielsweise an ein externes Gerät oder eine interne Quantifizierung erlaubt.

Aus Gründen der Störungssicherheit sowie der Benutzerfreundlichkeit ist eine digitale Schnittstelle vom Sensor zum auswertenden System vorteilhaft. Die analogen Mess-Ströme sollen also bereits in der Nähe des Sensors in digitale Signale gewandelt werden, was mittels eines integrierten Analog-Digital-Wandlers (ADC) erfolgen kann. Ein solches integriertes Konzept zum Digitalisieren eines analogen, kleinen Stromsignals ist beispielsweise in [4] beschrieben.

Um den erforderlichen Dynamikbereich zu erreichen, sollte der ADC eine entsprechend große Auflösung und ein ausreichend hohes Signal-Rausch-Verhältnis aufweisen. Das Integrieren eines derartigen Analog-Digital-Wandlers in unmittelbarer Nähe einer Sensor-Elektrode stellt ferner eine hohe technologische Herausforderung dar, die entsprechende Prozessführung ist aufwändig und teuer. Ferner ist das Erreichen eines ausreichend hohen Signal-Rausch-Verhältnisses in dem Sensor außerordentlich schwierig.

[5] offenbart einen Current-Mode Analog-/Digital-Wandler, der für einen maximalen Eingabestrombereich von 5nA und eine Auflösung in der Größenordnung von 1pA gestaltet ist.

[6] offenbart eine Vorrichtung zum Bestimmen und Charakterisieren der Steigungen von Zeit-veränderlichen Signalen.

[7] offenbart einen elektronischen Schaltkreis zum Verfolgen eines elektronischen Signals zum Ermitteln, ob die Steigung des Signals zu einer vorgegebenen Zeit größer oder gleich einem vorgegebenen Wert ist.

[8] Offenbart einen Sensor mit Sensor- und Kollektor-Elektrode, deren Potentiale mithilfe von Regelschaltkreisen eingestellt werden.

Der Erfindung liegt das Problem zugrunde, eine fehlerrobuste Schaltkreis-Anordnung mit einer verbesserten Nachweisempfindlichkeit für zeitlich sehr schwach veränderliche elektrische Ströme zu schaffen.

Das Problem wird durch eine Schaltkreis-Anordnung mit den Merkmalen des unabhängigen Patentanspruchs gelöst.

Im Weiteren wird die Funktionalität der erfindungsgemäßen Schaltkreis-Anordnung anschaulich erläutert. Die Schaltkreis-Anordnung der Erfindung weist eine Sensor-Elektrode auf, an der ein Sensor-Ereignis stattfinden kann. Beispielsweise kann an der Sensor-Elektrode ein Hybridisierungs-Ereignis von in einer zu untersuchenden Flüssigkeit enthaltenen DNA-Halbsträngen mit auf der Sensor-Elektrode immobilisierten Fängermolekülen erfolgen. Weisen die zu erfassenden Moleküle beispielsweise ein Enzym-Label auf, das freie elektrische Ladungsträger in der zu untersuchenden Flüssigkeit erzeugt, so fließt ausgehend von der Sensor-Elektrode in die Schaltkreis-Anordnung der Erfindung ein zu detektierendes elektrisches Strom-Signal. Die erste Schaltungseinheit der Schaltkreis-Anordnung ist derart eingerichtet, dass diese anschaulich das elektrische Potential der Sensor-Elektrode innerhalb eines ersten Referenz-Bereichs hält. Solange das elektrische Potential der Sensor-Elektrode innerhalb dieses Referenz-Bereichs ist, entkoppelt die erste Schaltungseinheit die Sensor-Elektrode von einem Kondensator der zweiten Schaltungseinheit. Gerät das elektrische Potential der Sensor-Elektrode außerhalb des ersten Referenz-Bereichs, so stellt die erste Schaltungseinheit eine graduelle elektrische Kopplung zwischen der Sensor-Elektrode und dem ersten Kondensator der zweiten Schaltungseinheit her. Infolge dieser elektrischen Kopplung ist ein Angleichen des elektrischen Potentials der Sensor-Elektrode mit jenem des ersten Kondensators der zweiten Schaltungseinheit ermöglicht. Anschaulich können zwischen dem Kondensator und der Sensor-Elektrode freie elektrische Ladungen hin- und her- fließen, derart, dass das elektrische Potential der Sensor-Elektrode in den ersten Referenz-Bereich zurückgeführt wird. Dadurch können sukzessive kleine Ladungsmengen ausgehend von der Sensor-Elektrode auf den zweiten Kondensator der zweiten Schaltungseinheit verlagert werden oder umgekehrt. Anschaulich werden kleine Sensor-Ströme zu einem Ladungspaket auf dem Kondensator aufintegriert, bis das Ladungspaket eine vorgegebene, ausreichende Größe aufweist, um nachgewiesen zu werden. Daher ändert sich die auf dem ersten Kondensator der zweiten Schaltungseinheit befindliche Ladungsmenge in für die Anzahl der auf der Sensor-Elektrode erfolgten Sensor-Ereignisse charakteristischer Weise. Mit anderen Worten liefert der erste Kondensator der zweiten Schaltungseinheit diejenige Ladungsmenge auf die Sensor-Elektrode nach, die infolge der Sensor-Ereignisse von der Sensor-Elektrode abfließt. Daher fungieren die erste Schaltungseinheit und der Kondensator unter anderem ähnlich wie ein Potentiostat, indem sie die elektrische Spannung der Sensor-Elektrode innerhalb des ersten Referenz-Bereichs, vorzugsweise auf dem elektrischen Soll-Potential, halten.

Wenn jedoch das elektrische Potential des ersten Kondensators infolge der mit der Sensor-Elektrode ausgetauschten Ladungsträger außerhalb des zweiten Referenz-Bereichs gerät, so wird dieses Ereignis von der zweiten Schaltungseinheit detektiert, und die zweite Schaltungseinheit sorgt dafür, dass der erste Kondensator auf ein erstes elektrisches Referenzpotential gebracht wird. Anschaulich ausgedrückt nimmt die zweite Schaltungseinheit die folgende Funktionalität wahr: Wenn eine ausreichend große Ladungsmenge von der Sensor-Elektrode dem ersten Kondensator entnommen worden ist (oder umgekehrt eine ausreichend große Ladungsmenge von der Sensor-Elektrode auf den ersten Kondensator geflossen ist), wird dieses Ereignis von der zweiten Schaltungseinheit beispielsweise mittels Ausgebens eines Pulses detektiert. Ferner wird dem ersten Kondensator die auf die Sensor-Elektrode abgeflossene elektrische Ladung nachgeliefert (oder dem ersten Kondensator die von der Sensor-Elektrode auf den ersten Kondensator geflossene elektrische Ladung entnommen), um den Kondensator wiederum auf einen definierten Arbeitspunkt, d.h. auf das erste elektrische Referenzpotential, zurückzubringen.

Die erfindungsgemäße Schaltkreis-Anordnung mit der beschriebenen Funktionalität ist dazu geeignet, kleinste analoge elektrische Strom-Signale zu erfassen und in ein digitales Signal, d.h. eine Abfolge von zeitlich aufeinanderfolgenden, getrennten Pulsen umzuwandeln. Die Digitalisierung des analogen Mess-Signals erfolgt in unmittelbarer räumlicher Nähe zu der Sensor-Elektrode, so dass parasitäres, zusätzliches Rauschen infolge eines zeitlich wie räumlich langen Übermittlungsweges eines AnalogSignals weitgehend vermieden ist. Daher weist die erfindungsgemäße Schaltkreis-Anordnung ein hohes Signal-Rausch-Verhältnis beim Erfassen elektrischer Ströme auf.

Die erfindungsgemäße Schaltkreis-Anordnung ist insbesondere geeignet zum Detektieren eines gemäß dem Redox-Cycling-Prinzip erzeugten, sukzessive ansteigenden Strom-Signals (vgl. **Fig.5**). Mittels geeigneten Einstellens der Messzeit bzw. der für die Funktionalität der erfindungsgemäßen Schaltkreis-Anordnung relevanten Referenz-Bereiche des elektrischen Potentials der Sensor-Elektroden und des ersten Kondensators ist die Anzahl der zu detektierenden Ereignisse (z.B. in der Form von Pulsen) flexibel auf die Bedürfnisse des Einzelfalls einstellbar.

Vorzugsweise weist die Schaltkreis-Anordnung ein mit der zweiten Schaltungseinheit elektrisch gekoppeltes Zähler-Element auf, das derart eingerichtet ist, das es die Anzahl und/oder die zeitliche Abfolge der Ereignisse zählt. Ferner kann die Schaltkreis-Anordnung derart eingerichtet sein, dass ein direktes Ausgeben der Sensorfrequenz, d.h. der Frequenz der Ereignisse, vorgesehen ist.

Gemäß einer vorteilhaften Weiterbildung ist das Zähler-Element derart eingerichtet, dass es die zeitliche Abfolge der Ereignisse in mindestens zwei Zeitintervallen in einem zeitlichen Abstand voneinander erfasst.

Mit anderen Worten werden die von der zweiten Schaltungseinheit detektierten Ereignisse, dass das elektrische Potential des ersten Kondensators außerhalb des zweiten Referenz-Bereichs gerät, mittels des Zähler-Elements gezählt, und insbesondere der zeitliche Abstand zwischen aufeinanderfolgenden Ereignissen detektiert. Das Zählen der zeitlichen Abstände der Ereignisse entspricht dem Ermitteln der Frequenz der Ereignisse. Dies bedeutet, dass das analoge Strom-Signal auf der Sensor-Elektrode in ein digitales Signal, das in der ermittelten Frequenz enthalten ist, umgewandelt ist. Dadurch ist insbesondere ein hoher Dynamikbereich der Schaltkreis-Anordnung erreichbar. Technisch ist es mit vertretbarem Aufwand möglich, beispielsweise Frequenzen zwischen 100Hz und 10MHz zu erzeugen, zu detektieren und zu verarbeiten, so dass ein Dynamikbereich von fünf und mehr Dekaden erreichbar ist.

Vorzugsweise weist die erfindungsgemäße Schaltkreis-Anordnung eine mit der ersten Schaltungseinheit koppelbare Kalibrier-Einrichtung zum Kalibrieren der Schaltkreis-Anordnung auf, die derart eingerichtet ist, dass mittels der Kalibrier-Einrichtung an die erste Schaltungseinheit ein zweites elektrisches Referenzpotential anlegbar ist, wobei die zweite Schaltungseinheit entweder mit der Kalibrier-Einrichtung oder mit der Sensor-Elektrode gekoppelt ist.

Die Möglichkeit, erfindungsgemäß die Schaltkreis-Anordnung kalibrieren zu können, erhöht den Grad der Verlässlichkeit der erfassten Signale und ermöglicht eine Kontrolle der einwandfreien Funktionsfähigkeit der Schaltkreis-Anordnung. Ferner kann mittels einer Kalibrierungs-Einrichtung die Messgenauigkeit der Schaltkreis-Anordnung erhöht werden.

Vorzugsweise weist die erste Schaltungseinheit ein erstes Komparator-Element mit zwei Eingängen und einem Ausgang auf, wobei der erste Eingang derart mit der Sensor-Elektrode gekoppelt ist, dass der erste Eingang auf dem elektrischen Potential der Sensor-Elektrode ist, wohingegen der zweite Eingang auf ein drittes elektrisches Referenzpotential gebracht ist, welches das elektrische Soll-Potential definiert. Das erste Komparator-Element ist derart eingerichtet, dass an dessen Ausgang ein derartiges elektrisches Signal erzeugt wird, dass, das elektrische Potential der Sensor-Elektrode in dem vorgebbaren ersten Referenz-Bereich um das vorgebbare elektrische Soll-Potential gehalten wird.

Die erste Schaltungseinheit dient zur Konstanthaltung einer vorgebbaren Spannung, hier als elektrisches Soll-Potential bezeichnet, an den Sensor-Elektroden.

Gemäß einer vorteilhaften Ausgestaltung weist bei der Schaltkreis-Anordnung die erste Schaltungs-Einheit einen veränderbaren ohmschen Widerstand auf, mittels welchem die Sensor-Elektrode mit dem ersten Kondensator der zweiten Schaltungs-Einheit derart koppelbar ist, dass das Potential der Sensor-Elektrode in dem vorgebbaren ersten Referenz-Bereich um das vorgebbare elektrische Soll-Potential gehalten wird.

Mit anderen Worten kann zum Konstanthalten des Potentials der Sensor-Elektrode die Kopplung der Sensor-Elektrode mit dem ersten Kondensator mittels eines regelbaren ohmschen Widerstands realisiert sein. Der jeweils aktuell eingestellte Wert des ohmschen Widerstands ist ein Maß für die aktuelle Stärke der elektrischen Kopplung zwischen der Sensor-Elektrode und dem ersten Kondensator.

Ferner weist die erste Schaltungseinheit vorzugsweise einen Transistor auf, dessen Gate-Bereich mit dem Ausgang des ersten Komparator-Elements gekoppelt ist, dessen erster Source-/Drain-Bereich mit der Sensor-Elektrode gekoppelt ist und dessen zweiter Source-/Drain-Bereich mit dem ersten Kondensator gekoppelt ist.

Mit anderen Worten fungiert der beschriebene Transistor als Regel-Element, der den Stromfluss zwischen der Sensor-Elektrode und dem ersten Kondensator einstellt.

Ferner kann die zweite Schaltungseinheit ein zweites Komparator-Element mit zwei Eingängen und einem Ausgang aufweisen, wobei der erste Eingang derart mit dem ersten Kondensator gekoppelt ist, dass der erste Eingang auf dem elektrischen Potential des ersten Kondensators ist, und wobei der zweite Eingang auf einem vierten elektrischen Referenzpotential ist, das den zweiten elektrischen Referenz-Bereich definiert. Das zweite Komparator-Element ist derart eingerichtet, dass an dessen Ausgang ein derartiges elektrisches Signal erzeugt wird, dass, wenn das elektrische Potential des ersten Kondensators das vierte elektrische Referenzpotential überschreitet, der erste Kondensator auf das erste elektrische Referenzpotential gebracht wird.

Alternativ zu der beschriebenen Ausgestaltung weist die zweite Schaltungseinheit der Schaltkreis-Anordnung ein zweites Komparator-Element mit zwei Eingängen und einem Ausgang auf, wobei der erste Eingang derart mit dem ersten Kondensator gekoppelt ist, dass der erste Eingang auf dem elektrischen Potential des ersten Kondensators ist, wobei der zweite Eingang auf einem vierten elektrischen Referenzpotential ist, das den zweiten elektrischen Referenz-Bereich definiert. Ferner ist das zweite Komparator-Element derart eingerichtet, dass an dessen Ausgang ein derartiges elektrisches Signal erzeugt wird, dass, wenn das elektrische Potential des ersten Kondensators das vierte elektrische Referenzpotential unterschreitet, der erste Kondensator auf das erste elektrische Referenzpotential gebracht wird.

Vorzugsweise ist das erste und/oder das zweite Komparator-Element ein Operationsverstärker.

Die obigen Ausführungen zeigen, dass die Elemente zum Ausbilden der erfindungsgemäßen Schaltkreis-Anordnung allesamt elektronische Standardbauteile sind, die in der Fertigung günstig sind und die mit Standardverfahren herstellbar sind. Daher ist die erfindungsgemäße Schaltkreis-Anordnung wenig aufwändig herstellbar.

Gemäß einer bevorzugten Weiterbildung der erfindungsgemäßen Schaltkreis-Anordnung weist ihre zweite Schaltungseinheit mindestens einen zweiten Kondensator auf, wobei die Schaltkreis-Anordnung derart eingerichtet ist, dass entweder einer der mindestens einen zweiten Kondensatoren oder der erste Kondensator oder mindestens zwei der Kondensatoren simultan der Schaltkreis-Anordnung zugeschaltet ist/sind.

Anschaulich weist die Schaltkreis-Anordnung mehrere parallel geschaltete Kondensatoren auf, die unterschiedliche oder gleiche Materialparameter (beispielsweise Kapazität C) aufweisen, und von denen jeweils einer oder mehrere wahlweise in die Schaltkreis-Anordnung aktiv eingeschaltet werden kann. Ein Benutzer hat daher die Möglichkeit, entsprechend der Bedürfnisse des Einzelfalls den oder die geeigneten der Kondensatoren auszuwählen, welcher oder welche hinsichtlich Messgenauigkeit und gewünschtem Dynamikbereich günstig ist oder sind. Mittels Bereitstellens unterschiedlicher Kondensatoren, von denen jeder in die Schaltkreis-Anordnung aktiv eingeschaltet werden kann, ist die Nachweisempfindlichkeit der Schaltkreis-Anordnung zum Erfassen elektrischer Ströme erhöht, und der Dynamikbereich ebenfalls erhöht.

Die erfindungsgemäße Schaltkreis-Anordnung kann als integrierter Schaltkreis ausgebildet sein.

Insbesondere kann die Schaltkreis-Anordnung der Erfindung in ein Halbleiter-Substrat (z.B. einen Chip eines Silizium-Wafers) integriert sein, bzw. teilweise auf dem Halbleiter-Substrat ausgebildet sein. Infolge der Integration der Schaltkreis-Anordnung ist die Sensitivität erhöht und die Schaltkreis-Anordnung miniaturisiert. Die Miniaturisierung bewirkt einen Kostenvorteil, da makroskopisches Messequipment eingespart ist. Ferner ist die erfindungsgemäße Schaltkreis-Anordnung mittels standardisierter halbleitertechnologischer Verfahren herstellbar, was sich ebenfalls günstig auf die Herstellungskosten auswirkt. Ferner ist es infolge der Integration der Schaltkreis-Anordnung in ein Halbleiter-Substrat ermöglicht, das zu erfassende Strom-Signal On-Chip, d.h. in unmittelbarer Nähe des Sensor-Ereignisses zu verarbeiten. Kurze Übermittlungswege des Strom-Signals halten Stör-Einflüsse wie Rauschen, etc. gering, so dass ein hohes Signal-Rausch-Verhältnis erreichbar ist.

Ferner ist erfindungsgemäß ein elektrochemischer Sensor mit einer Schaltkreis-Anordnung mit den beschriebenen Merkmalen geschaffen. Der elektrochemische Sensor kann insbesondere als Redox-Cycling-Sensor ausgestaltet sein.

Wie oben bezugnehmend auf **Fig.4A, Fig.4B, Fig.4C** beschrieben, weist ein auf dem Prinzip des Redox-Cyclings basierender Sensor eine zeitlich sukzessive ansteigende Sensor-Strom-Charakteristik auf. Ein solches, im Wesentlichen zeitlich monoton ansteigendes Stromsignal ist zum Erfassen mittels der erfindungsgemäßen Schaltkreis-Anordnung gut geeignet, da das sukzessive anwachsende Stromsignal in auf dem ersten Kondensator akkumulierte Ladungspakete zerlegbar ist, die mittels Pulsen einzeln von der erfindungsgemäßen Schaltkreis-Anordnung detektiert werden. Insbesondere ist die Nachweisempfindlichkeit der erfindungsgemäßen Schaltkreis-Anordnung ausreichend hoch, um elektrische Ströme in der Größenordnung zwischen ungefähr 1pA und ungefähr 100nA, wie sie häufig von Bio-Sensoren gemäß dem Redox-Cycling-Prinzip mit üblichen Sensor-Elektroden-Geometrien generiert werden, zu erfassen.

Darüber hinaus ist erfindungsgemäß eine Sensor-Anordnung mit einer Mehrzahl von Schaltkreis-Anordnungen mit den oben beschriebenen Merkmalen bereitgestellt.

Daher ist eine parallele Analyse, beispielsweise das parallele Erfassen unterschiedlicher DNA-Halbstränge mit einer Mehrzahl von Redox-Cycling-Sensoren, an deren Sensor-Elektroden unterschiedliche Fängermoleküle immobilisiert sind, möglich. Eine parallele Analyse einer zu untersuchenden Flüssigkeit ist ein dringendes Bedürfnis hinsichtlich vieler Anwendungen in der Bio- und Gentechnologie oder in der Lebensmitteltechnik. Eine zeitlich parallele Analyse ist zeit- und daher kostensparend. Ferner kann die Sensor-Anordnung derart eingerichtet sein, dass die einzelnen Sensorzellen (gebildet jeweils von einer Schaltkreis-Anordnung) seriell auslesbar sind.

Insbesondere kann bei der Sensor-Anordnung jede der Schaltkreis-Anordnungen als autark arbeitendes Sensor-Element eingerichtet sein.

Die Schaltkreis-Anordnungen der Sensor-Anordnung können im Wesentlichen matrixförmig, alternativ jedoch auch z.B. hexagonal, angeordnet sein.

Ferner kann die Sensor-Anordnung einen zentralen Ansteuer-Schaltkreis zum Ansteuern einer Schaltkreis-Anordnung, einen zentralen Versorgungs-Schaltkreis zum Bereitstellen von Versorgungs-Spannungen bzw. Versorgungs-Strömen und/oder einen zentralen Auslese-Schaltkreis zum Auslesen der Schaltkreis-Anordnungen aufweisen. Dieser Schaltkreis bzw. diese Schaltkreise sind vorzugsweise mit zumindest einem Teil der Schaltkreis-Anordnungen gekoppelt.

Im Weiteren wird das erfindungsgemäße Verfahren zum Verarbeiten eines über eine Sensor-Elektrode bereitgestellten Stromsignals beschrieben. Ausgestaltungen der Schaltkreis-Anordnung, des elektrochemischen Sensors und der Sensor-Anordnung gelten auch für das Verfahren zum Verarbeiten eines über eine Sensor-Elektrode bereitgestellten Stromsignals.

Das erfindungsgemäße Verfahren zum Verarbeiten eines über eine Sensor-Elektrode bereitgestellten Stromsignals erfolgt unter Verwenden einer erfindungsgemäßen Schaltkreis-Anordnung mit den oben beschriebenen Merkmalen. Gemäß dem Verfahren wird das elektrische Potential der Sensor-Elektrode in dem vorgebbaren ersten Referenz-Bereich um das vorgebbare elektrische Soll-Potential gehalten, indem der erste Kondensator und die Sensor-Elektrode derart gekoppelt werden, dass ein Angleichen des elektrischen Potentials ermöglicht ist. Ferner wird, wenn das elektrische Potential des ersten Kondensators außerhalb des zweiten Referenz-Bereichs gerät, mittels der zweiten Schaltungseinheit dieses Ereignis detektiert und der erste Kondensator auf das erste elektrische Referenzpotential gebracht.

Gemäß einer bevorzugten Weiterbildung des erfindungsgemäßen Verfahrens wird mittels eines mit der zweiten Schaltungseinheit elektrisch gekoppelten Zähler-Elements die Anzahl und/oder die zeitliche Abfolge der Ereignisse gezählt.

Vorzugsweise wird mittels des Zähler-Elements die zeitliche Abfolge der Ereignisse in mindestens zwei Zeitintervallen in einem zeitlichen Abstand voneinander erfasst.

Gemäß einer anderen Ausgestaltung der Erfindung ist es vorgesehen, dass die Sensor-Elektrode als Generator-Elektrode eingerichtet ist. Ferner ist eine Kollektor-Elektrode vorgesehen. Die Schaltkreisanordnung weist ferner eine dritte Schaltungseinheit auf, die mit der Kollektor-Elektrode elektrisch gekoppelt ist. Eine vierte Schaltungseinheit weist einen zweiten Kondensator auf. Die dritte Schaltungseinheit ist derart eingerichtet, dass sie das elektrische Potential der Kollektor-Elektrode in einem vorgebbaren zweiten Referenz-Bereich um ein vorgebbares elektrisches zweites Soll-Potential hält, in dem der zweite Kondensator und die Kollektor-Elektrode derart gekoppelt werden, dass ein Angleichen des elektrischen Potentials der Kollektor-Elektrode möglich ist. Die zweite Schaltungseinheit und die vierte Schaltungseinheit sind derart eingerichtet, dass sie, wenn das elektrische Potential des zweiten Kondensators außerhalb eines zweiten Referenz-Bereichs ist, dieses Ergebnis detektieren und den zweiten Kondensator auf ein zweites elektrisches Referenzpotential bringen. Die Anzahl und/oder die zeitliche Abfolge der Ereignisse wird mittels eines mit der zweiten Schaltungseinheit und der vierten Schaltungseinheit elektrisch gekoppelten Zähler-Elements gezählt.

Der Vorteil dieser Ausgestaltung ist die Verbesserung des Signal/Rauschverhältnisses, weil die Information an zwei Elektroden ausgewertet wird.

Es ist jedoch nicht zwingend nötig, die Signale an beiden Elektroden unabhängig voneinander zu messen. Deshalb werden zwei weitere Ausführungsformen angegeben, die die Summe (genauer: die betragsmäßige Summe) der Signale an beiden Elektroden, d.h. an der Generator-Elektrode und an der Kollektor-Elektrode, auswerten. Anschaulich ist die Grundlage für diese Ausgestaltungen der Erfindung die Erkenntnis, dass beim Redox-Cycling-Verfahren die elektrischen Ströme an beiden Elektroden, das heißt an der Generator-Elektrode und an der Kollektor-Elektrode im Prinzip die gleiche Information tragen und deshalb nicht separat verarbeitet werden müssen. Eine Beschränkung der Auswertung auf ein Signal würde jedoch das Signal-/Rauschverhältnis verschlechtern.

Der Ausdruck "Summe" ist derart zu verstehen, dass auch der Fall mit umfasst ist, bei dem beispielsweise für Testzwecke und für Grundsatzuntersuchungen die Schaltungen auch die Möglichkeit bieten, die Elektroden einzeln (aber nicht gleichzeitig) zu messen und deren Signale in der jeweiligen Schaltung auszuwerten.

Gemäß dieser Ausgestaltungen der Erfindung wird es ermöglicht, wie im Folgenden noch näher erläutert wird, einen Komparator und ein Zähler-Element, einzusparen und damit die für den Sensor und die Auswerteschaltung benötigte Fläche auf einem Chip erheblich zu reduzieren. Dies erlaubt den Aufbau von erheblich dichteren Sensor-Arrays.

Gemäß der ersten Ausgestaltung der Erfindung zur Messung des Summensignals ist ein mit dem ersten Kondensator gekoppelter dritter Kondensator vorgesehen, welcher eine größere Kapazität aufweist als der erste Kondensator, wobei der erste Kondensator und der dritte Kondensator einen kapazitiven ersten Spannungsteiler bilden. Ferner ist ein mit dem zweiten Kondensator gekoppelter vierter Kondensator vorgesehen, wobei der vierte Kondensator eine größere Kapazität aufweist als der zweite Kondensator. Der zweite Kondensator und der vierte Kondensator bilden einen kapazitiven zweiten Spannungsteiler.

Bevorzugt ist die Kapazität des dritten Kondensators mindestens um den Faktor zwei größer als die Kapazität des ersten Kondensators und die Kapazität des vierten Kondensators ist mindestens um den Faktor zwei größer als die Kapazität des zweiten Kondensators. Besonders bevorzugt ist die Kapazität des dritten Kondensators mindestens um den Faktor zehn größer als die Kapazität des ersten Kondensators und die Kapazität des vierten Kondensators ist ebenfalls mindestens um den Faktor zehn größer als die Kapazität des zweiten Kondensators.

Anschaulich wird gemäß dieser Ausgestaltung somit der Strom für die Generator-Elektrode dem ersten kapazitiven Spannungsteiler entnommen und der Strom für die Kollektor-Elektrode dem kapazitiven zweiten Spannungsteiler. Für den Fall, dass die Kapazität des dritten Kondensators erheblich größer als die des ersten sowie die Kapazität des vierten Kondensators erheblich größer als die des zweiten Kondensators ist, ist das Potential, das heißt der Spannungshub, auf den Knoten auf beiden Seiten des dritten Kondensators bzw. des vierten Kondensators im Wesentlichen gleich, da die beiden Knoten kapazitiv stark gekoppelt sind.

Die zweite Schaltungseinheit und die vierte Schaltungseinheit weisen gemeinsam ein Summen-Komparator-Element auf mit zwei Eingängen und einem Ausgang, wobei

- ein erster Eingang zwischen den ersten Kondensator und den dritten Kondensator geschaltet ist,
- ein zweiter Eingang zwischen den zweiten Kondensator und den vierten Kondensator geschaltet ist, und
- der Ausgang mit einem Zähler-Element gekoppelt ist, das derart eingerichtet ist, dass es die Anzahl und/oder die zeitliche Abfolge der Ereignisse zählt.

Gemäß der zweiten Ausgestaltung der Erfindung zur Messung des Summensignals ist es vorgesehen, dass

- die Generator-Elektrode, wie oben beschrieben, mit einer erste Schaltungseinheit zur Regelung des elektrischen Potentials verbunden ist; des weiteren wird mittels eines ersten Kondensators sowie einer zweiten Schaltungseinheit, wie oben beschrieben, eine erste Pulsfolge am Ausgang der zweiten Schaltungseinheit erzeugt;
- die Kollektor-Elektrode, wie oben beschrieben, mit einer dritten Schaltungseinheit zur Regelung des elektrischen Potentials verbunden ist; des weiteren wird mittels eines zweiten Kondensators, sowie einer vierten Schaltungseinheit eine zweite Pulsfolge am Ausgang der vierten Schaltungseinheit erzeugt.
- Ferner ist ein Synchronisationselement mit zwei Eingängen und einem Ausgang vorgesehen, wobei
- die erste Pulsfolge, d.h. das erste Signal, an einem ersten Eingang anliegt, d.h. der erste Eingang ist mit dem Ausgang der zweiten Schaltungseinheit gekoppelt,
- die zweite Pulsfolge, d.h. das zweite Signal, an einem zweiten Eingang anliegt, d.h. der zweite Eingang ist mit dem Ausgang der vierten Schaltungseinheit gekoppelt.

Das Synchronisationselement ist derart eingerichtet, dass bei überlappenden Pulsen, d.h. einander überlappenden Signalen, eine der Pulsfolgen soweit verzögert wird, dass die Überlappung aufgelöst wird. Aus zwei überlappenden Pulsen an den Eingängen entsteht so ein Doppelpuls am Ausgang des Synchronisationselements. Das Synchronisations-Element weist bevorzugt einen Pufferspeicher auf.

Ausführungsbeispiele der Erfindung sind in den Figuren dargestellt und werden im Weiteren näher erläutert.

Es zeigen:

Figur 1           eine schematische Ansicht einer Schaltkreis-Anordnung gemäß einem ersten Ausführungsbeispiel der Erfindung,

Figur 2A           eine Querschnitts-Ansicht eines Sensors gemäß dem Stand der Technik in einem ersten Betriebszustand,

Figur 2B           eine Querschnitts-Ansicht des Sensors gemäß dem Stand der Technik in einem zweiten Betriebszustand,

Figur 3A           eine Draufsicht von Interdigitalelektroden gemäß dem Stand der Technik,

| Figur 3B | eine Querschnittsansicht entlang der Schnittlinie I-I' der in Figur 3A gezeigten Interdigitalelektroden gemäß dem Stand der Technik, |
| --- | --- |
| Figur 4A | einen auf dem Prinzip des Redox-Cyclings basierenden Biosensor in einem ersten Betriebszustand gemäß dem Stand der Technik, |
| Figur 4B | einen auf dem Prinzip des Redox-Cyclings basierenden Biosensor in einem zweiten Betriebszustand gemäß dem Stand der Technik, |
| Figur 4C | einen auf dem Prinzip des Redox-Cyclings basierenden Biosensor in einem dritten Betriebszustand gemäß dem Stand der Technik, |
| Figur 5 | einen Funktionsverlauf eines Sensor-Stroms im Rahmen eines Redox-Cycling-Vorgangs, |
| Figur 6A | eine schematische Ansicht einer Schaltkreis-Anordnung gemäß einem zweiten Ausführungsbeispiel der Erfindung, |
| Figur 6B | eine schematische Ansicht einer Schaltkreis-Anordnung gemäß einem dritten Ausführungsbeispiel der Erfindung, |
| Figur 7 | einen Blockschaltplan einer Schaltkreis-Anordnung gemäß einem vierten Ausführungsbeispiel der Erfindung, |
| Figur 8 | einen Blockschaltplan, der den Aufbau einer in Figur 7 gezeigten ersten Schaltungseinheit (Spannungsregler) zeigt, |
| Figur 9 | einen weiteren Blockschaltplan, der den Aufbau des in Figur 8 gezeigten ersten Komparator-Elements zeigt, |
| Figur 10 | einen weiteren Blockschaltplan, der den Aufbau eines in Figur 7 gezeigten zweiten Komparator-Elements zeigt, |
| Figur 11 | einen weiteren Blockschaltplan, der den Aufbau einer Stufe des Zählers bzw. des Schieberegisters aus Figur 7 zeigt, |
| Figur 12 | ein bevorzugtes Ausführungsbeispiel der erfindungsgemäßen Sensor-Anordnung. |
| Figur 13 | eine Schaltungsanordnung gemäß einem ersten Ausführungsbeispiel der Erfindung; |
| Figur 14 | eine Schaltungsanordnung gemäß einem zweiten Ausführungsbeispiel der Erfindung; |
| Figuren 15a und 15b | Spannungsverläufe an den Knoten K1 bis K4 aus der Schaltungsanordnung gemäß Figur 14 über die Zeit (Figur 15b) und des Rücksetzpulses (Figur 15a); und |
| Figur 16 | eine Schaltungsanordnung gemäß einem dritten Ausführungsbeispiel der Erfindung. |

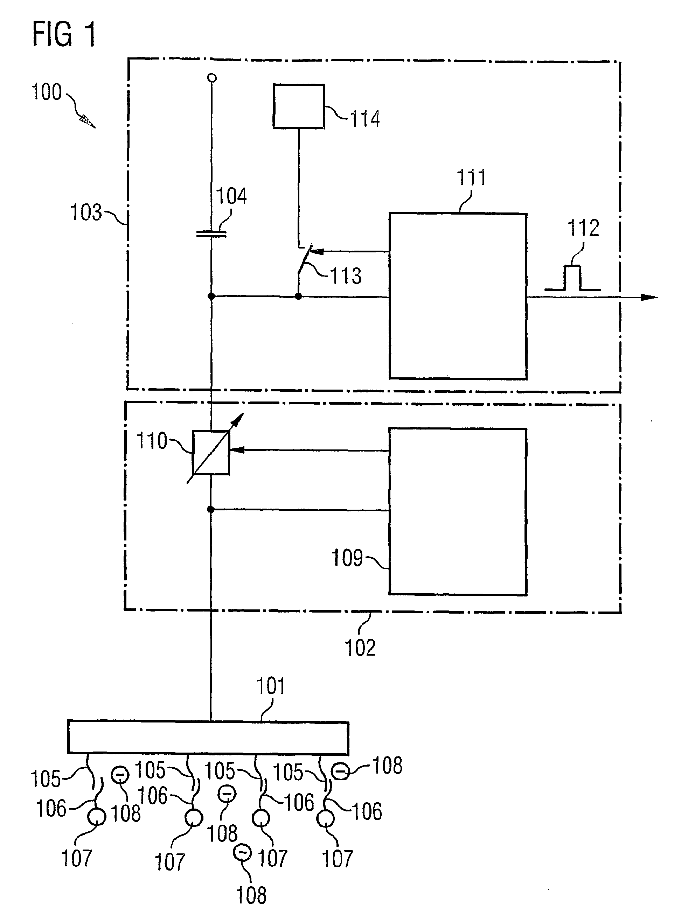

Im Weiteren wird bezugnehmend auf **Fig.1** ein erstes bevorzugtes Ausführungsbeispiel der erfindungsgemäßen Schaltkreis-Anordnung beschrieben.

Die in **Fig.1** gezeigte Schaltkreis-Anordnung 100 weist eine Sensor-Elektrode 101, eine erste Schaltungseinheit 102, die mit der Sensor-Elektrode 101 elektrisch gekoppelt ist und eine zweite Schaltungseinheit 103, die einen ersten Kondensator 104 aufweist, auf. Die erste Schaltungseinheit 102, anschaulich ein Potentiostat, ist derart eingerichtet, dass sie das elektrische Potential der Sensor-Elektrode 101 in einem vorgebbaren ersten Referenz-Bereich um ein vorgebbares elektrisches Soll-Potential hält, indem der erste Kondensator 104 und die Sensor-Elektrode 101 derart gekoppelt werden, dass ein Angleichen des elektrischen Potentials (mittels eines Stromflusses zum Regeln) ermöglicht ist. Ferner ist die zweite Schaltungseinheit 103 derart eingerichtet, dass sie, wenn das elektrische Potential des ersten Kondensators 104 außerhalb eines zweiten Referenz-Bereichs ist, dieses Ereignis detektiert und den ersten Kondensator 104 auf ein erstes elektrisches Referenzpotential bringt.

Wie ferner in **Fig.1** gezeigt, sind an der Oberfläche der Sensor-Elektrode 101 Fängermoleküle 105 immobilisiert.

Die Fängermoleküle 105 aus **Fig.1** haben mit zu erfassenden Molekülen 106 hybridisiert, wobei jedes der zu erfassenden Moleküle 106 ein Enzymlabel 107 aufweist.

Die in **Fig.1** gezeigte Sensor-Elektrode 101 mit den daran immobilisierten Fängermolekülen funktioniert nach dem Prinzip des Redox-Cyclings (vgl. **Fig.4A, Fig.4B, Fig.4C**). Daher sind in **Fig.1** elektrisch geladene Partikel 108 gezeigt, welche mittels der Enzymlabel 107 in der zu untersuchenden Flüssigkeit erzeugt werden, und welche einen elektrischen Sensor-Strom erzeugen, der von der ersten Sensor-Elektrode 101 aus in die Schaltkreis-Anordnung 100 eingekoppelt wird.

Dieser Sensor-Strom verändert das elektrische Potential der Sensor-Elektrode 101 in charakteristischer Weise. Dieses elektrische Potential liegt am Eingang einer ersten Regelungs-Einheit 109 der ersten Schaltungseinheit 102 an. Die erste Schaltungseinheit 102 und insbesondere die erste Regelungs-Einheit 109 sorgen dafür, dass die Sensor-Elektrode 101 auf einem vorgebbaren, konstanten elektrischen Potential verbleibt, indem bei einem ausreichend starken Abweichen des Sensor-Elektroden-Potentials von dem elektrischen Soll-Potential eine Verschiebung von Ladungsträgern zwischen dem ersten Kondensator 104 und der Sensor-Elektrode 101 durchgeführt wird. Dies ist in **Fig.1** schematisch mittels des regelbaren ohmschen Widerstands 110, der von der ersten Regelungs-Einheit 109 regelbar ist, angedeutet Der gezeigte Schaltungsblock ist ein analoger Regelkreis, der den Stromfluss zwischen dem Kondensator 104 und der Sensor-Elektrode 101 so regelt, dass die Spannung an der Sensor-Elektrode 101 konstant bleibt. Mittels des steuerbaren Widerstands 110 ist eine kontinuierliche Regelung des Stromflusses ermöglicht. Gerät das elektrische Potential der Sensor-Elektrode 101 infolge einer ausreichend großen Anzahl von Sensor-Ereignissen an deren Oberfläche außerhalb des ersten Referenz-Bereichs, so sorgt die erste Schaltungseinheit 102 und insbesondere die erste Regelungs-Einheit 109 dafür, dass der Stromfluss zwischen der Sensor-Elektrode 101 und dem ersten Kondensator 104 zunimmt bzw. abnimmt, so dass zwischen dem ersten Kondensator 104 und der Sensor-Elektrode 101 ein Angleichen des elektrischen Potentials ermöglicht ist. Anschaulich wird dadurch mittels der ersten Regelungs-Einheit 109 der ersten Schaltungseinheit 102 der Widerstandswert des steuerbaren Widerstandes 110 erhöht bzw. erniedrigt, so dass ein Stromfluss zwischen der Sensor-Elektrode 101 und dem ersten Kondensator 104 ermöglicht ist. In diesem Szenario kann elektrische Ladung zwischen dem ersten Kondensator 104 und der Sensor-Elektrode 101 hin- und her- fließen.

Wenn infolge dieser Ladungsverschiebung das elektrische Potential des ersten Kondensators 104 außerhalb eines zweiten Referenz-Bereichs gerät, so wird dieses Ereignis von der zweiten Schaltungseinheit 103 und insbesondere von einer zweiten Regelungs-Einheit 111, die vorzugsweise einen Komparator aufweist, der zweiten Schaltungseinheit 103 detektiert. Wie in **Fig.1** gezeigt, kann dieses Detektieren darin bestehen, dass an einem Ausgang der zweiten Regelungs-Einheit 111 ein elektrischer Puls 112 generiert wird.

Ferner wird, wenn das elektrische Potential des ersten Kondensators 104 außerhalb des zweiten Referenz-Bereichs gerät, mittels der zweiten Schaltungseinheit 103 und insbesondere mittels der zweiten Regelungs-Einheit 111 der zweiten Schaltungseinheit 103 der erste Kondensator 104 auf das erste elektrische Referenzpotential gebracht. Dies ist in **Fig.1** dadurch angedeutet, dass ein weiterer Schalter 113 infolge eines von der zweiten Regelungs-Einheit 111 der zweiten Schaltungseinheit 103 ausgelösten Signals geschlossen wird, wodurch der erste Kondensator 104 mit einer Spannungsquelle 114 elektrisch gekoppelt wird, wodurch der erste Kondensator 104 auf das erste elektrische Referenzpotential gebracht wird, das mittels der Spannungsquelle 114 definiert ist.

Eine Grundidee der erfindungsgemäßen Schaltkreis-Anordnung kann anschaulich darin gesehen werden, dass ein zu erfassender Sensor-Strom ohne eine vorherige analoge Verstärkung in eine dem Strom proportionale Frequenz überführt wird. Mittels der erfindungsgemäßen Schaltkreis-Anordnung wird das Potential an der Sensor-Elektrode konstant gehalten und die hierfür erforderliche elektrische Ladung (positiven oder negativen Vorzeichens) einem Kondensator mit der Kapazität C entnommen. Infolge der Ladungsentnahme ∆Q

$$\Delta Q \;=\; \int I\,dt \qquad (1)$$

aufgrund eines Stromflusses I zwischen dem ersten Kondensator und der Sensor-Elektrode integriert über die Zeit t, ändert sich die an dem ersten Kondensator anliegende Spannung ∆U gemäß der Beziehung

$$\Delta Q \;=\; C\Delta U \qquad (2)$$

Die an dem Kondensator anliegende Spannung wird mittels einer Schwellwertschaltung überwacht. Wird ein bestimmter Wert über- oder unterschritten, so löst die Schaltung einen digitalen Impuls aus, mittels dem ein Schalter geschlossen wird, wodurch die elektrische Spannung an dem Kondensator auf einen vorgegebenen Wert zurückgesetzt wird. Als Resultat erhält man im Messbetrieb eine Impulsfolge aus der Schwellwertschaltung, deren Frequenz proportional zu dem Signal-Strom ist.

Wie oben bezugnehmend auf **Fig.1** beschrieben, weist die erfindungsgemäße Schaltkreis-Anordnung zum Betrieb eines elektrochemischen Sensors im Wesentlichen zwei Schaltungseinheiten auf. Die erste Schaltungseinheit kontrolliert das elektrische Potential (d.h. die Spannung gegenüber einem Bezugspunkt), das an der Sensor-Elektrode anliegt. Beispielsweise kann ein Operationsverstärker dazu verwendet werden, das elektrische Potential der Sensor-Elektrode mit einem Referenzpotential zu vergleichen, und den elektrischen Stromfluss zwischen der Sensor-Elektrode und dem ersten Kondensator derart zu regeln, dass das elektrische Potential der Sensor-Elektrode konstant bleibt.

Der zum Angleich des Sensor-Stroms erforderliche Gegenstrom wird, wie beschrieben, dem ersten Kondensator der zweiten Schaltungseinheit entnommen. Die Spannung an dem ersten Kondensator wird in der zweiten Schaltungseinheit von einer Schwellwertschaltung, beispielsweise einer Komparatorschaltung, überwacht. Bei Über- oder Unterschreiten eines zweiten Referenz-Bereichs des elektrischen Potentials, auf dem sich der erste Kondensator befindet, gibt die zweite Schaltungseinheit einen Rücksetzimpuls aus. Dieser digitale Impuls, der vorzugsweise eine feste zeitliche Länge aufweist, setzt das Potential des Kondensators (bzw. die elektrische Spannung zwischen den beiden Kondensatorplatten) auf ein erstes elektrisches Referenzpotential zurück. Der Impuls sollte eine konstante Länge aufweisen, da während dieser Zeit der Gegenstrom einer Spannungsquelle entnommen wird. Diese Totzeit reduziert die gemessene Frequenz und ist, sofern die Totzeit nicht vernachlässigbar klein ist, bei der Auswertung der Daten zu berücksichtigen.

Um in einem Szenario, in dem die Totzeit nicht vernachlässigbar ist bzw. kompensiert werden soll, den Messfehler durch das Rücksetzen der Schaltung zu minimieren, können zwei (oder mehr) Kondensatoren vorgesehen werden, die wechselweise in der beschriebenen Art betrieben werden. Wird der eine (aktive) Kondensator durch den Sensor-Strom geladen, so wird der andere (passive) Kondensator in diesem Zeitintervall auf das erste elektrische Referenzpotential zurückgesetzt. Überschreitet das Potential am aktiven Kondensator den vorgegebenen Wert, so wird vorzugsweise durch die zweite Schaltungs-Einheit 103 nicht unmittelbar ein Rücksetzimpuls ausgelöst, sondern zwischen den beiden Kondensatoren zunächst umgeschaltet und erst anschließend der nun passive Kondensator zurückgesetzt. Mittels dieser Vorgehensweise wird der Sensorstrom zu keinem Zeitpunkt einer Spannungsquelle direkt entnommen, sondern stets einem Kondensator, der als Ladungsreservoir dient.

Wiederum bezugnehmend auf **Fig.1** erfolgt der Rücksetzvorgang vorzugsweise mittels eines Schalttransistors, der den ersten Kondensator in der Rücksetzphase auf ein vorgebbares Potential entlädt (zum Beispiel völlig entlädt). Das erste elektrische Referenzpotential ist vorzugsweise ein MassePotential. Der Sensor-Strom lädt den ersten Kondensator anschließend wieder auf. Die zeitliche Abhängigkeit der elektrischen Spannung an dem ersten Kondensator ist durch folgenden Ausdruck beschreibbar:

$$U(t) = 1/C \int_0^t I_{Sensor} dt' \qquad (3)$$

Der von der Sensor-Elektrode abgeleitete Sensor-Strom $I_{Sensor}$ weist, wie oben bezugnehmend auf **Fig.5** beschrieben, einen konstanten Offset-Anteil $T_{Offset}$ und einen (idealerweise) linear mit der Zeit ansteigendem Signal-Strom auf:

$$I_{Sensor} = I_{Offset} + mt \qquad (4)$$

Setzt man Gleichung (4) in Gleichung (3) ein und berechnet man das Integral, so ergibt sich für die elektrische Spannung, die sich zwischen einem ersten Zeitpunkt $t_1$ und einem zweiten Zeitpunkt $t_2$ aufbaut, zu:

$$U(t) = 1/C (I_{Offset}[t_2-t_1] + m/2[t_2^2 - t_1^2]) \qquad (5)$$

Das Zeitintervall $\Delta t$, in der eine bestimmte Spannungsdifferenz $\Delta U$ aufgebaut wird, ist daher:

$$\Delta t \;=\; t_2 - t_1 \;=\; (C\Delta U)/(I_{Offset} + mt) \qquad (6)$$

Dabei ist t die mittlere Zeit des betrachteten Intervalls, d.h.

$$t \;=\; (t_1 + t_2)/2 \qquad (7)$$

Die innerhalb eines ausreichend kurzen Intervalls $\Delta t$ gemessene Frequenz f unter Vernachlässigung einer Totzeit $t_{Tot}$ beim Rücksetzen des Kondensators ($t_{Tot} \ll \Delta t$) ergibt sich demnach zu:

$$f \;=\; \Delta t^{-1} \;=\; I_{Offset}/(C\Delta U) + mt/(C\Delta U) \qquad (8)$$

Diese Frequenz f kann als digitales Signal direkt von der Schaltkreis-Anordnung weggeleitet (beispielsweise von einem Chip, falls die Schaltkreis-Anordnung in ein Halbleiter-Substrat integriert ist) und weiterverarbeitet bzw. ausgewertet werden. Gleichung (8) zeigt, dass die Frequenz f einen konstanten Anteil aufweist, der auf den Offset-Strom $I_{Offset}$ der Sensor-Elektrode zurückgeht. Der zweite Term in (8) repräsentiert den linear mit der Zeit ansteigenden Frequenzanteil (die Annahme eines exakt linear ansteigenden Stromsignals ist selbstverständlich idealisierend), der auf Sensor-Ereignisse gemäß dem Redox-Cycling-Prinzip zurückgeht, und der die eigentliche Messgröße m beinhaltet.
Die messtechnisch relevante Größe m erhält man, indem beispielsweise zwei Perioden- oder Frequenzmessungen mit einem vorgegebenen Zeitabstand $\Delta t_{Mess} = t_B - t_A$ durchgeführt werden. Setzt man $t_A$ bzw. $t_B$ in Gleichung (8) ein und subtrahiert man die daraus erhaltenen Frequenzen $f_A$ und $f_B$ voneinander, so erhält man als Frequenzunterschied $\Delta f$:

$$\Delta f \;=\; f_B - f_A \;=\; m\Delta t_{Mess}/(C\Delta U) \qquad (9)$$

Daraus ergibt sich die messtechnisch relevante Größe m zu:

$$m \;=\; \Delta f C\Delta U/\Delta t_{Mess} \qquad (10)$$

Aus zwei Messungen der Ausgangsfrequenz des Sensors kann demnach direkt die messtechnisch relevante Größe m, anschaulich die Steigung des Kurvenverlauf Strom-Zeit 503 aus **Fig.5,** ermittelt werden.
Alternativ zu der beschriebenen Frequenz- oder Periodendauer-Messung ist es möglich, die Pulse der zweiten Schaltungseinheit dem Eingang eines Zähler-Elements bereitzustellen, das die Anzahl bzw. die zeitliche Abfolge der Pulse summiert und vorzugsweise in ein Binärwort umwandelt, welches die Anzahl der verstrichenen Zeitintervalle $\Delta t$ kodiert.
Ein derartiges Zähler-Element kann eine vorgegebene Zeit lang die Rücksetz-Pulse des ersten Kondensators zählen, nach einem externen Puls den Zählerstand digital ausgeben und anschließend das Zähler-Element zurücksetzen.
Der Zählerstand n des Zähler-Elements der Schaltkreis-Anordnung nach Ablauf des mittels der Zeitpunkte $t_{c1}$ und $t_{c2}$ definierten Zeitraums $t_{Count} = t_{c2} - t_{c1}$ berechnet sich in guter Näherung zu:

$$n \;=\; \int_{t_{c1}}^{t_{c2}} f \, d\underline{t} \;=\; I_{Offset}(t_{c2} - t_{c1})/(C\Delta U) + m(t_{c2}{}^2 - t_{c1}{}^2)/(2C\Delta U) \qquad (11)$$

Entsprechend dem oben bezugnehmend auf das Ermitteln von m aus Frequenzmessungen Dargestellten sind mindestens zwei Messungen der Zählerstände n erforderlich, aus denen mittels Gleichung (11) sowohl $I_{Offset}$ als auch die messtechnisch relevante Größe m bestimmt werden können.

Ein Vorteil der Integration eines Zähler-Elements in die Schaltkreis-Anordnung der Erfindung ist die daraus resultierende automatisch erfolgende zeitliche Mittelung des Messergebnisses. Da bei den - insbesondere beim Nachweis von Biomolekülen - zu erwartenden kleinen Sensor-Strömen Fluktuationen im Momentanwert der Messgröße möglich sind (beispielsweise infolge von Rauscheffekten etc.) ist eine Mittelung besonders vorteilhaft.

Gemäß einem bevorzugten Ausführungsbeispiel der erfindungsgemäßen Schaltkreis-Anordnung weist die zweite Schaltungseinheit mindestens einen zweiten Kondensator auf, wobei die Schaltkreis-Anordnung derart eingerichtet ist, dass entweder einer der mindestens einen zweiten Kondensatoren oder der erste Kondensator oder mindestens zwei der Kondensatoren simultan der Schaltkreis-Anordnung zugeschaltet ist/sind.

Zur Erweiterung des Dynamikbereichs und zur Verbesserung der Messgenauigkeit ist anschaulich eine umschaltbare Speicherkapazität bereitgestellt. Liefert die Sensor-Elektrode einen erhöhten elektrischen Sensor-Strom, was eine erhöhte Ausgangsfrequenz zur Folge hätte, kann dem ersten Kondensator beispielsweise ein weiterer parallel zugeschaltet werden. Dadurch reduziert sich die Ausgangsfrequenz und damit eventuelle Messungenauigkeiten aufgrund der Totzeit beim Rücksetzen des ersten Kondensators. Neben der so realisierten Messbereichsumschaltung kann auch das Intervall $\Delta U$, innerhalb dem die Kondensator-Spannung pendelt, verändert werden. Dies erlaubt eine kontinuierliche Durchstimmung des Messbereichs.

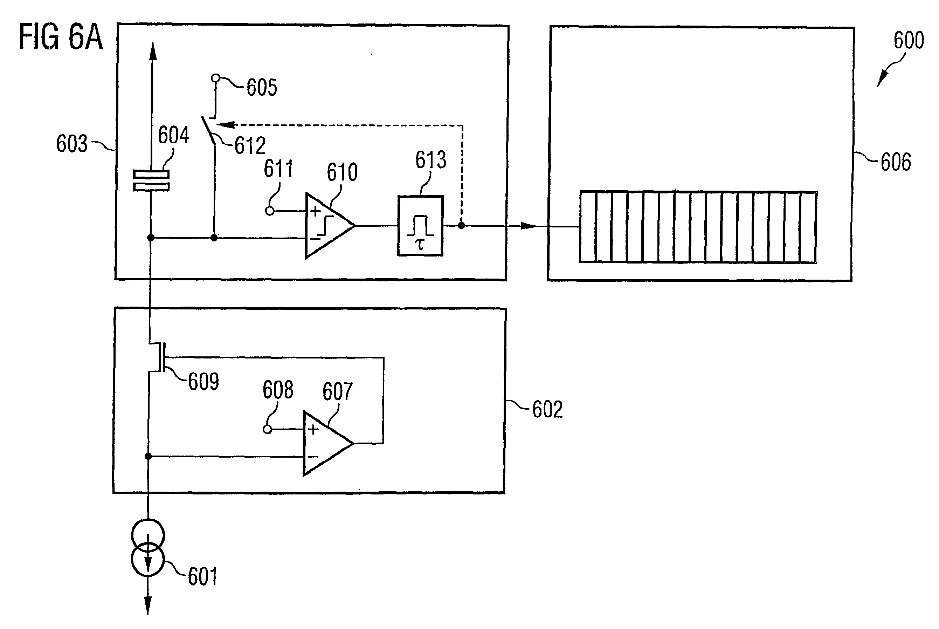

Im Weiteren wird bezugnehmend auf **Fig.6A** eine Schaltkreis-Anordnung 600 gemäß einem zweiten bevorzugten Ausführungsbeispiel der Erfindung beschrieben.

Die Schaltkreis-Anordnung 600 weist eine Sensor-Elektrode 601, eine erste Schaltungseinheit 602, die mit der Sensor-Elektrode 601 gekoppelt ist und eine zweite Schaltungseinheit 603, die einen ersten Kondensator 604 aufweist, auf. Die erste Schaltungseinheit 602 ist derart eingerichtet, dass sie das elektrische Potential der Sensor-Elektrode 601 in einem vorgebbaren ersten Referenz-Bereich um ein vorgebbares elektrisches Soll-Potential hält, indem der erste Kondensator 604 und die Sensor-Elektrode 601 derart gekoppelt werden, dass ein Angleichen des elektrischen Potentials ermöglicht ist. Ferner ist die zweite Schaltungseinheit 603 derart eingerichtet, dass sie, wenn das elektrische Potential des ersten Kondensators 604 außerhalb eines zweiten Referenz-Bereichs ist, dieses Ereignis detektiert und den ersten Kondensator 604 auf ein erstes elektrisches Referenzpotential, bereitgestellt von der ersten Spannungsquelle am Knoten 605, der zweiten Schaltungseinheit 603, bringt.

Darüber hinaus weist die Schaltkreis-Anordnung 600 ein mit der zweiten Schaltungseinheit 603 elektrisch gekoppeltes Zähler-Element 606 auf, das derart eingerichtet ist, dass es die Anzahl und die zeitliche Abfolge der Ereignisse zählt.

Darüber hinaus weist die erste Schaltungseinheit 602 ein erstes Komparator-Element 607 mit zwei Eingängen und einem Ausgang auf, wobei der erste Eingang derart mit der Sensor-Elektrode 601 gekoppelt ist, dass der erste Eingang auf dem elektrischen Potential der Sensor-Elektrode 601 ist. Der zweite Eingang ist auf ein drittes elektrisches Referenzpotential gebracht, welches das elektrische Soll-Potential (bzw. den ersten elektrischen Referenz-Bereich) definiert. Das dritte elektrische Referenzpotential, auf dem der zweite Eingang des ersten Komparator-Elements 607 befindlich ist, ist von einer zweiten Spannungsquelle 608 bereitgestellt. Ferner ist das erste Komparator-Element 607 derart eingerichtet, dass an dessen Ausgang ein derartiges elektrisches Signal erzeugt wird, dass das elektrische Potential der Sensor-Elektrode 601 in dem vorgebbaren ersten Referenz-Bereich um das vorgebbare elektrische Soll-Potential gehalten wird.

Wie ferner in **Fig.6A** gezeigt, weist die erste Schaltungseinheit 602 einen Transistor 609 auf, dessen Gate-Bereich mit dem Ausgang des ersten Komparator-Elements 607 gekoppelt ist, dessen erster Source-/Drain-Bereich mit der Sensor-Elektrode 601 gekoppelt ist und dessen zweiter Source-/Drain-Bereich mit dem ersten Kondensator 604 gekoppelt ist.

Anschaulich ist der Feldeffekttransistor 609 ein veränderbarer (von dem ersten Komparator-Element 607 steuerbarer) ohmscher Widerstand, mittels welchem die Sensor-Elektrode 601 mit dem ersten Kondensator 604 der zweiten Schaltungs-Einheit 603 derart koppelbar ist, dass das elektrische Potential der Sensor-Elektrode 601 in dem vorgebbaren ersten Referenz-Bereich um das vorgebbare elektrische Soll-Potential gehalten wird. Mit anderen Worten kann mittels des Transistors 609 jeder Zwischenwert zwischen vollständiger Kopplung und vollständiger Entkopplung von Sensor-Elektrode 601 und Kondensator 604 eingestellt werden.

Ferner weist die zweite Schaltungseinheit 603 ein zweites Komparator-Element 610 mit zwei Eingängen und einem Ausgang auf, wobei der erste Eingang derart mit dem ersten Kondensator 604 gekoppelt ist, dass der erste Eingang auf dem elektrischen Potential des ersten Kondensators 604 ist, und wobei der zweite Eingang auf einem vierten elektrischen Referenzpotential ist, das von einer dritten Spannungsquelle 611 bereitgestellt ist, welches vierte elektrische Referenzpotential den zweiten elektrischen Referenz-Bereich definiert. Das zweite Komparator-Element 610 ist derart eingerichtet, dass an dessen Ausgang ein derartiges elektrisches Signal erzeugt wird, dass, wenn das elektrische Potential des ersten Kondensators 604 das vierte elektrische Referenzpotential überschreitet, der erste Kondensator 604 auf das erste elektrische Referenzpotential gebracht wird. Dazu wird von der zweiten Schaltungseinheit 603 dem Schalter 612 (der beispielsweise als Transistor ausgebildet sein kann) ein derartiges elektrisches Signal bereitgestellt, dass der Schalter 612 geschlossen wird und eine elektrische Kopplung zwischen der ersten Spannungsquelle 605 und dem ersten Kondensator 604 erzeugt wird.

Ferner wird an dem Ausgang des zweiten Komparators 610 ein Pulsgeber 613 angeschlossen, der das Ereignis detektiert, dass das elektrische Potential des ersten Kondensators 604 außerhalb des zweiten Referenz-Bereichs ist und einen digitalen Impuls definierter Länge τ ausgibt.

Wie ferner in **Fig.6A** gezeigt, wird dieses Puls-Signal des Pulsgebers 613 dem Zähler-Element 606 bereitgestellt, das die Anzahl der Pulse und deren zeitliche Abfolge (d.h. die Frequenz, mit der die Pulse eintreffen) zählt.

Das erste Komparator-Element 607 und das zweite Komparator-Element 610 der Schaltkreis-Anordnung 600 sind jeweils als ein Operationsverstärker ausgestaltet.

Das in **Fig.6A** gezeigte Prinzipschaltbild der erfindungsgemäßen Schaltkreis-Anordnung 600 weist also eine Potentiostaten-Einheit auf, die mittels der ersten Schaltungseinheit 602 bzw. mittels des ersten Kondensators 604 realisiert ist. Diese hält das elektrische Potential der Sensor-Elektrode 601 auf dem elektrischen Soll-Potential innerhalb des ersten Referenz-Bereichs, definiert mittels des dritten elektrischen Referenzpotentials. Der von der Sensor-Elektrode 601 abgeleitete Sensor-Strom wird der zweiten Schaltungseinheit 603 entnommen, die darüber hinaus als Strom-Frequenz-Wandler fungiert. Der erste Kondensator 604 liefert der Sensor-Elektrode 601 elektrische Ladung zum Halten deren elektrischen Potentials nach, wobei die an dem ersten Kondensator 604 anliegende elektrische Spannung mittels der beschriebenen Komparator-Schaltung überwacht wird. Fällt die elektrische Spannung des ersten Kondensators 604 unter einen Schwellwert, so löst der Komparator 610 bzw. der Pulsgeber 613 einen Puls der definierten Länge τ aus, der mittels des Schalters 612 den ersten Kondensator 604 auf das elektrische Potential der ersten Spannungsquelle 605 umlädt. Der Puls dient darüber hinaus als Zählpuls für das mit dem Ausgang des zweiten Komparator-Elements 610 gekoppelte Zähler-Element 606.

Es ist zu betonen, dass die in **Fig.6A** gezeigte Schaltkreis-Anordnung 600 derart eingerichtet ist, dass sie der Sensor-Elektrode 601 elektrische Ströme bereitstellt, die Sensor-Elektrode 601 arbeitet hier als Stromsenke. Sollen dagegen an der Sensor-Elektrode 601 generierte elektrische Ströme von der Schaltkreis-Anordnung 600 aufgenommen werden, müsste diese komplementär aufgebaut werden.

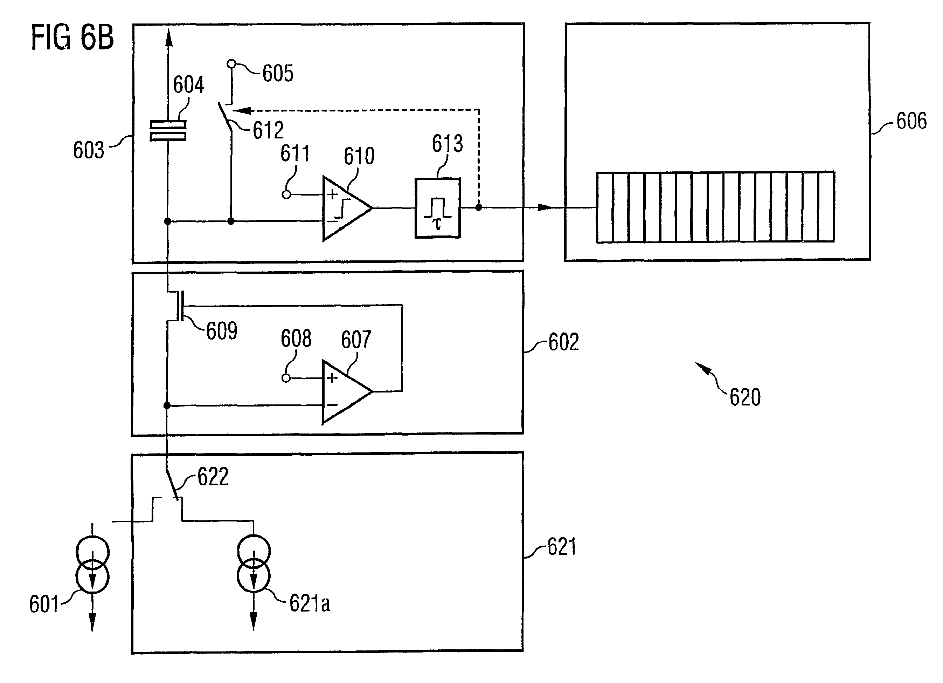

Im Weiteren wird bezugnehmend auf **Fig.6B** ein drittes bevorzugtes Ausführungsbeispiel der erfindungsgemäßen Schaltkreis-Anordnung beschrieben. Diejenigen Elemente der Schaltkreis-Anordnung 620, die der in **Fig.6A** gezeigten und oben beschriebenen Schaltkreis-Anordnung 600 entsprechen, sind mit dem gleichen Bezugszeichen versehen. Im Weiteren werden lediglich diejenigen Komponenten der Schaltkreis-Anordnung 620 näher beschrieben, die von der in **Fig.6A** gezeigten Schaltkreis-Anordnung 600 abweichen.

Die Schaltkreis-Anordnung 620 weist eine mit der ersten Schaltungseinheit 602 koppelbare Kalibrier-Einrichtung 621 zum Kalibrieren der Schaltkreis-Anordnung 620 auf, die derart eingerichtet ist, dass mittels der Kalibrier-Einrichtung 621 an die erste Schaltungseinheit 602 ein zweites elektrisches Referenzpotential anlegbar ist, wobei die erste Schaltungseinheit 602 entweder mit der Kalibrier-Einrichtung 621 oder mit der Sensor-Elektrode 601 gekoppelt ist.

Besonders vorteilhaft bei der in **Fig.6B** gezeigten Schaltkreis-Anordnung 620 ist, dass die Sensor-Elektrode 601 optional von der ersten Schaltungseinheit 602 abkoppelbar ist und stattdessen mit der Kalibrier-Einrichtung 621, deren wesentliche Komponente eine Referenz-Stromquelle 621a ist, koppelbar ist. Mittels eines von der Kalibrier-Einrichtung 621 erzeugten Kalibrier-Stroms kann eine Eichung der Schaltkreis-Anordnung 620 vorgenommen werden. Dies ist insbesondere dann vorteilhaft, wenn der exakte Wert der Kapazität C des ersten Kondensators 604 nicht bekannt ist. Neben statistischen Schwankungen der Kapazität des ersten Kondensators 604 infolge Variationen in der Prozesstechnik während des Herstellungsverfahrens des ersten Kondensators 604 tragen die nur mit großem Aufwand oder gar nicht exakt berechenbaren parasitären Kapazitäten der Schaltkreis-Anordnung 620 wesentlich zur Gesamtkapazität des Speicherknotens bei und beeinflussen die resultierende Ausgangsfrequenz, in der das zu erfassende Stromsignal kodiert ist, maßgeblich. Auch Offset-Spannungen, insbesondere des zweiten Komparators 610 in dem Strom-Frequenz-Wandler sowie eventuelle Leckströme haben einen direkten Einfluss auf die zu erfassende Ausgangsfrequenz. Wie in **Fig.6B** gezeigt, weist die Kalibrier-Einrichtung 621 eine zuschaltbare Referenz-Stromquelle 621a auf, die einen bekannten Sensor-Strom bereitstellt, oder diesen um einen bestimmten Betrag erhöht oder reduziert, wenn die Referenz-Stromquelle 621a parallel zum Sensor zugeschaltet wird. Die infolge des Zuschaltens resultierende Frequenzänderung dient dann zum Eichen der Schaltkreis-Anordnung 620. Eine derartige Eichung kann insbesondere vor dem Aufbringen eines Analyten auf die Sensor-Elektrode 601 durchgeführt werden. In diesem Falle liefert die Sensor-Elektrode 601 keinen von Sensor-Ereignissen stammenden Signal-Strom, und die Ausgangsfrequenz wird von dem Referenz-Strom der Referenz-Stromquelle 621a bestimmt.

Das wahlweise Zuschalten entweder der Sensor-Elektrode 601 oder der Kalibrier-Einrichtung 621 zu der ersten Schaltungseinheit 602 ist mittels eines weiteren Schalters 622 realisiert. Der Schalter 622 kann derart umgeschaltet werden, dass in dem in **Fig.6B** gezeigten Betriebszustand die Kalibrier-Einrichtung 621 der zweiten Schaltungseinheit 602 zugeschaltet ist, wohingegen in dem in **Fig.6B** gezeigten Betriebszustand die Sensor-Elektrode 601 der ersten Schaltungseinheit 602 nicht zugeschaltet ist. In einem komplementären Szenario, die einem Umlegen des in **Fig.6B** gezeigten weiteren Schalters 622 entspricht, ist die Sensor-Elektrode 601 der ersten Schaltungseinheit 602 zugeschaltet, wohingegen die Kalibrier-Einrichtung 621 dem ersten Schaltkreis nicht zugeschaltet ist.

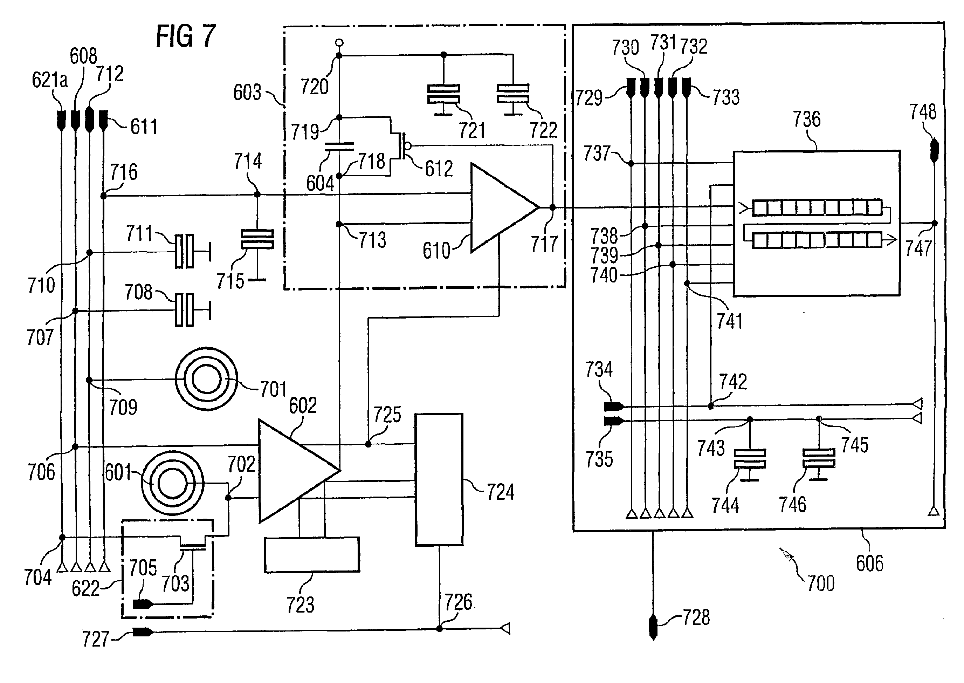

Im Weiteren wird bezugnehmend auf **Fig.7** ein viertes bevorzugtes Ausführungsbeispiel der erfindungsgemäßen Schaltkreis-Anordnung 700 beschrieben. Diejenigen Komponenten bzw. Blöcke aus **Fig.7**, die in **Fig.6B** eine direkte Entsprechung finden, sind in **Fig.7** mit den gleichen Bezugsziffern bezeichnet wie in **Fig.6B.**

**Fig.7** stellt eine Ausführungsform einer Sensor-Einheit dar, wie sie in einer matrixförmigen Anordnung mehrerer Sensor-Einheiten eingesetzt werden kann.

In **Fig.7** ist die Sensor-Elektrode 601 gezeigt. Ferner ist in **Fig.7** eine weitere Sensor-Elektrode 701 gezeigt. Die Sensor-Elektrode 601 ist mit einem ersten elektrischen Knoten 702 gekoppelt. Der erste elektrische Knoten 702 ist mit dem invertierten Eingang der ersten Schaltungseinheit 602 (funktionell ein Spannungsregler bzw. Potentiostat, im Weiteren auch als Regel-Element 602 bezeichnet) gekoppelt. Ferner ist der erste elektrische Knoten 702 mit dem einen Source-/Drain-Bereich eines ersten Transistors 703 gekoppelt. Der andere Source-/Drain-Bereich des ersten Transistors 703 ist mit einem zweiten elektrischen Knoten 704 gekoppelt. Der zweite elektrische Knoten 704 ist mit der Referenz-Stromquelle 621a der Kalibrier-Einrichtung gekoppelt. Der Gate-Bereich des ersten Transistors 703 ist mit einer ersten Spannungsversorgung 705 gekoppelt. Die erste Spannungsversorgung 705 und der erste Transistor 703 bilden den weiteren Schalter 622 aus. Der nicht-invertierte Eingang des Regel-Elements 602, das unter anderem einen Operationsverstärker enthält, ist mit einem dritten elektrischen Knoten 706 gekoppelt. Der dritte elektrische Knoten 706 ist identisch mit einem vierten elektrischen Knoten 707 -. "Identisch" in diesem Sinne bedeutet "elektrisch identisch", d.h., dass der elektrische Knoten 706 und der elektrische Knoten 707 (annähernd) auf dem gleichen elektrischen Potential liegen. Der vierte elektrische Knoten 707 ist ferner mit einer ersten Kapazität 708 sowie mit der zweiten Spannungsquelle 608 gekoppelt. Die weitere Elektrode 701 ist mit einem fünften elektrischen Knoten 709 gekoppelt. Der fünfte elektrische Knoten 709 ist identisch mit einem sechsten elektrischen Knoten 710. Der sechste elektrische Knoten 710 ist mit einer zweiten Kapazität 711 gekoppelt. Ferner ist der sechste elektrische Knoten 710 mit einer zweiten Spannungsversorgung 712 gekoppelt. Der Ausgang des ersten Regel-Elements 602 ist mit einem siebten elektrischen Knoten 713 gekoppelt. Der siebte elektrische Knoten 713 ist mit dem invertierten Eingang des zweiten Komparator-Elements 610, das als Operationsverstärker ausgebildet ist, gekoppelt. Der nicht-invertierte Eingang des zweiten Komparator-Elements 610 ist mit einem achten elektrischen Knoten 714 gekoppelt. Der achte elektrische Knoten 714 ist mit einer dritten Kapazität 715 gekoppelt. Ferner ist der achte elektrische Knoten 714 identisch mit einem neunten elektrischen Knoten 716 -. Der neunte elektrische Knoten 716 ist mit der dritten Spannungsquelle 611 gekoppelt. Ferner ist der Ausgang des Komparator-Elements 610 mit einem zehnten elektrischen Knoten 717 gekoppelt. Der zehnte elektrische Knoten 717 ist mit dem Gate-Bereich des Schalters 612 gekoppelt, welcher Schalter 612 als Transistor ausgebildet ist. Der eine Source-/Drain-Bereich des Schalters 612 ist mit einem elften elektrischen Knoten 718 gekoppelt. Der elfte elektrische Knoten 718 ist identisch mit dem siebten elektrischen Knoten 713 - und ist mit dem ersten Kondensator 604 gekoppelt. Der andere Source-/Drain-Bereich des Schalters 612 ist mit einem zwölften elektrischen Knoten 719 gekoppelt. Der zwölfte elektrische Knoten 719 ist einerseits mit dem ersten Kondensator 604 gekoppelt und andererseits identisch mit einem dreizehnten elektrischen Knoten 720. Der dreizehnte elektrische Knoten 720 ist mit einer vierten Kapazität 721 und mit einer fünften Kapazität 722 gekoppelt. An dem Knoten 720 liegt die positive Betriebsspannung. Ferner weist die Schaltkreis-Anordnung 700 eine erste Spannungsversorgungs-Einheit 723 und eine zweite Spannungsversorgungs-Einheit 724 auf. Ein erster und ein zweiter Anschluss der ersten Spannungsversorgungs-Einheit 723 sind mit zwei weiteren Anschlüssen des Regel-Elements 602 gekoppelt und diese weiteren Anschlüsse sind ferner mit zwei Anschlüssen der zweiten Spannungsversorgungs-Einheit 724 gekoppelt. Ein weiterer Anschluss der zweiten Spannungsversorgungs-Einheit 724 ist mit einem vierzehnten elektrischen Knoten 725 gekoppelt. Der vierzehnte elektrischen Knoten 725 ist sowohl mit einem weiteren Anschluss des Regel-Elements 602 als auch mit einem weiteren Anschluss des Komparator-Elements 610 gekoppelt. Ein weiterer Anschluss der zweiten Spannungsversorgungs-Einheit 724 ist mit einem fünfzehnten elektrischen Knoten 726 gekoppelt. Der fünfzehnte elektrische Knoten 726 ist mit einer dritten Spannungsversorgung 727 gekoppelt.

Ferner ist in **Fig.7** das Zähler-Element 606 gezeigt. Das Zähler-Element 606 ist mit einer vierten Spannungsversorgung 728 gekoppelt. Das Zähler-Element 606 weist ein erstes Steuersignal 729, ein zweites Steuersignal 730, ein drittes Steuersignal 731, ein viertes Steuersignal 732, ein fünftes Steuersignal 733, ein sechstes Steuersignal 734 und ein siebtes Steuersignal 735 auf. Ferner weist das Zähler-Element 606 eine Zähler-Einheit 736 auf. Das erste Steuersignal 729 ist mit einem sechzehnten elektrischen Knoten 737 gekoppelt. Der sechzehnte elektrische Knoten 737 ist mit einem Eingang der Zähler-Einheit 736 gekoppelt. Das zweite Steuersignal 730 ist mit einem siebzehnten elektrischen Knoten 738 gekoppelt. Der siebzehnte elektrische Knoten 738 ist mit einem weiteren Eingang der Zähler-Einheit 736 gekoppelt. Das dritte Steuersignal 731 ist mit einem achtzehnten elektrischen Knoten 739 gekoppelt. Der achtzehnte elektrische Knoten 739 ist mit einem weiteren Eingang der Zähler-Einheit 736 gekoppelt. Das vierte Steuersignal 732 ist mit einem neunzehnten elektrischen Knoten 740 gekoppelt. Der neunzehnte elektrische Knoten 740 ist mit einem weiteren Eingang der Zähler-Einheit 736 gekoppelt. Das fünfte Steuersignal 733 ist mit einem zwanzigsten elektrischen Knoten 741 gekoppelt. Der zwanzigste elektrische Knoten 741 ist mit einem weiteren Eingang der Zähler-Einheit 736 gekoppelt. Das sechste Steuersignal 734 ist mit einem einundzwanzigsten elektrischen Knoten 742 gekoppelt. Der einundzwanzigste elektrische Knoten 742 ist mit einem weiteren Eingang der Zählereinheit 736 gekoppelt. Das siebte Steuersignal 735 ist mit einem zweiundzwanzigsten elektrischen Knoten 743 gekoppelt. Der zweiundzwanzigste elektrische Knoten 743 ist mit einer sechsten Kapazität 744 gekoppelt. Ferner ist der zweiundzwanzigste elektrische Knoten 743 identisch mit einem dreiundzwanzigsten elektrischen Knoten 745. Der dreiundzwanzigste elektrische Knoten 745 ist mit einer siebten Kapazität 746 gekoppelt. An dem Ausgang der Zähler-Einheit 736 liegt ein Signal an, in dem der Zählerstand kodiert ist. Dieses Signal wird einem vierundzwanzigsten elektrischen Knoten 747 bereitgestellt. Das Zählerstand-Signal wird seriell von dem vierundzwanzigsten elektrischen Knoten 747 an einen Ausgangs-Anschluss 748 übermittelt.

Zusammenfassend sind wesentliche Komponenten der in **Fig.7** gezeigten Schaltkreis-Anordnung 700 die beiden Sensor-Elektroden 601, 701, die erste Schaltungseinheit 602, der als Speicherkapazität dienende erste Kondensator 604 mit dem dazu parallel geschalteten, als Transistor ausgebildeten Schalter 612, der zum Rücksetzen der Kondensator-Spannung dient. Dieses Rücksetzen wird mittels des zweiten Komparator-Elements 610, das ebenfalls als Operationsverstärker ausgebildet ist, ausgelöst, das die Spannung über dem ersten Kondensator 604 mit dem Spannungs-Signal der dritten Spannungsquelle 611 vergleicht und den als Transistor ausgebildeten Schalter 612 entsprechend ansteuert.

Es ist zu betonen, dass in der in **Fig.7** gezeigten Realisierung ein eigenständiger Schaltungsblock zum Erzeugen eines Pulses einer konstanten Länge nicht vorgesehen ist. Eine geeignete zeitliche Pulsdauer ergibt sich infolge der gezeigten Schaltung automatisch aus der Reaktionszeit des Systems 'zweites Komparator-Element 610 - erster Kondensator 604 - Schalter 612' und weist über einen großen Messbereich hinreichend konstante Werte auf.

Die Pulse des zweiten Komparator-Elements 610 werden in der Zähler-Einheit 736 des Zähler-Elements 606 gezählt. Mittels der Steuersignale kann die Zähler-Einheit 736 in einen Schieberegister-Betrieb umgeschaltet werden, wodurch der aktuelle Zählerstand an dem Ausgangs-Anschluss 748 seriell ausgegeben wird.

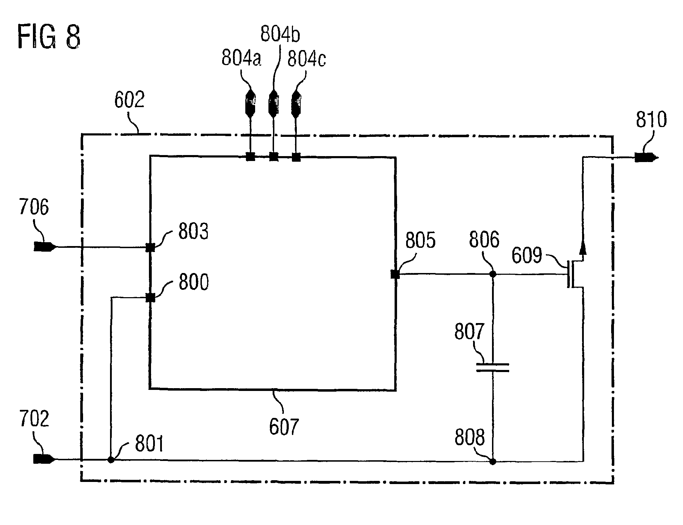

Im Weiteren wird bezugnehmend auf **Fig.8** die schaltungstechnische Ausgestaltung des ersten Komparator-Elements 607 in der in **Fig.7** gezeigten Schaltkreis-Anordnung 700 näher beschrieben. Diejenigen in F**ig.8** gezeigten Komponenten, die in **Fig.7** bzw. **Fig.6B** eine Entsprechung finden, sind mit den gleichen Bezugsziffern versehen.

In **Fig.8** ist das erste Regel-Element 602 (auch als erste Schaltungseinheit 602 bezeichnet) gezeigt. Der erste elektrische Knoten 702 aus **Fig.7** ist mit einem ersten elektrischen Knoten 801 gekoppelt und der erste elektrische Knoten 801 ist mit dem nicht-invertierten Eingang des Operationsverstärkers 607 (des ersten Komparator-Elements 607) gekoppelt. Der dritte elektrische Knoten 706 aus **Fig.7** ist mit dem invertierten Eingang 803 des Operationsverstärkers 607 gekoppelt. Ferner ist der Operationsverstärker 607 mit einem ersten Anschluss 804a, einem zweiten Anschluss 804b und einem dritten Anschluss 804c gekoppelt. Der erste Anschluss 804a ist mit der zweiten Spannungsversorgungs-Einheit 724 gekoppelt. Der zweite Anschluss 804b und der dritte Anschluss 804b sind jeweils mit der ersten Spannungsversorgungs-Einheit 823 gekoppelt. Ein Ausgang 805 des Operationsverstärkers 607 ist mit einem zweiten elektrischen Knoten 806 gekoppelt. Der zweite elektrische Knoten 806 ist mit einem Kondensator 807 gekoppelt. Der Kondensator 807 ist mit einem dritten elektrischen Knoten 808 gekoppelt. Der dritte elektrische Knoten 808 ist identisch mit dem ersten elektrischen Knoten 801. Ferner ist der zweite elektrische Knoten 806 mit dem Gate-Bereich des Transistors 609 gekoppelt. Der eine Source-/Drain-Bereich des Transistors 609 ist mit dem dritten elektrischen Knoten 808 gekoppelt und der andere Source-/Drain-Bereich des Transistors 609 ist mit einem Ausgangs-Anschluss 810 gekoppelt, welcher Ausgangs-Anschluss 810 dem Ausgang des ersten Regel-Elements 602 in **Fig.7** entspricht.

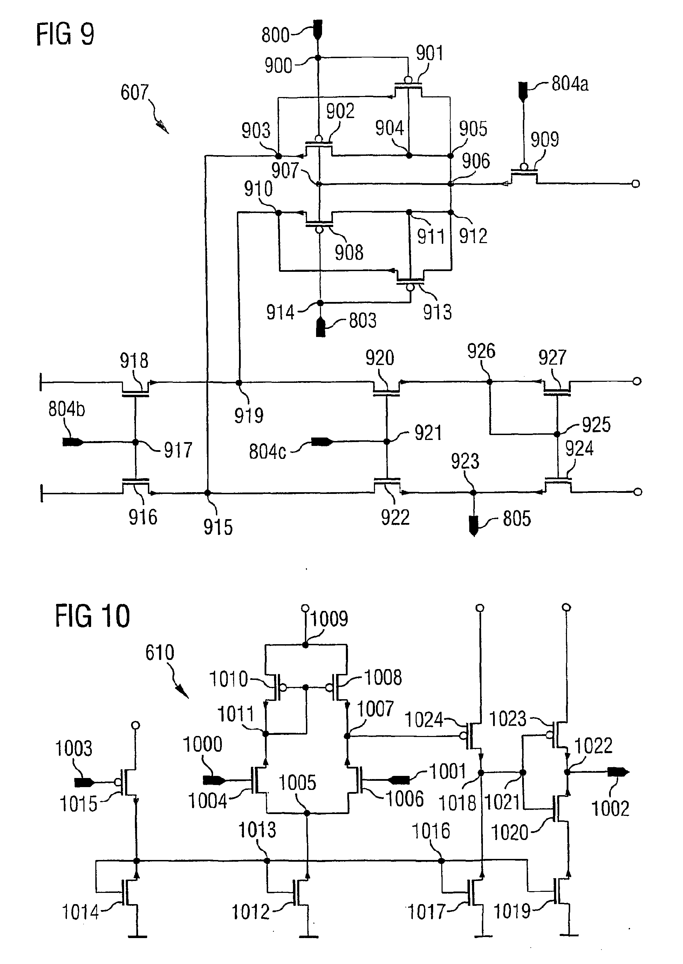

Im Weiteren wird bezugnehmend auf **Fig.9** der schaltungstechnische Aufbau des Operationsverstärkers 607 aus **Fig.8** näher beschrieben. Die Ein- und Ausgänge bzw. die Anschlüsse des Operationsverstärkers 607, die in **Fig. 8** gezeigt sind, sind in **Fig.9** mit den gleichen Bezugsziffern versehen.

Der in **Fig.9** gezeigte nicht-invertierte Eingang 800 des Operationsverstärkers 607 ist mit einem ersten elektrischen Knoten 900 gekoppelt. Der erste elektrische Knoten 900 ist mit dem Gate-Bereich eines ersten Transistors 901 gekoppelt. Ferner ist der erste elektrische Knoten 900 mit dem Gate-Bereich eines zweiten Transistors 902 gekoppelt. Der eine Source-/Drain-Bereich des ersten Transistors 901 ist mit einem zweiten elektrischen Knoten 903 gekoppelt. Der andere Source-/Drain-Bereich des zweiten Transistors 902 ist mit einem dritten elektrischen Knoten 904 gekoppelt. Der dritte elektrische Knoten 904 ist mit dem ersten Transistor 901 gekoppelt und ist identisch mit einem vierten elektrischen Knoten 905 -. Der vierte elektrische Knoten 905 ist mit dem anderen Source-/Drain-Bereich des ersten Transistors 901 gekoppelt. Ferner ist der vierte elektrische Knoten 905 identisch mit einem fünften elektrischen Knoten 906 . Der fünfte elektrische Knoten 906 ist identisch mit einem sechsten elektrischen Knoten 907. Der sechste elektrische Knoten 907 ist sowohl mit dem zweiten Transistor 902 als auch mit einem dritten Transistor 908 gekoppelt. Der fünfte elektrische Knoten 906 ist ferner mit dem einen Source-/Drain-Bereich eines vierten Transistors 909 gekoppelt. Der Gate-Bereich des vierten Transistors 909 ist mit dem ersten Anschluss 804a des Operationsverstärkers 607 gekoppelt. Der eine Source-/Drain-Bereich des dritten Transistors 908 ist mit einem siebten elektrischen Knoten 910 gekoppelt. Der andere Source-/Drain-Bereich des dritten Transistors 908 ist mit einem achten elektrischen Knoten 911 gekoppelt. Der achte elektrische Knoten 911 ist identisch mit einem neunten elektrischen Knoten 912. Der neunte elektrische Knoten 912 ist mit dem einen Source-/Drain-Bereich eines fünften Transistors 913 gekoppelt. Ferner ist der neunte elektrische Knoten 912 identisch mit dem fünften elektrischen Knoten 906. Der andere Source-/Drain-Bereich des fünften Transistors 913 ist mit dem siebten elektrischen Knoten 910 gekoppelt. Darüber hinaus ist der achte elektrische Knoten 911 mit dem fünften Transistor 913 gekoppelt. Der Gate-Bereich des dritten Transistors 908 ist mit einem zehnten elektrischen Knoten 914 gekoppelt. Der zehnte elektrische Knoten 914 ist ferner mit dem Gate-Bereich des fünften Transistors 913 gekoppelt. Darüber hinaus ist der zehnte elektrische Knoten 914 mit dem invertierten Eingang 803 des Operationsverstärkers 607 gekoppelt. Der zweite elektrische Knoten 903 ist identisch mit einem elften elektrischen Knoten 915. Der elfte elektrische Knoten 915 ist mit dem einen Source-/Drain-Bereich eines sechsten Transistors 916 gekoppelt. Der Gate-Bereich des sechsten Transistors 916 ist mit einem zwölften elektrischen Knoten 917 gekoppelt. Der zwölfte elektrische Knoten 917 ist mit dem zweiten Anschluss 804b der Komparator-Einheit 607 gekoppelt. Ferner ist der zwölfte elektrische Knoten 917 mit dem Gate-Bereich eines siebten Transistors 918 gekoppelt. Der eine Source-/Drain-Bereich des siebten Transistors 918 ist mit einem dreizehnten elektrischen Knoten 919 gekoppelt. Der dreizehnte elektrische Knoten 919 ist identisch mit dem siebten elektrischen Knoten 910. Ferner ist der dreizehnte elektrische Knoten 919 mit dem ersten Source-/Drain-Bereich eines achten Transistors 920 gekoppelt. Der Gate-Bereich des achten Transistors 920 ist mit einem vierzehnten elektrischen Knoten 921 gekoppelt. Der vierzehnte elektrische Knoten 921 ist mit dem dritten Anschluss 804c des Operationsverstärkers 607 gekoppelt und ist ferner mit dem Gate-Bereich eines neunten Transistors 922 gekoppelt. Der eine Source-/Drain-Bereich des neunten Transistors 922 ist mit dem elften elektrischen Knoten 915 gekoppelt und der andere Source-/Drain-Bereich des neunten Transistors 922 ist mit einem fünfzehnten elektrischen Knoten 923 gekoppelt. Der fünfzehnte elektrische Knoten 923 ist mit dem Ausgang 805 des Operationsverstärkers 607 gekoppelt und ist ferner mit dem einen Source-/Drain-Bereich eines zehnten Transistors 924 gekoppelt. Der Gate-Bereich des zehnten Transistors 924 ist mit einem sechzehnten elektrischen Knoten 925 gekoppelt. Der sechzehnte elektrische Knoten 925 ist ferner identisch mit einem siebzehnten elektrischen Knoten 926. Der siebzehnte elektrische Knoten 926 ist mit dem einen Source-/Drain-Bereich eines elften Transistors 927 gekoppelt, und der Gate-Bereich des elften Transistors 927 ist mit dem sechzehnten elektrischen Knoten 925 gekoppelt. Ferner ist der siebzehnte elektrische Knoten 926 mit dem anderen Source-/Drain-Bereich des achten Transistors 920 gekoppelt.

Im Weiteren wird bezugnehmend auf **Fig.10** ein bevorzugtes Ausführungsbeispiel des in **Fig.6B, Fig.7** gezeigten zweiten Komparator-Elements 610 beschrieben.

Das in **Fig.10** gezeigte Komparator-Element 610 weist einen ersten Eingang 1000 auf, der mit dem in **Fig.7** gezeigten siebten elektrischen Knoten 713 gekoppelt ist. Das Komparator-Element 610 weist ferner einen zweiten Eingang 1001 auf, der mit dem achten elektrischen Knoten 714 aus **Fig.7** gekoppelt ist. Ferner weist das Komparator-Element 610 einen Ausgang 1002 auf, der mit dem zehnten elektrischen Knoten 717 der Schaltkreis-Anordnung 700 aus **Fig.7** gekoppelt ist. Darüber hinaus weist das zweite Komparator-Element 610 einen Versorgungs-Eingang 1003 auf, der mit dem vierzehnten elektrischen Knoten 725 der Schaltkreis-Anordnung 700 gekoppelt ist und der damit indirekt mit der zweiten Spannungsversorgungs-Einheit 724 elektrisch gekoppelt ist.

Der erste Eingang 1000 ist mit dem Gate-Bereich eines ersten Transistors 1004 gekoppelt. Der eine Source-/Drain-Bereich des ersten Transistors 1004 ist mit einem ersten elektrischen Knoten 1005 gekoppelt. Der erste elektrische Knoten 1005 ist ferner mit dem einen Source-/Drain-Bereich eines zweiten Transistors 1006 gekoppelt. Der Gate-Bereich des zweiten Transistors 1006 ist mit dem zweiten Eingang 1001 des zweiten Komparator-Elements 610 gekoppelt. Der andere Source-/Drain-Bereich des zweiten Transistors 1006 ist mit einem zweiten elektrischen Knoten 1007 gekoppelt. Der zweite elektrische Knoten 1007 ist mit dem einen Source-/Drain-Bereich eines dritten Transistors 1008 gekoppelt. Der andere Source-/Drain-Bereich des dritten Transistors 1008 ist mit einem dritten elektrischen Knoten 1009 gekoppelt. Der dritte elektrische Knoten 1009 ist mit dem einen Source-/Drain-Bereich eines vierten Transistors 1010 gekoppelt. Der Gate-Bereich des dritten Transistors 1008 ist mit dem Gate-Bereich des vierten Transistors 1010 gekoppelt, und der Gate-Bereich des vierten Transistors 1010 ist ferner mit einem vierten elektrischen Knoten 1011 gekoppelt. Der vierte elektrische Knoten 1011 ist mit dem anderen Source-/Drain-Bereich des ersten Transistors 1004 gekoppelt. Ferner ist der erste elektrische Knoten 1005 mit dem einen Source-/Drain-Bereich eines fünften Transistors 1012 gekoppelt. Der Gate-Bereich des fünften Transistors 1012 ist mit einem fünften elektrischen Knoten 1013 gekoppelt. Der fünfte elektrische Knoten 1013 ist mit dem Gate-Bereich und mit dem einen Source-/Drain-Bereich eines sechsten Transistors 1014 gekoppelt. Der eine Source-/Drain-Bereich des sechsten Transistors 1014 ist mit dem einen Source-/Drain-Bereich eines siebten Transistors 1015 gekoppelt. Ferner ist der Gate-Bereich des siebten Transistors 1015 mit dem Versorgungs-Eingang 1003 gekoppelt. Der fünfte elektrische Knoten 1013 ist identisch mit einem sechsten elektrischen Knoten 1016. Ferner ist der sechste elektrische Knoten 1016 mit dem Gate-Bereich eines achten Transistors 1017 gekoppelt. Der eine Source-/Drain-Bereich des achten Transistors 1017 ist mit einem siebten elektrischen Knoten 1018 gekoppelt. Der sechste elektrische Knoten 1016 ist ferner mit dem Gate-Bereich eines neunten Transistors 1019 gekoppelt. Der eine Source-/Drain-Bereich des neunten Transistors 1019 ist mit dem einen Source-/Drain-Bereich eines zehnten Transistors 1020 gekoppelt. Der siebte elektrische Knoten 1018 ist identisch mit einem achten elektrischen Knoten 1021. Der Gate-Bereich des zehnten Transistors 1020 ist mit dem achten elektrischen Knoten 1021 gekoppelt. Der andere Source-/Drain-Bereich des zehnten Transistors 1020 ist mit einem neunten elektrischen Knoten 1022 gekoppelt. Der neunte elektrische Knoten 1022 ist mit dem Ausgang 1002 des zweiten Komparator-Elements 610 gekoppelt. Ferner ist der neunte elektrische Knoten 1022 mit dem einen Source-/Drain-Bereich eines elften Transistors 1023 gekoppelt. Der achte elektrische Knoten 1021 ist mit dem Gate-Bereich des elften Transistors 1023 gekoppelt. Ferner ist der siebte elektrische Knoten 1018 mit dem einen Source-/Drain-Bereich eines zwölften Transistors 1024 gekoppelt. Der Gate-Bereich des zwölften Transistors 1024 ist mit dem zweiten elektrischen Knoten 1007 gekoppelt.

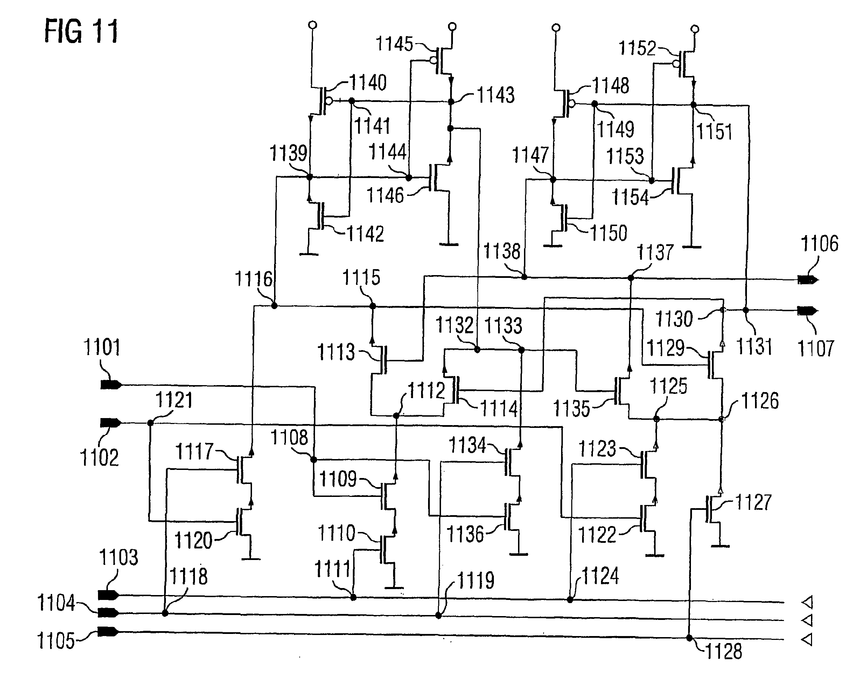

Im Folgenden wird bezugnehmend auf **Fig.11** ein bevorzugtes Ausführungsbeispiel für ein Zähler-Element der erfindungsgemäßen Schaltkreis-Anordnung beschrieben.

Das in **Fig.11** gezeigte Zähler-Element 1100 weist einen ersten Eingang 1101, einen zweiten Eingang 1102, einen dritten Eingang 1103, einen vierten Eingang 1104 und einen fünften Eingang 1105 auf. Ferner weist das Zähler-Element 1100 einen ersten Ausgang 1106 und einen zweiten Ausgang 1107 auf. Der erste Eingang 1101 ist mit einem ersten elektrischen Knoten 1108 gekoppelt. Der erste elektrische Knoten 1108 ist mit dem Gate-Bereich eines ersten Transistors 1109 gekoppelt. Der eine Source-/Drain-Bereich des ersten Transistors 1109 ist mit dem einen Source-/Drain-Bereich eines zweiten Transistors 1110 gekoppelt. Der Gate-Bereich des zweiten Transistors 1110 ist mit einem zweiten elektrischen Knoten 1111 gekoppelt. Der zweite elektrische Knoten 1111 ist mit dem dritten Eingang 1103 des Zähler-Elements 1100 gekoppelt. Der andere Source-/Drain-Bereich des ersten Transistors 1109 ist mit einem dritten elektrischen Knoten 1112 gekoppelt. Der dritte elektrische Knoten 1112 ist mit dem einen Source-/Drain-Bereich eines dritten Transistors 1113 gekoppelt. Ferner ist der dritte elektrische Knoten 1112 mit dem einen Source-/Drain-Bereich eines vierten Transistors 1114 gekoppelt. Der andere Source-/Drain-Bereich des dritten Transistors 1113 ist mit einem vierten elektrischen Knoten 1115 gekoppelt. Der vierte elektrische Knoten 1115 ist mit einem fünften elektrischen Knoten 1116 gekoppelt. Der fünfte elektrische Knoten 1116 ist mit dem einen Source-/Drain-Bereich eines fünften Transistors 1117 gekoppelt. Der Gate-Bereich des fünften Transistors 1117 ist mit einem sechsten elektrischen Knoten 1118 gekoppelt. Der sechste elektrische Knoten 1118 ist mit dem vierten Eingang 1104 des Zähler-Elements 1100 gekoppelt. Ferner ist der sechste elektrische Knoten 1118 identisch mit einem siebten elektrischen Knoten 1119 Der andere Source-/Drain-Bereich des fünften Transistors 1117 ist mit dem einen Source-/Drain-Bereich eines sechsten Transistors 1120 gekoppelt. Der Gate-Bereich des sechsten Transistors 1120 ist mit einem achten elektrischen Knoten 1121 gekoppelt. Der achte elektrische Knoten 1121 ist mit dem zweiten Eingang 1102 des Zähler-Elements 1100 gekoppelt. Ferner ist der achte elektrische Knoten 1121 mit dem Gate-Bereich eines siebten Transistors 1122 gekoppelt. Der eine Source-/Drain-Bereich des siebten Transistors 1122 ist mit dem einen Source-/Drain-Bereich eines achten Transistors 1123 gekoppelt. Der Gate-Bereich des achten Transistors 1123 ist mit einem neunten elektrischen Knoten 1124 gekoppelt. Der neunte elektrische Knoten 1124 ist identisch mit dem zweiten elektrischen Knoten 1111. Der andere Source-/Drain-Bereich des achten Transistors 1123 ist mit einem zehnten elektrischen Knoten 1125 gekoppelt. Der zehnte elektrische Knoten 1125 ist identisch mit einem elften elektrischen Knoten 1126. Der elfte elektrische Knoten 1126 ist mit dem einen Source-/Drain-Bereich eines neunten Transistors 1127 gekoppelt. Der Gate-Bereich des neunten Transistors 1127 ist mit einem zwölften elektrischen Knoten 1128 gekoppelt. Der zwölfte elektrische Knoten 1128 ist mit dem fünften Eingang 1105 des Zähler-Elements 1100 gekoppelt. Der elfte elektrische Knoten 1126 ist mit dem einen Source-/Drain-Bereich eines zehnten Transistors 1129 gekoppelt. Der Gate-Bereich des zehnten Transistors 1129 ist mit dem vierten elektrischen Knoten 1115 gekoppelt. Der andere Source-/Drain-Bereich des zehnten Transistors 1129 ist mit einem dreizehnten elektrischen Knoten 1130 gekoppelt. Der dreizehnte elektrische Knoten 1130 ist identisch mit einem vierzehnten elektrischen Knoten 1131. Der vierzehnte elektrische Knoten 1131 ist mit dem zweiten Ausgang 1107 des Zähler-Elements 1100 gekoppelt. Ferner ist der dreizehnte elektrische Knoten 1130 mit dem Gate-Bereich des vierten Transistors 1114 gekoppelt. Der andere Source-/Drain-Bereich des vierten Transistors 1114 ist mit einem fünfzehnten elektrischen Knoten 1132 gekoppelt. Der fünfzehnte elektrische Knoten 1132 ist identisch mit einem sechzehnten elektrischen Knoten 1133. Der sechzehnte elektrische Knoten 1133 ist mit dem einen Source-/Drain-Bereich eines elften Transistors 1134 gekoppelt. Der sechzehnte elektrische Knoten 1133 ist ferner mit dem Gate-Bereich eines zwölften Transistors 1135 gekoppelt. Der eine Source-/Drain-Bereich des zwölften Transistors 1135 ist mit dem zehnten elektrischen Knoten 1125 gekoppelt. Der Gate-Bereich des elften Transistors 1134 ist mit dem siebten elektrischen Knoten 1119 gekoppelt. Der andere Source-/Drain-Bereich des elften Transistors 1134 ist mit dem einen Source-/Drain-Bereich eines dreizehnten Transistors 1136 gekoppelt. Der Gate-Bereich des dreizehnten Transistors 1136 ist mit dem ersten elektrischen Knoten 1108 gekoppelt. Der andere Source-/Drain-Bereich des zwölften Transistors 1135 ist mit einem siebzehnten elektrischen Knoten 1137 gekoppelt. Der siebzehnte elektrische Knoten 1137 ist identisch mit einem achtzehnten elektrischen Knoten 1138. Ferner ist der siebzehnte elektrische Knoten 1137 mit dem ersten Ausgang 1106 des Zähler-Elements 1100 gekoppelt. Der Gate-Bereich des dritten Transistors 1113 ist ferner mit dem achtzehnten elektrischen Knoten 1138 gekoppelt. Der fünfte elektrische Knoten 1116 ist identisch mit einem neunzehnten elektrischen Knoten 1139. Der neunzehnte elektrische Knoten 1139 ist mit dem einen Source-/Drain-Bereich eines vierzehnten Transistors 1140 gekoppelt. Der Gate-Bereich des vierzehnten Transistors 1140 ist mit einem zwanzigsten elektrischen Knoten 1141 gekoppelt. Der zwanzigste elektrische Knoten 1140 ist mit dem Gate-Bereich eines fünfzehnten Transistors 1142 gekoppelt. Der neunzehnte elektrische Knoten 1139 ist mit dem einen Source-/Drain-Bereich des fünfzehnten Transistors 1142 gekoppelt. Der Gate-Bereich des vierzehnten Transistors 1140 ist mit einem einundzwanzigsten elektrischen Knoten 1143 gekoppelt. Ein zweiundzwanzigster elektrischen Knoten 1144 ist identisch mit dem neunzehnten elektrischen Knoten 1139. Der zweiundzwanzigste elektrische Knoten 1143 ist mit dem einen Source-/Drain-Bereich eines sechzehnten Transistors 1145 gekoppelt. Der Gate-Bereich des sechzehnten Transistors 1145 ist mit dem zweiundzwanzigsten elektrischen Knoten 1144 gekoppelt. Der Gate-Bereich eines siebzehnten Transistors 1146 ist mit dem zweiundzwanzigsten elektrischen Knoten 1144 gekoppelt. Der eine Source-/Drain-Bereich des siebzehnten Transistors 1146 ist mit dem einundzwanzigsten elektrischen Knoten 1143 gekoppelt. Der zweiundzwanzigste elektrische Knoten 1143 ist identisch mit dem fünfzehnten elektrischen Knoten 1132. Ferner ist der zweiundzwanzigste elektrische Knoten 1144 mit dem Gate-Bereich eines siebzehnten Transistors 1146 gekoppelt, wobei der eine Source-/Drain-Bereich des siebzehnten Transistors 1146 mit dem einundzwanzigsten elektrischen Knoten 1143 gekoppelt ist. Der neunzehnte elektrische Knoten 1138 ist identisch mit einem dreiundzwanzigsten elektrischen Knoten 1147. Der dreiundzwanzigste elektrische Knoten 1147 ist mit dem einen Source-/Drain-Bereich eines achtzehnten Transistors 1148 gekoppelt. Der Gate-Bereich des achtzehnten Transistors 1148 ist mit einem vierundzwanzigsten elektrischen Knoten 1149 gekoppelt. Der vierundzwanzigste elektrische Knoten 1149 ist mit dem Gate-Bereich eines neunzehnten Transistors 1150 gekoppelt. Der dreiundzwanzigste elektrische Knoten 1147 ist mit dem einen Source-/Drain-Bereich des neunzehnten Transistors 1150 gekoppelt. Der Gate-Bereich des achtzehnten Transistors 1148 ist mit einem fünfundzwanzigsten elektrischen Knoten 1151 gekoppelt. Der fünfundzwanzigste elektrische Knoten 1151 ist mit dem einen Source-/Drain-Bereich eines zwanzigsten Transistors 1152 gekoppelt. Der Gate-Bereich des zwanzigsten Transistors 1152 ist mit einem sechsundzwanzigsten elektrischen Knoten 1153 gekoppelt. Der sechsundzwanzigste elektrische Knoten 1153 ist identisch mit dem dreiundzwanzigsten elektrischen Knoten 1147. Der fünfundzwanzigste elektrische Knoten 1151 ist mit dem einen Source-/Drain-Bereich eines einundzwanzigsten Transistors 1154 gekoppelt. Der Gate-Bereich des einundzwanzigsten Transistors 1154 ist mit dem sechsundzwanzigsten elektrischen Knoten 1153 gekoppelt. Der fünfundzwanzigste elektrische Knoten 1151 ist identisch mit dem vierzehnten elektrischen Knoten 1131. Der erste elektrische Knoten 1108 ist mit dem Gate-Bereich des dreizehnten Transistors 1136 gekoppelt.

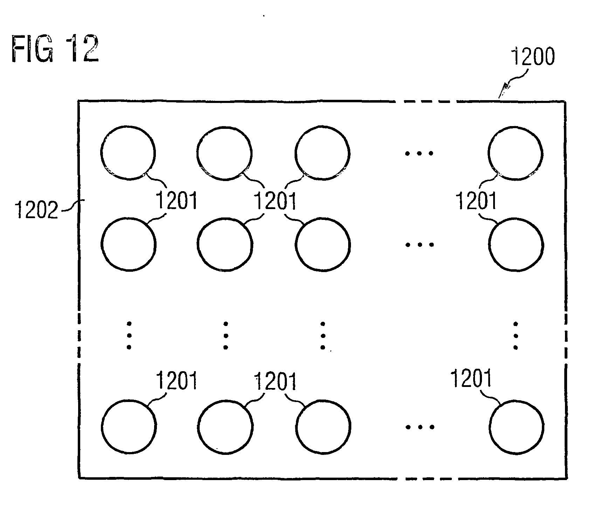

In **Fig.12** ist ein bevorzugtes Ausführungsbeispiel der erfindungsgemäßen Sensor-Anordnung 1200 mit einer Mehrzahl von auf einem Chip 1202 matrixförmig angeordneten Schaltkreis-Anordnungen 1201 (von denen jede wie die in **Fig.7** gezeigte Schaltkreis-Anordnung 700 ausgestaltet sein kann) gezeigt. Jede der Schaltkreis-Anordnungen 1200 kann unabhängig von den anderen als Sensor betrieben werden. Sind die Schaltkreis-Anordnungen 1200 als Sensoren zum Nachweis unterschiedlicher Moleküle ausgestaltet (z.B. jede weist mit einer speziellen Art von DNA-Strängen hybridisierbare Fängermoleküle auf), so ist mittels der Sensor-Anordnung 1200 eine parallele Analyse einer zu untersuchenden Flüssigkeit möglich. Am Rande der matrixförmigen Anordnung von Sensor-Elektroden befinden sich hierbei Schaltungseinheiten, die zur Ansteuerung, zur Spannungs- sowie Stromversorgung und zum Auslesen der Sensorzellen dienen.

Diese Schaltungseinheiten liefern beispielsweise den Referenzstrom 621a zur Kalibrierung der einzelnen Sensorfelder, Versorgungs- und Referenz-Spannungen für die in den Sensor-Elementen enthaltene Regel-Einheit 602 und Komparator-Einheit 603, sowie die digitalen Steuersignale für den Zähler 736. Dies sind insbesondere ein Rücksetzsignal für den Zähler, ein Umschaltsignal für den Zähler-/Schieberegisterbetrieb, sowie gegebenenfalls ein Umschaltesignal für dem ersten Kondensator 604 parallelgeschaltete weitere Kondensatoren. Insbesondere enthalten die Einheiten am Rande der Matrix die Schaltungen zur Vorauswertung der gemessenen Signale, insbesondere zum Auslesen, Speichern und Weiterverarbeitung der Zählerinhalte der einzelnen Sensor-Elemente.

Die Vorteile der erfindungsgemäßen Sensor-Schaltung kommen bei einer Anordnung einer Vielzahl von Sensor-Einheiten auf einem Halbleiterchip besonders zum tragen, da jedes Sensor-Element in der Lage ist, autark das Strom-Signal der Sensorelektroden zu messen und in Form eines digitalen Zählersignals innerhalb des Sensorelements zu speichern. Gleichzeitig wird das Elektroden-Potential auf dem Soll-Potential konstant gehalten. Dieses Zählersignal kann dann zu einem beliebigen Zeitpunkt mittels der Schaltungseinheiten am Rande der Matrix abgefragt und weiterverarbeitet werden.

Aufgrund der hohen Wortbreite des Binärzählers 736 ist es zweckmäßig, den Zählerstand aus den Sensorelementen seriell auszulesen, da bei parallelem Auslesen sehr breite Datenbusse über die gesamte Matrix geführt werden müssten. Die serielle Ausgabe des Zählerstandes erfolgt durch Umschalten des Binärzählers 736 aus der Zählerbetriebsart in die Schieberegisterbetriebsart. Durch Anlegen eines Taktsignals wird dann der Zählerinhalt, das heißt die einzelnen Datenbits in den Zählerstufen sukzessive in die jeweils nachfolgende Zählerstufe weitergeschoben, so dass am Ausgang des Zählers nach n Taktimpulsen alle Datenbits eines n-stufigen Zählers ausgegeben werden. Die Anzahl der notwendigen Zählerstufen hängt direkt mit dem geforderten Dynamikumfang zusammen. Soll beispielsweise ein Messsignal mit einer Genauigkeit von 6 bit in einem Messbereich von 5 Dekaden erfasst werden, ist ein Zähler mit einer Wortbreite von 23 bit notwendig. Die Verwendung serieller Protokolle zur Datenübermittlung ist insbesondere auch deshalb vorteilhaft, da dadurch gleichzeitig die Kommunikation mit dem Auslesegerät, in das der Chip eingesetzt wird, vereinfacht ist.

Die Verwendung einer Zählerschaltung innerhalb der Sensoreinheit ist nicht zwingend notwendig, stattdessen kann beispielsweise auch direkt das Ausgangssignal des Pulsgebers 613 ausgegeben werden, in dem die gemessene Stromstärke an den Sensorelektroden in Form einer Frequenz codiert ist. Die Schaltungseinheiten am Rande der Matrix dienen dann dazu, die Frequenzen bzw. Pulsdauern der einzelnen Sensoreinheiten zu messen und weiter zu verarbeiten.

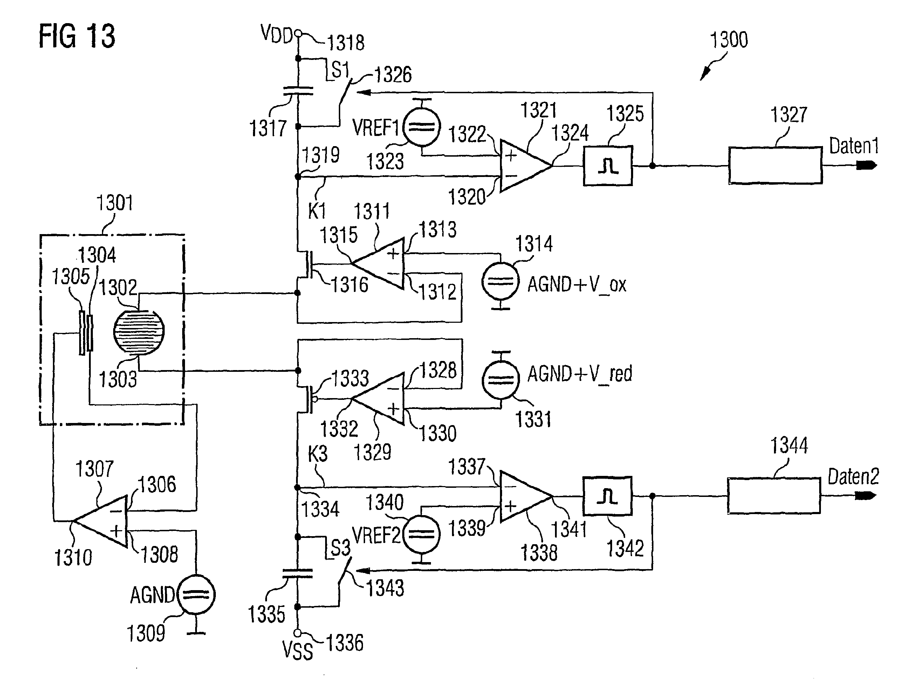

**Fig.13** zeigt eine Schaltkreisanordnung 1300 gemäß obigem Ausführungsbeispiel in Gesamtdarstellung.

Wie in Fig.13 dargestellt, weist ein elektrochemisches System 1301 einen Analyten auf, der die zu erfassenden makromolekularen Biopolymere enthalten kann. Ferner ist eine Generator-Elektrode 1302 vorgesehen, welche gemeinsam mit einer Kollektor-Elektrode 1303 als Interdigital-Elektrode eingerichtet ist. Ferner sind eine Referenz-Elektrode 1304 und eine Gegen-Elektrode 1305 vorgesehen zur Einstellung des gewünschten elektrischen Potentials in dem Analyten.

Die Referenz-Elektrode 1304 ist mit dem invertierenden Eingang 1306 eines Referenzpotential-Einstell-Operationsverstärkers 1307, dessen nicht invertierender Eingang 1308 über eine Referenzpotential-Spannungsquelle 1309 mit dem Massepotential gekoppelt ist. Der Ausgang 1310 des Referenzpotential-Einstell-Operationsverstärkers 1307 ist mit der Gegen-Elektrode 1305 gekoppelt. Somit weist das elektrochemische System 1301 vier Elektroden 1302, 1303, 1304 und 1305 auf, welche elektrochemisch mittels des Analyten gekoppelt sind.

Der von dem Referenzpotential-Einstell-Operationsverstärker 1307 gebildete Potentiostat misst das elektrochemische Potential über die Referenz-Elektrode 1304 und das elektrochemische Potential wird mittels der Gegen-Elektrode 1305 auf den vorgegebenen Sollwert, der mittels der Referenzpotential-Spannungsquelle 1309 vorgegeben wird, nachgeregelt.

Für ein korrektes Funktionieren der Schaltungen sollte dieser vorgegebene Sollwert zwischen der positiven und der negativen Betriebsspannung des Schaltkreises liegen. Typischerweise liegt der Wert des Referenzpotentials in der Mitte zwischen der positiven Betriebsspannung $V_{DD}$ und der negativen Betriebsspannung $V_{SS}$ des Schaltkreises. Es ist jedoch anzumerken, dass der absolute Wert in diesem Fall nicht entscheidend ist, da für das elektrochemische System 1301 nur die Spannungsdifferenzen zwischen den Elektroden 1302, 1303, 1304 und 1305 von Bedeutung sind. Die von dem Referenzpotential-Einstell-Operationsverstärker 1307 gebildete Potentiostatenschaltung ist nur einmal auf dem gesamten Sensorchip vorhanden und wird im Weiteren nicht näher erläutert.

Ferner zeigt Fig.13 ein die erste Schaltungseinheit bildendes Generator-Komparator-Element 1311, dessen invertierender Eingang 1312 mit der Generator-Elektrode 1302 gekoppelt ist und dessen nicht invertierender Eingang 1313 über eine erste Referenz-Spannungsquelle 1314 mit dem Massepotential gekoppelt ist, so dass mittels des von einem Gegen-Operationsverstärker gebildeten Generator-Komparator-Elements 1311, welcher einen Regelverstärker darstellt, das Generator-Soll-Potential $AGND_{+V\_ox}$ eingeregelt wird. Ein Ausgang 1315 des Generator-Komparator-Elements 1311 ist mit einem Gate-Anschluss eines ersten NMOS-Transistors 1316 gekoppelt, dessen erster Source/Drain-Bereich mit der Generator-Elektrode 1302 und dem invertierenden Eingang 1312 des Generator-Komparator-Elements 1311 gekoppelt ist und dessen zweiter Source/Drain-Bereich mit einem ersten Anschluss eines ersten Kondensators 1317 gekoppelt ist, dessen zweiter Anschluss mit dem positiven Betriebspotential $V_{DD}$ 1318 gekoppelt ist.

Der erste Anschluss des ersten Kondensators 1317 ist ferner mit einem ersten Knoten K1 1319 gekoppelt, welcher wiederum mit einem invertierenden Eingang 1320 eines zweiten Generator-Komparator-Elements 1321 gekoppelt ist, dessen nicht invertierender Eingang 1322 über eine ein zweites Generator-Soll-Potential definierende zweite Referenz-Spannungsquelle 1323 mit dem Massepotential gekoppelt ist. Ein Ausgang 1324 des zweiten Generator-Komparator-Elements 1321, welches ebenfalls von einem Operationsverstärker gebildet wird, ist mit einem Eingang eines Impuls-Generators 1325 gekoppelt, welcher ausgangsseitig einen ersten Schalter S1 1326 schließt, solange der Impuls-Generator 1325 einen Impuls erzeugt. Der erste Schalter S1 1326 weist einen ersten Anschluss auf, welcher mit dem ersten Knoten K1 1319 gekoppelt ist und einen zweiten Anschluss, der mit dem positiven Betriebspotential $V_{DD}$ 1318 gekoppelt ist.

Anschaulich wird somit der erste Knoten K1 1319 bei Schließen des ersten Schalters S1 1326 mit dem positiven Betriebspotential $V_{DD}$ gekoppelt. Ferner ist der Ausgang des ersten Impuls-Generators 1325 mit einem ersten Zähler-Element 1327 gekoppelt, welches in einen Schieberegister-Modus geschaltet werden kann und ausgangsseitig erste Ergebnisdaten Daten1 bereitstellt.

Ferner ist ein invertierender Eingang 1328 eines Kollektor-Komparator-Elements 1329 mit der Kollektor-Elektrode 1303 gekoppelt. Der nicht invertierende Eingang 1330 des als Operationsverstärker ausgebildeten Kollektor-Komparator-Elements 1329 ist über eine dritte Referenz-Spannungsquelle 1331 mit dem Massepotential gekoppelt. Der Ausgang 1332 des Kollektor-Komparator-Elements 1329 ist mit einem Gate-Anschluss eines ersten PMOS-Transistors 1333 gekoppelt, dessen erster Source/Drain-Bereich mit der Kollektor-Elektrode 1303 gekoppelt ist und dessen zweiter Source/Drain-Bereich mit einem dritten Knoten K3 1334 gekoppelt ist.

Die oben beschriebene zweite Potentiostatenschaltung dient zur Einregelung des Potentials an der Kollektor-Elektrode 1303 auf das mittels der dritten Referenz-Spannungsquelle 1331 definierten Soll-Potentials $AGND_{+V\_red}$.

Mit dem dritten Knoten K3 1334 ist ferner ein erster Anschluss eines dritten Kondensators 1335 gekoppelt, dessen zweiter Anschluss mit dem negativen Betriebspotential $V_{SS}$ 1336 gekoppelt ist.

Ferner ist der dritte Knoten K3 1334 mit dem invertierenden Eingang 1337 eines zweiten Kollektor-Komparator-Elements 1338 gekoppelt, dessen nicht invertierender Eingang 1339 über eine vierte Referenz-Spannungsquelle 1340 mit dem Massepotential verbunden ist.

Ein Ausgang 1341 des zweiten Kollektor-Komparator-Elements 1338 ist mit einem Eingang eines zweiten Impuls-Generators 1342 gekoppelt, dessen Ausgang auf einen dritten Schalter S3 1343 rückgekoppelt ist und diesen Schalter schließt, solange ein Puls erzeugt wird. Ein erster Anschluss des dritten Schalters S3 1343 ist mit dem negativen Betriebspotential $V_{SS}$ 1336 gekoppelt und ferner mit dem zweiten Anschluss des dritten Kondensators 1335 und ein zweiter Anschluss des dritten Schalters S3 1343 ist mit dem dritten Knoten K3 1334 gekoppelt. Somit wird mittels des dritten Schalters S3 1343 bei Schließen des Schalters der dritte Knoten K3 1334 mit dem negativen Betriebspotential $V_{SS}$ 1336 gekoppelt.

Ferner ist der Ausgang des zweiten Impuls-Generators 1342 mit einem ebenfalls mit einem Schieberegister-Modus ausgestatteten zweiten Zähler-Element 1344 gekoppelt, welcher ausgangsseitig zweite Ergebnisdaten Daten2 bereitstellt.

Anschaulich werden bei der oben beschriebenen Schaltkreisanordnung die Spannungen an den Sensor-Elektroden relativ zu der Spannung AGND mittels der Regelverstärker 1311 und 1329, das heißt mittels des ersten Generator-Komparator-Elements 1311 und des ersten Kollektor-Komparator-Elements 1329 eingeregelt, und zwar das Potential an der Generator-Elektrode 1302 auf das Potential $AGND_{+V\_ox}$ der ersten Referenz-Spannungsquelle 1314 und das Potential der Kollektor-Elektrode 1303 auf die dritte Referenz-Spannung $AGND_{+V\_red}$, wobei V_ox das Oxidationspotential und V_red das Reduktionspotential der am Redox-Cycling beteiligten elektrochemischen Spezies ist.

Die Analog-/Digital-Wandlung erfolgt unabhängig für beide Elektroden 1302, 1303 mit Hilfe je eines Sägezahn-Generators, das heißt mittels der Impuls-Generatoren 1325, 1342.

Der vom Sensor abgeleitete Strom wird dabei dem jeweiligen Kondensator 1317, 1335 entnommen. Hierdurch entlädt sich der jeweilige Kondensator. Die Spannung an den Kondensatoren wird mittels zweier Komparatoren überwacht. Erreicht die Spannung einen vorgegebenen Wert, so lösen die Komparatoren einen Rücksetzimpuls aus. Mit diesem Impuls wird der jeweilige Kondensator wieder nachgeladen. Die Zahl der Rücksetzimpulse je Zeiteinheit ist ein Maß für den Strom des Sensors. Die beiden Teilschaltungen an den beiden Elektroden sind in diesem Fall komplementär zueinander ausgelegt, die Schaltung für die Generator-Elektrode (obere Hälfte) arbeitet als Stromquelle, die Schaltung für die Kollektor-Elektrode 1303 (untere Hälfte) als Stromsenke. Außerdem arbeiten die beiden Teilschaltungen voneinander unabhängig.

Da die Ströme in der Generator-Elektrode 1302 und in der Kollektor-Elektrode 1303 im Wesentlichen die gleiche Information tragen, ist es möglich, nur die betragsmäßige Summe der beiden Signale zu messen.

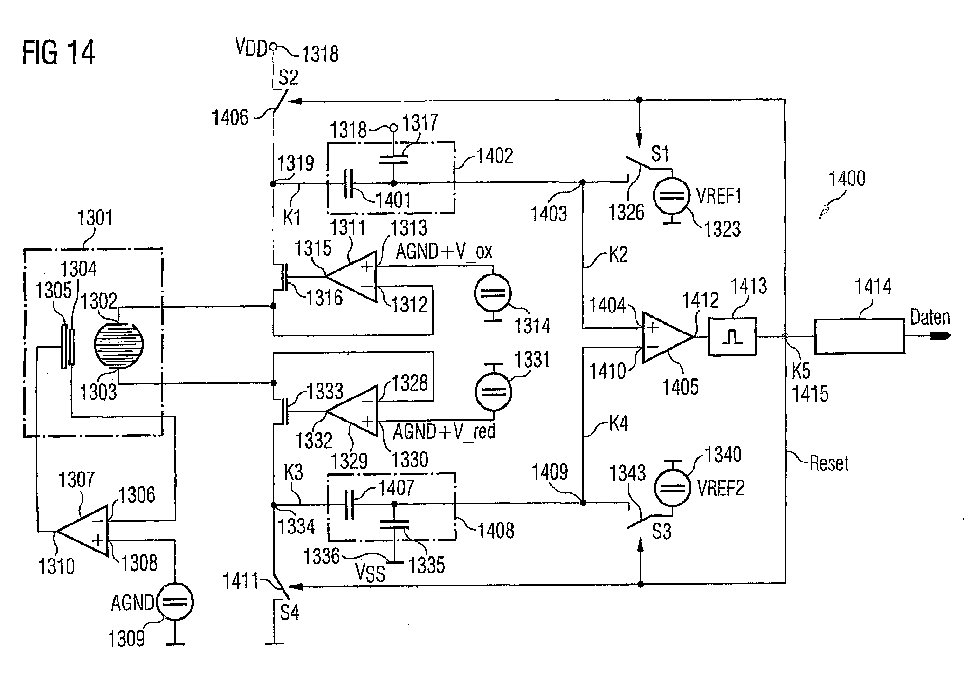

Eine geeignete Schaltung zur Messung der betragsmäßigen Summe der beiden Stromsignale ist in **Fig.14** gezeigt.

Gleiche Bauelemente in den Schaltkreisanordnungen 1300 und 1400 sind mit identischen Bezugszeichen versehen.

Die Schaltkreisanordnung 1400 gemäß dem zweiten Ausführungsbeispiel der Erfindung weist einen Komparator und ein Zähler-Element weniger auf als die Schaltkreisanordnung 1300 gemäß dem ersten Ausführungsbeispiel der Erfindung.

Im Unterschied zu oben beschriebener Schaltkreisanordnung 1300 weist die Schaltkreisanordnung 1400 gemäß dem zweiten Ausführungsbeispiel der Erfindung eine unterschiedliche zweite und vierte Schaltungseinheit auf.

Das elektrochemische System selbst und die erste Schaltungseinheit und die dritte Schaltungseinheit bleiben unverändert.

Bei der Schaltkreisanordnung 1400 ist der erste Knoten K1 1319 mit einem zweiten Kondensator 1401 gekoppelt, dessen zweiter Anschluss mit dem ersten Kondensator in Serie geschaltet ist, welcher erste Kondensator 1317 mit seinem zweiten Anschluss mit dem positiven Betriebspotential $V_{DD}$ 1318 gekoppelt ist.

Der erste Kondensator 1317 und der zweite Kondensator 1401 bilden einen ersten kapazitiven Spannungsteiler 1402.

Der zweite Anschluss des zweiten Kondensators 1401 und der erste Anschluss des ersten Kondensators 1317 sind ferner mit einem zweiten Knoten K2 1403 gekoppelt, welcher wiederum mit dem nicht invertierenden Eingang 1404 eines Summen-Komparator-Elements 1405 gekoppelt ist. Ferner ist der zweite Knoten K2 1403 mit einem ersten Anschluss des ersten Schalters S1 1326 gekoppelt, dessen zweiter Anschluss mit der zweiten Referenz-Spannungsquelle 1323 und darüber mit dem Massepotential gekoppelt ist, so dass für den Fall, dass der erste Schalter S1 1326 geschlossen ist, der zweite Knoten K2 1403 auf der zweiten Referenz-Spannung VREF1 liegt. Der erste Schalter S1 1326 ist geschlossen, solange der Summen-Impuls-Generator 1413 einen Impuls erzeugt. In gleicher Weise schließt der Summen-Impuls-Generator 1413 einen zweiten Schalter S2 1406, welcher in geschlossenem Zustand den ersten Knoten K1 1319 mit dem positiven Betriebspotential $V_{DD}$ 1318 koppelt.

Ferner ist der dritte Knoten K3 1334 mit einem vierten Kondensator 1407 gekoppelt, dessen zweiter Anschluss mit dem ersten Anschluss des dritten Kondensators 1335 gekoppelt ist, dessen zweiter Anschluss mit dem negativen Betriebspotential $V_{SS}$ 1336 gekoppelt ist.

Damit sind der dritte Kondensator 1335 und der vierte Kondensator 1407 ebenfalls in Serie geschaltet und bilden einen zweiten kapazitiven Spannungsteiler 1408.

Der zweite Anschluss des vierten Kondensators 1407 und der erste Anschluss des dritten Kondensators 1335 sind mit einem vierten Knoten K4 1409 gekoppelt, welcher wiederum mit dem invertierenden Eingang 1410 des Summen-Komparator-Elements 1405 gekoppelt ist. Ferner ist der vierte Knoten K4 1409 mit dem dritten Schalter S3 1343 gekoppelt, welcher in geschlossener Schalterposition die vierte Referenz-Spannungsquelle 1340 mit dem vierten Knoten K4 1409 verbindet, welcher in diesem Fall das vierte Referenzpotential VREF2 aufweist. Der dritte Schalter S3 1343 ist geschlossen, solange der Summen-Impuls-Generator 1413 einen Impuls erzeugt. In gleicher Weise schließt der Summen-Impuls-Generator 1413 einen vierten Schalter S4 1411. Dieser vierte Schalter S4 1411 koppelt in geschlossener Schalterstellung das negative Betriebspotential $V_{SS}$ 1336 mit dem dritten Knoten K3 1334, so dass an diesem das negative Betriebspotential $V_{SS}$ 1336 anliegt.

Der Ausgang 1412 des Summen-Komparator-Elements 1405 ist mit einem Summen-Impuls-Generator 1413 gekoppelt, welcher ausgangsseitig einerseits mit dem ersten Schalter S1 1326 und dem zweiten Schalter S2 1406 und andererseits mit dem dritten Schalter S3 1343 und dem vierten Schalter S4 1411 gekoppelt ist und diese steuert. Ferner ist der Ausgang des Summen-Impuls-Generators 1413 mit einem Summen-Zähler-Element 1414 gekoppelt, welches ebenfalls mit einem Schieberegister-Modus ausgestaltet ist und welches ausgangsseitig die Ergebnisdaten Daten des Sensorverfahrens bereitstellt.

Es ist in diesem Zusammenhang anzumerken, dass die Kapazität des zweiten Kondensators wesentlich größer gewählt ist, als die des ersten Kondensators, vorzugsweise mindestens doppelt so groß, besonders bevorzugt um den Faktor zehn größer. Entsprechend ist die Kapazität des vierten Kondensators 1407 wesentlich größer gewählt als die Kapazität des dritten Kondensators 1335, wiederum bevorzugt mindestens doppelt so groß, besonders bevorzugt um einen Faktor zehn größer.

Die Funktionsweise der Schaltkreisanordnung 1400 ist sehr ähnlich zu der Funktionsweise der Schaltkreisanordnung 1300 gemäß dem ersten Ausführungsbeispiel der Erfindung.

Der Strom für die Generator-Elektrode 1302 wird gemäß diesem Ausführungsbeispiel der Erfindung dem ersten kapazitiven Spannungsteiler 1402 entnommen. Da die Kapazität des zweiten Kondensators 1401 wesentlich größer ist als die Kapazität des ersten Kondensators 1317, sind der erste Knoten K1 1319 und der zweite Knoten K2 1403 stark miteinander gekoppelt und der Spannungshub ist auf beiden Knoten im Wesentlichen gleich. Entsprechendes gilt auf der Kollektorseite. Da die Kapazität des vierten Kondensators 1407 wesentlich größer gewählt ist als die Kapazität des dritten Kondensators 1335, wird der Spannungshub von dem dritten Knoten K3 1334 nahezu vollständig auf den vierten Knoten K4 1409 übertragen. Der zweite Knoten K2 1403 und der vierte Knoten K4 1409 weisen gegenläufige Signale auf, die in dem Summen-Komparator-Element 1405 miteinander verglichen werden. Eine nachgeschaltete Pulsformung mittels des Summen-Impuls-Generators 1413 sorgt wiederum für einen wohlgeformten Rücksetzpuls.

Während des Rücksetzpulses sind alle Schalter S1 bis S4 geschlossen. Der erste Knoten K1 1319 wird damit auf das positive Betriebspotential $V_{DD}$ 1318, der dritte Knoten K3 1334 auf das negative Betriebspotential $V_{SS}$ 1336, der zweite Knoten K2 1403 auf das dritte Referenzpotential VREF1 und der vierte Knoten K4 1409 auf das vierte Referenzpotential VREF2 zurückgesetzt. Das dritte Referenzpotential und das vierte Referenzpotential werden dabei so gewählt, dass das Summen-Komparator-Element 1405 in einem günstigen Arbeitsbereich betrieben wird.

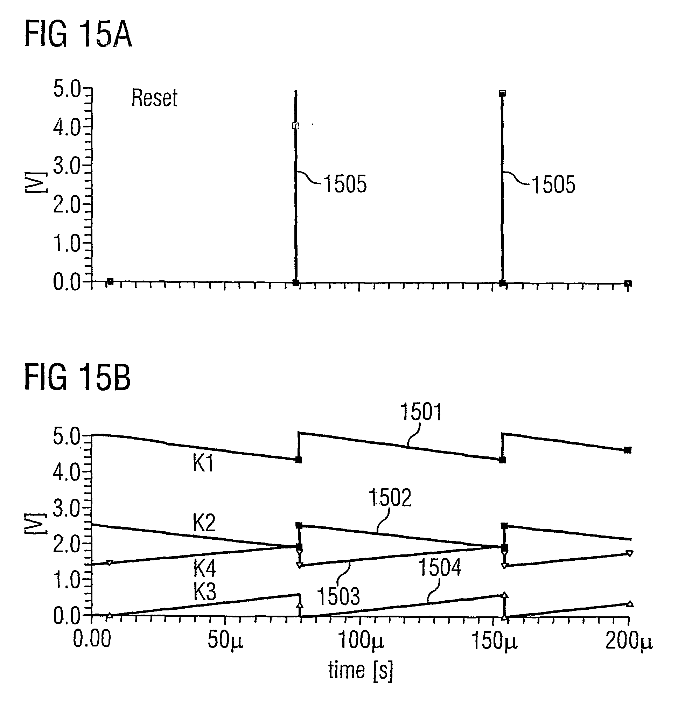

Eine Simulation des Zeitverlaufs der Spannungen an den vier Knoten K1 bis K4 zeigt **Fig.15b,** wobei

- ein erster Spannungsverlauf 1501 den Spannungsverlauf an dem ersten Knoten K1 1319,
- ein zweiter Spannungsverlauf 1502 den Spannungsverlauf an dem zweiten Knoten K2 1403,
- ein dritter Spannungsverlauf 1503 den Spannungsverlauf am dritten Knoten K3 1334, und
- ein vierter Spannungsverlauf 1504 den Spannungsverlauf am vierten Knoten K4 1409

zeigt.

Ferner sind in **Fig.15a** entsprechende Rücksetzpulse 1505, d.h. der Spannungsverlauf an einem fünften Knoten K5 1415, dem Ausgang des Summen-Impuls-Generators 1413 dargestellt.

Zur Ermittlung des in den Fig.15a und Fig.15b dargestellten Simulationsergebnisses wurden folgende Simulationsparameter verwendet:

- Elektrodenstrom: 1 nA,
- Kapazität des ersten Kondensators = 100 fF,
- Kapazität des dritten Kondensators = 100 fF,
- Kapazität zweiter Kondensator = 1 pF,
- Kapazität vierter Kondensator = 1 pF,
- positive Betriebsspannung $V_{DD}$ = 5 V,
- AGND = 2,5 V,
- V_ox = 0,1 V,
- V_red = -0,1 V,

- VREF1 = 2,5 V,
- VREF2 = 1, 5 V.

Die Simulation erfolgte mit nominellen Parametern für eine Schaltung in einem 0,5 $\mu$m - 5 V - Standard-CMOS-Prozess.

In einer alternativen Ausgestaltung der Schaltkreisanordnung 1400 ist es vorgesehen, wahlweise nur den Kollektorstrom oder nur den Generatorstrom zu messen. Zum Messen des Kollektorstroms werden beispielsweise der erste Schalter S1 1326 und der zweite Schalter S2 1406 dauerhaft geschlossen. Auf diese Weise ist der Regler für die Generatorspannung, das heißt das erste Generator-Komparator-Element 1311 permanent mit dem positiven Betriebspotential VDD 1318 verbunden, der nicht invertierende Komparatoreingang des Summen-Komparator-Elements 1405 (1404) liegt auf dem dritten Referenzpotential VREF1. Das Potential an dem vierten Knoten K4 1409 steigt somit so lange an, bis das dritte Referenzpotential VREF1 erreicht ist und wird dann mittels des Rücksetzimpulses auf das vierte Referenzpotential VRER2 zurückgesetzt.

Entsprechend können auch der dritte Schalter S3 1343 und der vierte Schalter S4 1410 dauerhaft geschlossen werden. In diesem Fall wird nur der Generatorstrom gemessen. Die Messung nur eines Stromes kann eine gute Testmöglichkeit zur Kontrolle der Symmetrie der elektrochemischen Signale sein.

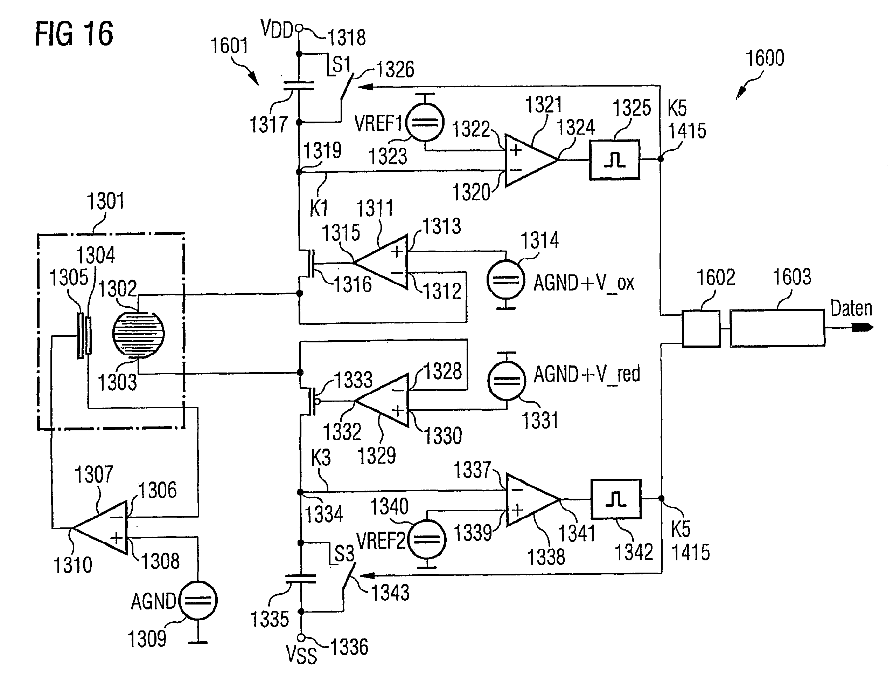

**Fig.16** zeigt eine Schaltkreisanordnung 1600 gemäß einem dritten Ausführungsbeispiel der Erfindung. Gleiche elektrische Komponenten der Schaltkreisanordnung 1600 gemäß dem dritten Ausführungsbeispiel und der Schaltkreisanordnung 1300 gemäß dem ersten Ausführungsbeispiel der Erfindung sind mit gleichen Bezugszeichen versehen.

Bei der Schaltkreisanordnung 1600 ist die gesamte Analogseite 1601 in gleicher Weise ausgestaltet wie bei der Schaltkreisanordnung 1300 gemäß dem ersten Ausführungsbeispiel der Erfindung.

Gemäß diesem Ausführungsbeispiel ist jedoch eine der beiden in der Schaltkreisanordnung 1300 vorgesehenen Zähler-Elemente 1327 und 1344 eingespart, indem beide von dem ersten Impuls-Generator 1325 und dem zweiten Impuls-Generator 1342 erzeugten Impulsfolgen mit nur einem Summen-Zähler-Element 1603 verarbeitet werden.

Erfindungsgemäß ist in diesem Fall nur eine einfache Digitalschaltung, vorzugsweise als Pufferschaltung, eingerichtet, allgemein als Synchronisationselement 1602 bezeichnet, welche auch bei einem zeitlichen Überlappen der beiden erzeugten Impulsfolgen kontrolliert zwei zeitlich nacheinander ausgegebene Impulse abgibt, vorgesehen.

Auch in diesem Fall ist es natürlich möglich, wie bei der oben beschriebenen Schaltkreisanordnung 1400 gemäß dem zweiten Ausführungsbeispiel der Erfindung, zu Testzwecken nur eine Pulsfolge zu zählen und die andere zu sperren.

In diesem Dokument sind folgende Veröffentlichungen zitiert:

[1] Hintsche, R, Paeschke, M, Uhlig, A, Seitz, R (1997) "Microbiosensors using Electrodes made in Si-technology", Frontiers in Biosensorics, Fundamental Aspects, Scheller, FW, Schubert, F, Fedrowitz, J (eds.), Birkhauser Verlag Basel, Schweiz, S.267-283

[2] van Gerwen, P (1997) "Nanoscaled Interdigitated Electrode Arrays for Biochemical Sensors", IEEE, International Conference on Solid-State Sensors and Actuators, 16.-19. Juni 1997, Chicago, S.907-910

[3] Paeschke, M, Dietrich, F, Uhlig, A, Hintsche, R (1996) "Voltammetric Multichannel Measurements Using Silicon Fabricated Microelectrode Arrays", Electroanalysis, Vol. 7, No.1, S.1-8

[4] Uster, M, Loeliger, T, Guggenbühl, W, Jäckel, H (1999) "Integrating ADC Using a Single Transistor as Integrator and Amplifier for Very Low (1fA Minimum) Input Currents", Advanced A/D and D/A Conversion Techniques and Their Applications, Konferenz der Universität Strathclyde (Großbritannien) 27.-28. Juli 1999, Conference Publication No. 466, S. 86-89, IEE

[5] Breten, M, Lehmann, T, Bruun, E (2000) "Integrating data converter for picoampere currents from electrochemical transducers", ISCAS 2000, IEEE International Symposium on Circuits and Systems, 28.-31. Mai 2000, Genf, Schweiz

[6] US 3,711,779

[7] US 4,199,728

[8] Thewes, R et al. (2002) "Sensor arrays for fully-electronic DNA detection on CMOS", Solid-State Circuits Conference, IEEE International 3.-7. Februar 2002

**Bezugszeichenliste**

| | |
|---|---|
| 100 | Schaltkreis-Anordnung |
| 101 | Sensor-Elektrode |
| 102 | erste Schaltungs-Einheit |
| 103 | zweite Schaltungs-Einheit |
| 104 | erster Kondensator |
| 105 | Fängermoleküle |
| 106 | zu erfassendes Molekül |
| 107 | Enzymlabel |
| 108 | elektrisch geladene Partikel |
| 109 | erste Regelungs-Einheit |
| 110 | regelbarer ohmscher Widerstand |
| 111 | zweite Regelungs-Einheit |
| 112 | Puls |
| 113 | weiterer Schalter |
| 114 | Spannungsquelle |

| | |
|---|---|
| 200 | Sensor |
| 201 | Elektrode |
| 202 | Elektrode |
| 203 | Isolator |
| 204 | Elektrodenanschluss |
| 205 | Elektrodenanschluss |
| 206 | DNA-Sondenmolekül |
| 207 | Elektrolyt |
| 208 | DNA-Stränge |

| | |
|---|---|
| 300 | Interdigitalelektrode |

| | |
|---|---|
| 400 | Biosensor |
| 401 | erste Elektrode |
| 402 | zweite Elektrode |
| 403 | Isolatorschicht |
| 404 | Haltebereich erste Elektrode |
| 405 | DNA-Sondenmolekül |
| 406 | Elektrolyt |
| 407 | DNA-Strang |
| 408 | Enzym |
| 409 | spaltbares Molekül |
| 410 | negativ geladenes erstes Teilmolekül |
| 411 | Pfeil |
| 412 | weitere Lösung |
| 413 | oxidiertes erstes Teilmolekül |
| 414 | reduziertes erstes Teilmolekül |

| | |
|---|---|
| 500 | Diagramm |
| 501 | elektrischer Strom |
| 502 | Zeit |
| 503 | Kurvenverlauf Strom Zeit |
| 504 | Offset-Strom |

| | |
|---|---|
| 600 | Schaltkreis-Anordnung |
| 601 | Sensor-Elektrode |
| 602 | erste Schaltungseinheit |
| 603 | zweite Schaltungseinheit |

| | |
|---|---|
| 604 | erster Kondensator |
| 605 | Knoten |
| 606 | Zähler-Element |
| 607 | erstes Komparator-Element |
| 608 | zweite Spannungsquelle |
| 609 | Transistor |
| 610 | zweites Komparator-Element |
| 611 | dritte Spannungsquelle |
| 612 | Schalter |
| 613 | Pulsgeber |
| 620 | Schaltkreis-Anordnung |
| 621 | Kalibrier-Einrichtung |
| 621a | Referenz-Stromquelle |
| 622 | weiterer Schalter |

| | |
|---|---|
| 700 | Schaltkreis-Anordnung |
| 701 | weitere Sensor-Elektrode |
| 702 | erster elektrischer Knoten |
| 703 | erster Transistor |
| 704 | zweiter elektrischer Knoten |
| 705 | erste Spannungsversorgung |
| 706 | dritter elektrischer Knoten |
| 707 | vierter elektrischer Knoten |
| 708 | erste Kapazität |
| 709 | fünfter elektrischer Knoten |
| 710 | sechster elektrischer Knoten |
| 711 | zweite Kapazität |
| 712 | zweite Spannungsversorgung |
| 713 | siebter elektrischer Knoten |
| 714 | achter elektrischer Knoten |
| 715 | dritte Kapazität |
| 716 | neunter elektrischer Knoten |
| 717 | zehnter elektrischer Knoten |
| 718 | elfter elektrischer Knoten |
| 719 | zwölfter elektrischer Knoten |
| 720 | dreizehnter elektrischer Knoten |
| 721 | vierte Kapazität |
| 722 | fünfte Kapazität |
| 723 | erste Spannungsversorgungs-Einheit |
| 724 | zweite Spannungsversorgungs-Einheit |
| 725 | vierzehnter elektrischer Knoten |
| 726 | fünfzehnter elektrischer Knoten |
| 727 | dritte Spannungsversorgung |
| 728 | vierte Spannungsversorgung |
| 729 | erstes Steuersignal |
| 730 | zweites Steuersignal |
| 731 | drittes Steuersignal |
| 732 | viertes Steuersignal |
| 733 | fünftes Steuersignal |
| 734 | sechstes Steuersignal |
| 735 | siebtes Steuersignal |
| 736 | Zähler-Einheit |
| 737 | sechzehnter elektrischer Knoten |
| 738 | siebzehnter elektrischer Knoten |
| 739 | achtzehnter elektrischer Knoten |
| 740 | neunzehnter elektrischer Knoten |
| 741 | zwanzigster elektrischer Knoten |
| 742 | einundzwanzigster elektrischer Knoten |

| 743 | zweiundzwanzigster elektrischer Knoten |
|---|---|
| 744 | sechste Kapazität |
| 745 | dreiundzwanzigster elektrischer Knoten |
| 746 | siebte Kapazität |
| 747 | vierundzwanzigster elektrischer Knoten |
| 748 | Ausgangs-Anschluss |
| | |
| 800 | nicht-invertierter Eingang |
| 801 | erster elektrischer Knoten |
| 803 | invertierter Eingang |
| 804a | erster Anschluss |
| 804b | zweiter Anschluss |
| 804c | dritter Anschluss |
| 805 | Ausgang |
| 806 | zweiter elektrischer Knoten |
| 807 | Kondensator |
| 808 | dritter elektrischer Knoten |
| 810 | Ausgangs-Anschluss |
| | |
| 900 | erster elektrischer Knoten |
| 901 | erster Transistor |
| 902 | zweiter Transistor |
| 903 | zweiter elektrischer Knoten |
| 904 | dritter elektrischer Knoten |
| 905 | vierter elektrischer Knoten |
| 906 | fünfter elektrischer Knoten |
| 907 | sechster elektrischer Knoten |
| 908 | dritter Transistor |
| 909 | vierter Transistor |
| 910 | siebter elektrischer Knoten |
| 911 | achter elektrischer Knoten |
| 912 | neunter elektrischer Knoten |
| 913 | fünfter Transistor |
| 914 | zehnter elektrischer Knoten |
| 915 | elfter elektrischer Knoten |
| 916 | sechster Transistor |
| 917 | zwölfter elektrischer Knoten |
| 918 | siebter Transistor |
| 919 | dreizehnter elektrischer Knoten |
| 920 | achter Transistor |
| 921 | vierzehnter elektrischer Knoten |
| 922 | neunter Transistor |
| 923 | fünfzehnter elektrischer Knoten |
| 924 | zehnter Transistor |
| 925 | sechzehnter elektrischer Knoten |
| 926 | siebzehnter elektrischer Knoten |
| 927 | elfter Transistor |
| | |
| 1000 | erster Eingang |
| 1001 | zweiter Eingang |
| 1002 | Ausgang |
| 1003 | Versorgungs-Eingang |
| 1004 | erster Transistor |
| 1005 | erster elektrischer Knoten |
| 1006 | zweiter Transistor |
| 1007 | zweiter elektrischer Knoten |
| 1008 | dritter Transistor |
| 1009 | dritter elektrischer Knoten |

| | |
|---|---|
| 1010 | vierter Transistor |
| 1011 | vierter elektrischer Knoten |
| 1012 | fünfter Transistor |
| 1013 | fünfter elektrischer Knoten |
| 1014 | sechster Transistor |
| 1015 | siebter Transistor |
| 1016 | sechster elektrischer Knoten |
| 1017 | achter Transistor |
| 1018 | siebter elektrischer Knoten |
| 1019 | neunter Transistor |
| 1020 | zehnter Transistor |
| 1021 | achter elektrischer Knoten |
| 1022 | neunter elektrischer Knoten |
| 1023 | elfter Transistor |
| 1024 | zwölfter Transistor |
| | |
| 1100 | Zähler-Element |
| 1101 | erster Eingang |
| 1102 | zweiter Eingang |
| 1103 | dritter Eingang |
| 1104 | vierter Eingang |
| 1105 | fünfter Eingang |
| 1106 | erster Ausgang |
| 1107 | zweiter Ausgang |
| 1108 | erster elektrischer Knoten |
| 1109 | erster Transistor |
| 1110 | zweiter Transistor |
| 1111 | zweiter elektrischer Knoten |
| 1112 | dritter elektrischer Knoten |
| 1113 | dritter Transistor |
| 1114 | vierter Transistor |
| 1115 | vierter elektrischer Knoten |
| 1116 | fünfter elektrischer Knoten |
| 1117 | fünfter Transistor |
| 1118 | sechster elektrischer Knoten |
| 1119 | siebter elektrischer Knoten |
| 1120 | sechster Transistor |
| 1121 | achter elektrischer Knoten |
| 1122 | siebter Transistor |
| 1123 | achter Transistor |
| 1124 | neunter elektrischer Knoten |
| 1125 | zehnter elektrischer Knoten |
| 1126 | elfter elektrischer Knoten |
| 1127 | neunter Transistor |
| 1128 | zwölfter elektrischer Knoten |
| 1129 | zehnter Transistor |
| 1130 | dreizehnter elektrischer Knoten |
| 1131 | vierzehnter elektrischer Knoten |
| 1132 | fünfzehnter elektrischer Knoten |
| 1133 | sechzehnter elektrischer Knoten |
| 1134 | elfter Transistor |
| 1135 | zwölfter Transistor |
| 1136 | dreizehnte Transistor |
| 1137 | siebzehnter elektrischer Knoten |
| 1138 | achtzehnter elektrischer Knoten |
| 1139 | neunzehnter elektrischer Knoten |
| 1140 | vierzehnter Transistor |
| 1141 | zwanzigster elektrischer Knoten |

| | |
|---|---|
| 1142 | fünfzehnter Transistor |
| 1143 | einundzwanzigster elektrischer Knoten |
| 1144 | zweiundzwanzigster elektrischer Knoten |
| 1145 | sechzehnter Transistor |
| 1146 | siebzehnter Transistor |
| 1147 | dreiundzwanzigster elektrischer Knoten |
| 1148 | achtzehnter Transistor |
| 1149 | vierundzwanzigster elektrischer Knoten |
| 1150 | neunzehnter Transistor |
| 1151 | fünfundzwanzigster elektrischer Knoten |
| 1152 | zwanzigster Transistor |
| 1153 | sechsundzwanzigster elektrischer Knoten |
| 1154 | einundzwanzigster Transistor |

| | |
|---|---|
| 1200 | Sensor-Anordnung |
| 1201 | Schaltkreis-Anordnung |
| 1202 | Chip |

| | |
|---|---|
| 1300 | Schaltkreisanordnung |
| 1301 | Elektrochemisches System |
| 1302 | Generator-Elektrode |
| 1303 | Kollektor-Elektrode |
| 1304 | Referenz-Elektrode |
| 1305 | Gegen-Elektrode |
| 1306 | Invertierender Eingang Referenz-Potential-Einstell-Operationsverstärker |
| 1307 | Referenz-Potential-Einstell-Operationsverstärker |
| 1308 | Nicht-invertierender Eingang Referenz-Potential-Einstell-Operationsverstärker |
| 1309 | Analyten-Referenz-Potential-Spannungsquelle |
| 1310 | Ausgang Referenz-Potential-Einstell-Operationsverstärker |
| 1311 | Erstes Generator-Komparator-Element |
| 1312 | Invertierender Eingang erstes Generator-Komparator-Element |
| 1313 | Nicht-invertierender Eingang erstes Generator-Komparator-Element |
| 1314 | Erste Referenz-Spannungsquelle |
| 1315 | Ausgang erstes Generator-Komparator-Element |
| 1316 | Erster NMOS-Transistor |
| 1317 | Erster Kondensator |
| 1318 | Positives Betriebs-Potential |
| 1319 | Erster Knoten |
| 1320 | Invertierender Eingang zweites Generator-Komparator-Element |
| 1321 | Zweites Generator-Komparator-Element |
| 1322 | Nicht-invertierender Eingang zweites Generator-Komparator-Element |
| 1323 | Zweite Referenz-Spannungsquelle |
| 1324 | Ausgang zweites Generator-Komparator-Element |
| 1325 | Erster Impuls-Generator |
| 1326 | Erster Schalter |
| 1327 | Erstes Zählerelement |
| 1328 | Invertierender Eingang Kollektor-Komparator-Element |
| 1329 | Kollektor-Komparator-Element |
| 1330 | Nicht-invertierender Eingang Kollektor-Komparator-Element |
| 1331 | Dritte Referenz-Spannungsquelle |
| 1332 | Ausgang Kollektor-Komparator-Element |
| 1333 | Erster PMOS-Transistor |
| 1334 | Dritter Knoten |
| 1335 | Dritter Kondensator |
| 1336 | Negatives Betriebspotential |
| 1337 | Invertierender Eingang zweites Kollektor-Komparator-Element |
| 1338 | Zweites Kollektor-Komparator-Element |
| 1339 | Nicht-invertierender Eingang zweites KollektorKomparator-Element |

EP 1 636 599 B1

1340     Vierte Referenz-Spannungsquelle
1341     Ausgang zweites Kollektor-Komparator-Element
1342     Zweiter Impuls-Generator
1343     Dritter Schalter
1344     Zweites Zählerelement

1400     Schaltkreisanordnung
1401     Zweiter Kondensator
1402     Erster kapazitiver Spannungsteiler
1403     Zweiter Knoten
1404     Nicht-invertierender Eingang Summen-Komparator-Element
1405     Summen-Komparator-Element
1406     Zweiter Schalter
1407     Vierter Kondensator
1408     Zweiter kapazitiver Spannungsteiler
1409     Vierter Knoten
1410     Invertierender Eingang Summen-Komparator-Element
1411     Vierter Schalter
1412     Ausgang Summen-Komparator-Element
1413     Summen-Impuls-Generator
1414     Summen-Zählerelement
1415     Fünfter Knoten

1501     Erster Spannungsverlauf
1502     Zweiter Spannungsverlauf
1503     Dritter Spannungsverlauf
1504     Vierter Spannungsverlauf
1505     Rücksetzimpuls-Signal

1600     Schaltkreisanordnung
1601     Analogseite
1602     Synchronisationselement
1603     Summen-Zählerelement

## Patentansprüche

1.   Schaltkreis-Anordnung

    • mit einer Sensor-Elektrode (1302);
    • mit einer ersten Schaltungseinheit (1311), die mit der Sensor-Elektrode (1302) elektrisch gekoppelt ist;
    • mit einer zweiten Schaltungseinheit (1317, 1321), die einen ersten Kondensator (1317) aufweist;
    • wobei die erste Schaltungseinheit (1311, 1316) derart eingerichtet ist, dass sie das elektrische Potential der Sensor-Elektrode (1302) in einem vorgebbaren ersten Referenz-Bereich um ein vorgebbares elektrisches erstes Soll-Potential ($AGND_{+V\_ox}$) hält, indem der erste Kondensator (1317) und die Sensor-Elektrode (1302), wenn das Potential der Sensor-Elektrode (1302) außerhalb des ersten Referenz-Bereichs ist, miteinander derart elektrisch gekoppelt werden, dass ein Angleichen des elektrischen Potentials der Sensor-Elektrode (1302) möglich ist;
    • wobei die Sensor-Elektrode (1302) als Generator-Elektrode eingerichtet ist,
    • wobei die zweite Schaltungseinheit (1317, 1321) derart eingerichtet ist, dass sie, wenn das elektrische Potential des ersten Kondensators (1317) außerhalb eines zweiten Referenz-Bereichs ist,

        o dieses Ereignis detektiert; und
        o den ersten Kondensator (1317) auf ein zweites elektrisches Potential bringt.

    • mit einer Kollektor-Elektrode (1303),
    • mit einer dritten Schaltungseinheit (1329, 1333), die mit der Kollektor-Elektrode (1303) elektrisch gekoppelt ist;
    • mit einer vierten Schaltungseinheit (1335, 1338), die einen zweiten Kondensator (1335) aufweist;

• wobei die dritte Schaltungseinheit (1329, 1333) derart eingerichtet ist, dass sie das elektrische Potential der Kollektor-Elektrode (1303) in einem vorgebbaren dritten Referenz-Bereich um ein vorgebbares elektrisches zweites Soll-Potential (AGND$_{+V\_red}$) hält, indem der zweite Kondensator (1335) und die Kollektor-Elektrode (1303), wenn das Potential der Kollektor-Elektrode (1303) außerhalb des dritten Referenz-Bereichs ist, miteinander derart elektrisch gekoppelt werden, dass ein Angleichen des elektrischen Potentials der Kollektor-Elektrode (1303) möglich ist;

• wobei die vierte Schaltungseinheit (1335, 1338) derart eingerichtet ist, dass sie, wenn das Potential des zweiten Kondensators (1335) außerhalb eines vierten Referenz-Bereichs ist,

  o dieses Ereignis detektiert; und
  o den zweiten Kondensator (1335) auf ein viertes elektrisches Referenzpotential bringt.

2. Schaltkreis-Anordnung gemäß Anspruch 1 (vgl. Figur 14),

  • mit einem mit dem ersten Kondensator (1317) gekoppelten dritten Kondensator (1401), wobei der dritte Kondensator (1401) eine größere Kapazität aufweist als der erste Kondensator (1317) und wobei der erste Kondensator (1317) und der dritte Kondensator (1401) einen kapazitiven ersten Spannungsteiler (1402) bilden;
  • mit einem mit dem zweiten Kondensator (1335) gekoppelten vierten Kondensator (1407), wobei der vierte Kondensator (1407) eine größere Kapazität aufweist als der zweite Kondensator (1335) und wobei der zweite Kondensator (1335) und der vierte Kondensator (1407) einen kapazitiven zweiten Spannungsteiler (1408) bilden.

3. Schaltkreis-Anordnung gemäß Anspruch 2,

  • bei der die Kapazität des dritten Kondensators (1401) mindestens um den Faktor zwei größer ist als die Kapazität des ersten Kondensators (1317); und
  • bei der die Kapazität des vierten Kondensators (1407) mindestens um den Faktor zwei größer ist als die Kapazität des zweiten Kondensators (1335).

4. Schaltkreis-Anordnung gemäß Anspruche 2 oder 3, bei der die zweite Schaltungseinheit (1317) und die vierte Schaltungseinheit (1335) gemeinsam ein Summen-Komparator-Element (1405) aufweisen mit zwei Eingängen (1404, 1410) und einem Ausgang (1412), wobei

  • ein erster Eingang (1404) zwischen den ersten Kondensator (1317) und den dritten Kondensator (1401) geschaltet ist,
  • ein zweiter Eingang (1410) zwischen den zweiten Kondensator (1335) und den vierten Kondensator (1407) geschaltet ist, und
  • der Ausgang (1412) mit einem Zähler-Element (1414) gekoppelt ist, das derart eingerichtet ist, dass es die Anzahl und/oder die zeitliche Abfolge der Ereignisse zählt.

5. Schaltkreis-Anordnung gemäß Anspruch 1 (vgl. Figur 16), bei der die erste Schaltungseinheit (1311, 1316) ein Generator-Komparator-Element (1311) aufweist mit zwei Eingängen (1312, 1313) und einem Ausgang (1315), wobei

  • ein erster Eingang (1312) derart mit der Generator-Elektrode (1302) gekoppelt ist, dass der erste Eingang (1312) das elektrische Potential der Generator-Elektrode (1302) aufweist,
  • ein zweiter Eingang (1313) ein erstes elektrisches Referenzpotential aufweist, welches das elektrische erste Soll-Potential (AGND$_{+V\_ox}$) definiert,
  • das Generator-Komparator-Element (1311) derart eingerichtet ist, dass an dem Ausgang (1315) ein derartiges elektrisches Signal erzeugt wird, dass das elektrische Potential der Generator-Elektrode (1302) in dem ersten Referenz-Bereich um das elektrische erste Soll-Potential (AGND$_{+V\_ox}$) gehalten wird,

bei der die dritte Schaltungseinheit ein Kollektor-Komparator-Element (1329) aufweist mit zwei Eingängen (1328, 1330) und einem Ausgang (1332), wobei

  • ein erster Eingang (1328) derart mit der Kollektor-Elektrode (1303) gekoppelt ist, dass der erste Eingang (1328) das elektrische Potential der Kollektor-Elektrode (1303) aufweist,
  • ein zweiter Eingang (1330) ein drittes elektrisches Referenzpotential aufweist, welches das elektrische zweite Soll-Potential (AGND$_{+v\_red}$) definiert,
  • das Kollektor-Komparator-Element (1329) derart eingerichtet ist, dass an dem Ausgang (1332) ein derartiges elektrisches Signal erzeugt wird, dass das elektrische Potential der Kollektor-Elektrode (1303) in dem dritten Referenz-Bereich um das elektrische zweite Soll-Potential (AGND$_{+V\_red}$) gehalten wird,

bei der die zweite Schaltungseinheit (1317, 1321) ein zweites Generator-Komparator-Element (1321) mit zwei Eingängen (1320, 1322) und einem Ausgang (1324) aufweist, wobei

• der erste Eingang (1320) derart mit dem ersten Kondensator (1317) gekoppelt ist, dass der erste Eingang (1320) das elektrische Potential des ersten Kondensators (1317) aufweist,
• der zweite Eingang (1322) ein Generator-Referenzpotential (V$_{REF1}$) aufweist,
• das zweite Generator-Komparator-Element (1321) derart eingerichtet ist, dass an dessen Ausgang (1324) ein derartiges elektrisches Signal erzeugt wird, dass, wenn das elektrische Potential des ersten Kondensators (1317) das Generator-Referenzpotential (V$_{REF1}$) überschreitet, der erste Kondensator (1317) auf das Generator-Referenzpotential (V$_{REF1}$) gebracht wird,

bei der die vierte Schaltungseinheit (1335, 1338) ein zweites Kollektor-Komparator-Element (1338) mit zwei Eingängen (1337, 1339) und einem Ausgang (1341) aufweist, wobei

• der erste Eingang (1337) derart mit dem zweiten Kondensator (1335) gekoppelt ist, dass der erste Eingang (1337) das elektrische Potential des zweiten Kondensators (1335) aufweist,
• der zweite Eingang (1339) ein Kollektor-Referenzpotential (V$_{REF2}$) aufweist,
• das zweite Kollektor-Komparator-Element (1338) derart eingerichtet ist, dass an dessen Ausgang (1341) ein derartiges elektrisches Signal erzeugt wird, dass, wenn das elektrische Potential des zweiten Kondensators (1335) das Kollektor-Referenzpotential (V$_{REF2}$) überschreitet, der zweite Kondensator (1335) auf das Kollektor-Referenzpotential (V$_{REF2}$) gebracht wird,

bei der ein Synchronisationselement (1602) mit zwei Eingängen und einem Ausgang vorgesehen ist, wobei

• ein erster Eingang mit dem Ausgang (1324) des zweiten Generator-Komparator-Elements (1321) gekoppelt ist,
• ein zweiter Eingang mit dem Ausgang (1341) des zweiten Kollektor-Komparator-Elements (1338) gekoppelt ist,
• das Synchronisationselement (1602) derart eingerichtet ist, dass im Falle einer Überlappung der Signale an den beiden Eingängen eines der Signale verzögert wird, sodass zu einem Zeitpunkt nur das Ausgangssignal des zweiten Generator-Komparator-Elements (1321) oder nur das Ausgangssignal des zweiten Kollektor-Komparator-Elements (1338) an dem Ausgang des Synchronisationselements (1602) bereitgestellt wird.

6. Schaltkreis-Anordnung gemäß Anspruch 5,
bei der das Synchronisationselement (1602) einen Pufferspeicher aufweist.

## Claims

1. Circuit arrangement,

• having a sensor electrode (1302);
• having a first circuit unit (1311), which is electrically coupled to the sensor electrode (1302); having a second circuit unit (1317, 1321), which has a first capacitor (1317);
• the first circuit unit (1311, 1316) being set up in such a way that it holds the electrical potential of the sensor electrode (1302) in a predeterminable first reference range around a predeterminable electrical first desired potential (AGND$_{+V\_ox}$) by electrically coupling the first capacitor (1317) and the sensor electrode (1302), if the potential of the sensor electrode (1302) is outside the first reference range, to one another in such a way that a matching of the electrical potential of the sensor electrode (1302) is possible;
• the sensor electrode (1302) being set up as a generator electrode,
• the second circuit unit (1317, 1321) being set up in such a way that, if the electrical potential of the first capacitor (1317) is outside a second reference range, said second circuit unit

o detects this event; and
o brings the first capacitor (1317) to a second electrical potential,

• having a collector electrode (1303),

• having a third circuit unit (1329, 1333), which is electrically coupled to the collector electrode (1303);

• having a fourth circuit unit (1335, 1338), which has a second capacitor (1335);

• the third circuit unit (1329, 1333) being set up in such a way that it holds the electrical potential of the collector electrode (1303) in a predeterminable third reference range around a predeterminable electrical second desired potential ($AGND_{+V\_red}$) by electrically coupling the second capacitor (1335) and the collector electrode (1303), if the potential of the collector electrode (1303) is outside the third reference range, to one another in such a way that a matching of the electrical potential of the collector electrode (1303) is possible;

• the fourth circuit unit (1335, 1338) being set up in such a way that, if the potential of the second capacitor (1335) is outside a fourth reference range, said circuit unit

    o detects this event; and
    o brings the second capacitor (1335) to a fourth electrical reference potential.

**2.** Circuit arrangement according to claim 1 (cf. figure 14),

• having a third capacitor (1401) coupled to the first capacitor (1317), the third capacitor (1401) having a greater capacitance than the first capacitor (1317) and the first capacitor (1317) and the third capacitor (1401) forming a capacitive first voltage divider (1402);

• having a fourth capacitor (1407) coupled to the second capacitor (1335), the fourth capacitor (1407) having a greater capacitance than the second capacitor (1335) and the second capacitor (1335) and the fourth capacitor (1407) forming a capacitive second voltage divider (1408).

**3.** Circuit arrangement according to claim 2,

• in which the capacitance of the third capacitor (1401) is greater than the capacitance of the first capacitor (1317) at least by a factor of two; and

• in which the capacitance of the fourth capacitor (1407) is greater than the capacitance of the second capacitor (1335) at least by a factor of two.

**4.** Circuit arrangement according to claim 2 or 3,
in which the second circuit unit (1317) and the fourth circuit unit (1335) jointly have a summation comparator element (1405) having two inputs (1404, 1410) and an output (1412),

• a first input (1404) being connected between the first capacitor (1317) and the third capacitor (1401),

• a second input (1410) being connected between the second capacitor (1335) and the fourth capacitor (1407), and

• the output (1412) being coupled to a counter element (1414), which is set up in such a way that it counts the number and/or the temporal sequence of the events.

**5.** Circuit arrangement according to claim 1 (cf. figure 16), in which the first circuit unit (1311, 1316) has a generator comparator element (1311) having two inputs (1312, 1313) and an output (1315),

• a first input (1312) being coupled to the generator electrode (1302) in such a way that the first input (1312) has the electrical potential of the generator electrode (1302),

• a second input (1313) having a first electrical reference potential, which defines the electrical first desired potential ($AGND_{+V\_ox}$),

• the generator comparator element (1311) being set up in such a way that an electrical signal is generated at the output (1315) such that the electrical potential of the generator electrode (1302) is held in the first reference range around the electrical first desired potential ($AGND_{+V\_ox}$),

in which the third circuit unit has a collector comparator element (1329) having two inputs (1328, 1330) and an output (1332),

• a first input (1328) being coupled to the collector electrode (1303) in such a way that the first input (1328) has the electrical potential of the collector electrode (1303),

• a second input (1330) having a third electrical reference potential, which defines the electrical second desired potential ($AGND_{+V\_red}$),

• the collector comparator element (1329) being set up in such a way that an electrical signal is generated at the output (1332) such that the electrical potential of the collector electrode (1303) is held in the third reference range around the electrical second desired potential (AGND$_{+V\_red}$),

in which the second circuit unit (1317, 1321) has a second generator comparator element (1321) having two inputs (1320, 1322) and an output (1324),

• the first input (1320) being coupled to the first capacitor (1317) in such a way that the first input (1320) has the electrical potential of the first capacitor (1317),
• the second input (1322) having a generator reference potential (V$_{REF1}$),
• the second generator comparator element (1321) being set up in such a way that an electrical signal is generated at its output (1324) such that, if the electrical potential of the first capacitor (1317) exceeds the generator reference potential (V$_{REF1}$), the first capacitor (1317) is brought to the generator reference potential (V$_{REF1}$),

in which the fourth circuit unit (1335, 1338) has a second collector comparator element (1338) having two inputs (1337, 1339) and an output (1341),

• the first input (1337) being coupled to the second capacitor (1335) in such a way that the first input (1337) has the electrical potential of the second capacitor (1335),
• the second input (1339) having a collector reference potential (V$_{REF2}$),
• the second collector comparator element (1338) being set up in such a way that an electrical signal is generated at its output (1341) such that, if the electrical potential of the second capacitor (1335) exceeds the collector reference potential (V$_{REF2}$), the second capacitor (1335) is brought to the collector reference potential (V$_{REF2}$),

in which a synchronization element (1602) having two inputs and an output is provided,

• a first input being coupled to the output (1324) of the second generator comparator element (1321),
• a second input being coupled to the output (1341) of the second collector comparator element (1338),
• the synchronization element (1602) being set up in such a way that, in the case of an overlap of the signals at the two inputs, one of the signals is delayed, so that, at one point in time, only the output signal of the second generator comparator element (1321) or only the output signal of the second collector comparator element (1338) is provided at the output of the synchronization element (1602).

6. Circuit arrangement according to claim 5,
in which the synchronization element (1602) has a buffer memory.

**Revendications**

1. Agencement de circuit comportant

- une électrode de capteur (1302) ;
- une première unité de commutation (1311) qui est couplée électriquement à l'électrode de capteur (1302) ;
- une deuxième unité de commutation (1317, 1321) qui comporte un premier condensateur (1317) ;
- la première unité de commutation (1311, 1316) étant conçue de façon à maintenir le potentiel électrique de l'électrode de capteur (1302) dans un premier domaine de référence prescrit autour d'un premier potentiel électrique de consigne prescrit (AGND$_{+V\_ox}$) en couplant électriquement entre eux le premier condensateur (1317) et l'électrode de capteur (1302) lorsque le potentiel de l'électrode de capteur (1302) se trouve à l'extérieur du premier domaine de référence de façon à pouvoir équilibrer le potentiel électrique de l'électrode de capteur (1302) ;
- l'électrode de capteur (1302) étant conformée en électrode de générateur,
- la deuxième unité de commutation (1317, 1321) étant conçue de telle sorte que, lorsque le potentiel électrique du premier condensateur (1317) se trouve à l'extérieur d'un deuxième domaine de référence,

-- elle détecte cet événement ; et
-- elle porte le premier condensateur (1317) à un deuxième potentiel électrique,

- une électrode de collecteur (1303),

- une troisième unité de commutation (1329, 1333) qui est couplée électriquement à l'électrode de collecteur (1303) ;
- une quatrième unité de commutation (1335, 1338) qui comporte un deuxième condensateur (1335) ;
- la troisième unité de commutation (1329, 1333) étant conçue de façon à maintenir le potentiel électrique de l'électrode de collecteur (1303) dans un troisième domaine de référence prescrit autour d'un deuxième potentiel électrique de consigne prescrit (AGND$_{+v\_red}$) en couplant électriquement entre eux le deuxième condensateur (1335) et l'électrode de collecteur (1303) lorsque le potentiel de l'électrode de collecteur (1303) se trouve à l'extérieur du troisième domaine de référence de façon à pouvoir compenser le potentiel électrique de l'électrode de collecteur (1303) ;
- la quatrième unité de commutation (1335, 1338) étant conçue de telle sorte que, lorsque le potentiel du deuxième condensateur (1335) se trouve à l'extérieur d'un quatrième domaine de référence,

-- elle détecte ce résultat ; et
-- elle porte le deuxième condensateur (1335) à un quatrième potentiel électrique de référence.

**2.** Agencement de circuit selon la revendication 1 (cf. la figure 14), comportant

- un troisième condensateur (1401) couplé au premier condensateur (1317), le troisième condensateur (1401) ayant une capacité qui est supérieure à celle du premier condensateur (1317) et le premier condensateur (1317) et le troisième condensateur (1401) formant un premier diviseur de tension capacitif (1402) ;
- un quatrième condensateur (1407) couplé au deuxième condensateur (1335), le quatrième condensateur (1407) ayant une capacité qui est supérieure à celle du deuxième condensateur (1335) et le deuxième condensateur (1335) et le quatrième condensateur (1407) formant un deuxième diviseur de tension capacitif(1408).

**3.** Agencement de circuit selon la revendication 2, dans lequel

- la capacité du troisième condensateur (1401) étant supérieure d'un facteur d'au moins deux à la capacité du premier condensateur (1317) ; et
- la capacité du quatrième condensateur (1407) étant supérieure d'un facteur d'au moins deux à la capacité du deuxième condensateur (1335).

**4.** Agencement de circuit selon la revendication 2 ou 3, dans lequel la deuxième unité de commutation (1317) et la quatrième unité de commutation (1335) possèdent en commun un élément formant comparateur et additionneur (1405) qui possède deux entrées (1404, 1410) et une sortie (1412),

- une première entrée (1404) étant montée entre le premier condensateur (1317) et le troisième condensateur (1401),
- une deuxième entrée (1410) étant montée entre le deuxième condensateur (1335) et le quatrième condensateur (1407), et
- la sortie (1412) étant couplée à un élément formant compteur (1414) qui est conçu de façon à compter le nombre et/ou la succession des événements dans le temps.

**5.** Agencement de circuit selon la revendication 1 (cf. la figure 16) dans lequel la première unité de commutation (1311, 1316) comporte un élément formant générateur et comparateur (1311) qui possède deux entrées (1312, 1313) et une sortie (1315),

- une première entrée (1312) étant couplée à l'électrode de générateur (1302) de telle sorte que la première entrée (1312) possède le potentiel électrique de l'électrode de générateur (1302),
- une deuxième entrée (1313) ayant un deuxième potentiel électrique de référence qui définit le premier potentiel électrique de consigne (AGND$_{+v\_ox}$),
- l'élément formant générateur et comparateur (1311) étant conçu de façon à générer au niveau de la sortie (1315) un signal électrique tel que le potentiel électrique de l'électrode de générateur (1302) est maintenu dans le premier domaine de référence autour du premier potentiel électrique de consigne (AGND$_{+v\_ox}$),

la troisième unité de commutation comporte un élément formant collecteur et comparateur (1329) qui a deux entrées (1328, 1330) et une sortie (1332),

- une première entrée (1328) étant couplée à l'électrode de collecteur (1303) de telle sorte que la première entrée (1328) a le potentiel électrique de l'électrode de collecteur (1303),
- une deuxième entrée (1330) a un troisième potentiel électrique de référence qui définit le deuxième potentiel électrique de consigne (AGND$_{+V\_red)}$,
- l'élément formant collecteur et comparateur (1329) étant conçu de façon à générer au niveau de la sortie (1332) un signal électrique de telle que le potentiel électrique de l'électrode de collecteur (1303) est maintenu dans le troisième domaine de référence autour du deuxième potentiel électrique de consigne (AGND$_{+V\_red}$),

la deuxième unité de commutation (1317, 1321) comporte un deuxième élément formant générateur et comparateur (1321) qui possède deux entrées (1320, 1322) et une sortie (1324),

- la première entrée (1320) étant couplée au premier condensateur (1317) de telle sorte que la première entrée (1320) a le potentiel électrique du premier condensateur (1317),
- la deuxième entrée (1322) ayant un potentiel de générateur de référence (V$_{REF1}$),
- le deuxième élément formant générateur et comparateur (1321) étant conçu de façon à générer au niveau de sa sortie (1324) un signal électrique tel que, lorsque le potentiel électrique du premier condensateur (1317) dépasse le potentiel de générateur de référence (V$_{REF1}$), le premier condensateur (1317) est porté au potentiel de générateur de référence (V$_{REF1}$),

la quatrième unité de commutation (1335, 1338) comporte un deuxième élément formant collecteur et comparateur (1338) qui possède deux entrées (1337, 1339) et une sortie (1341),

- la première entrée (1337) étant couplée au deuxième condensateur (1335) de telle sorte que la première entrée (1337) a le potentiel électrique du deuxième condensateur (1335),
- la deuxième entrée (1339) ayant un potentiel de collecteur de référence (V$_{REF2}$),
- le deuxième élément formant collecteur et comparateur (1338) étant conçu de façon à générer au niveau de sa sortie (1341) un signal électrique tel que, lorsque le potentiel électrique du deuxième condensateur (1335) dépasse le potentiel de collecteur de référence (V$_{REF2}$), le deuxième condensateur (1335) est porté au potentiel de collecteur de référence (V$_{REF2}$),

Il est prévu un élément de synchronisation (1602) qui a deux entrées et une sortie,

- une première entrée étant couplée à la sortie (1324) du deuxième élément formant générateur et comparateur (1321),
- une deuxième entrée étant couplée à la sortie (1341) du deuxième élément formant collecteur et comparateur (1338),
- l'élément de synchronisation (1602) étant conçu de façon à retarder l'un des signaux en cas de chevauchement des signaux au niveau des deux entrées de sorte que à un instant donné seul le signal de sortie du deuxième élément formant générateur et comparateur (1321) ou seul le signal de sortie du deuxième élément formant collecteur et comparateur (1338) est délivré à la sortie de l'élément de synchronisation (1602).

6. Agencement de circuit selon la revendication 5, dans lequel l'élément de synchronisation (1602) comporte une mémoire tampon.

FIG 1

FIG 2A  Stand der Technik

FIG 2B  Stand der Technik

FIG 3A

FIG 3B  Stand der Technik

## FIG 4A  Stand der Technik

405

404

401 402

406

400

403

## FIG 4B  Stand der Technik

408

405

407

404

401 402

406

400

403

FIG 4C Stand der Technik

FIG 5 Stand der Technik

FIG 6A

FIG 6B

EP 1 636 599 B1

41

FIG 7

EP 1 636 599 B1

FIG 8

804b
804a    804c

602    810

706

803

805    806    609

800

807

607

702    801    808

**FIG 9**

**FIG 10**

44

FIG 11

FIG 12

1200

1202

1201    1201    1201

1201    1201    1201

# FIG 13

EP 1 636 599 B1

FIG 14

EP 1 636 599 B1

# FIG 15A

# FIG 15B

FIG 16